# EUROPEAN PATENT APPLICATION

(11) **EP 4 417 601 A1**
(43) Date of publication of application: **21.08.2024**
(21) Application number: 23156937.7
(22) Date of filing: 15.02.2023
(51) Int. Cl.: C07D 333/52, C07D 495/04, A61K 31/4375, A61K 31/381, A61P 1/00, A61P 35/00

(54) **SMALL MOLECULE TREATMENT OF FATTY LIVER DISEASE AND HCC**

(71) Applicant: ScandiEdge Therapeutics AB, 121 36 Johanneshov (SE)
(72) Inventor: MARDINOGLU, Adil, 412 60 Göteborg (SE); UHLÉN, Mathias, 181 90 Lidingö (SE); BORÉN, Jan, 414 67 Göteborg (SE); SEBHAOUI, Jihad, 34774 Istanbul (TR); ASHRAF, Sajda, 34774 Istanbul (TR); IQBAL, Shazia, 34774 Istanbul (TR)
(74) Representative: Kransell & Wennborg KB

(57) **Abstract**

There is provided a compound of formula (I) or a pharmaceutically acceptable salt thereof.

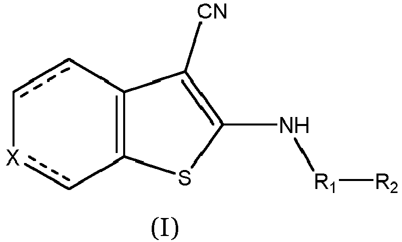

## Description

### TECHNICAL FIELD

The present disclosure relates to a pharmaceutical compound used for the treatment of fatty liver disease and hepatocellular carcinoma (HCC).

### BACKGROUND

Non-alcoholic fatty liver disease (NAFLD) is defined as the accumulation of fat in the liver due to the imbalance in the uptake and secretion of fat as well as the increased *de novo* lipogenesis (DNL) or decreased oxidation of fat in the liver (Gluchowski et al., 2017). NAFLD can progress to non-alcoholic steatohepatitis (NASH). It has been reported that the prevalence of NAFLD is approximately 25% of the population, but it has no approved effective pharmacological treatment. Hence, there is an urgent need for the development of an effective treatment strategy.

Previously, the research groups including the present inventors have generated gene co-expression networks (CNs) for the liver and 45 other primary human tissues, and identified the pyruvate kinase L/R (PKLR) as a target, whose inhibition may selectively inhibit DNL in the liver (Lee et al., 2017). The PKLR gene encodes for the liver (PKL) and erythrocyte (PKR) isoforms of the enzyme and catalyses the production of pyruvate and ATP from phosphoenolpyruvate and ADP. PKL and PKR isoforms are specifically expressed in the liver and erythrocytes, respectively. An independent mouse population study has also verified the driving role of PKLR in the development of NAFLD (Chella Krishnan et al., 2018). Recently, *in vitro* experiments were performed by inhibiting and overexpressing PKLR in HepG2 cells, and it was found that the expression of PKLR was significantly positively correlated with the expression of FASN, *de novo* lipogenesis (DNL), triacylglycerol (TAG) levels and cell growth (Liu et al., 2019).

Furthermore, the small molecule, JNK-IN-5A, has shown potential in supressing the expression levels of PKLR and may thereby be used in the treatment of NAFLD (Zhang et al., 2022).

### SUMMARY

The present inventors have realized that there is a possibility to develop and optimize small molecules, which may be used in the treatment of fatty liver disease and hepatocellular carcinoma (HCC). The development/optimization was carried out by altering the molecular structure of JNK-IN-5A to improve the effect.

According to a first aspect of the present disclosure, there is provided a compound of formula (I) 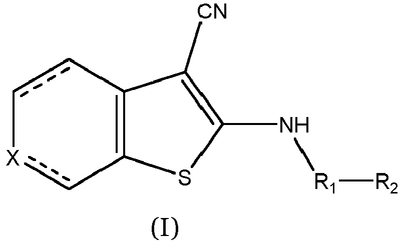 or a pharmaceutically acceptable salt thereof, wherein:
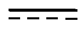 is a single bond or a double bond;
X is -CH₂-, -(CH₂)₂- or -NR₃- when 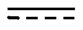 is a single bond;
X is -CH- when 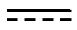 is a double bond;
R1 is a carbonyl or a sulphonyl;
R2 is selected from the group consisting of 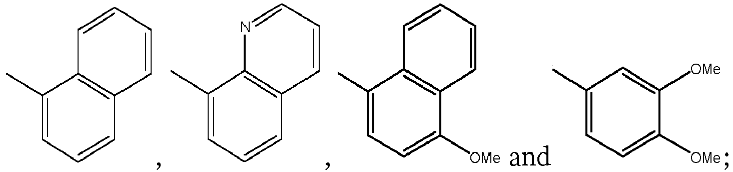
R3 is selected from the group consisting of -H, 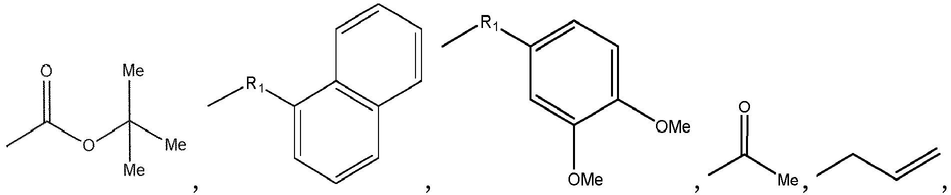 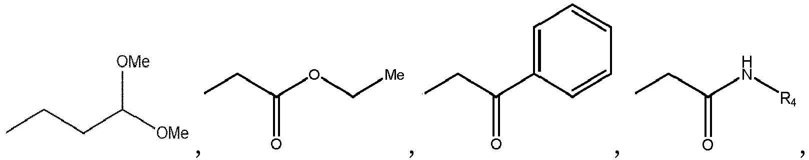 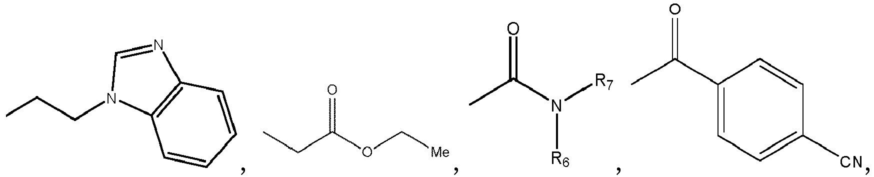 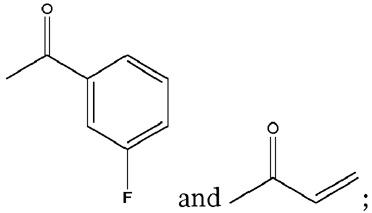
R4 is 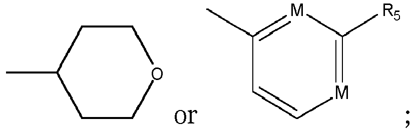
M is N or C;
R5 is -H, a halide or -CN;
R6 is -H, -Me or benzyl; and
R7 is C1-C3 alkyl or an optionally substituted aryl,
provided that it is not a compound according to any one of formulae (II)-(VII):

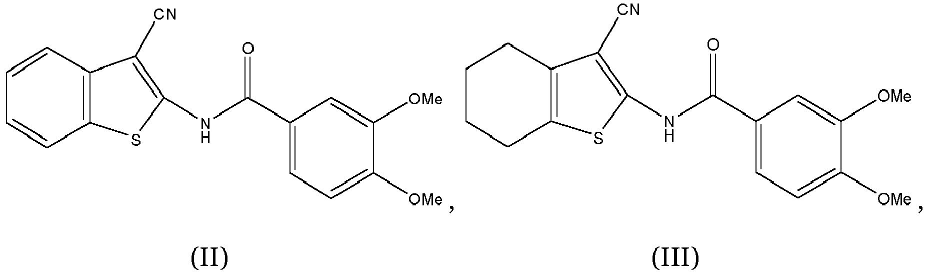

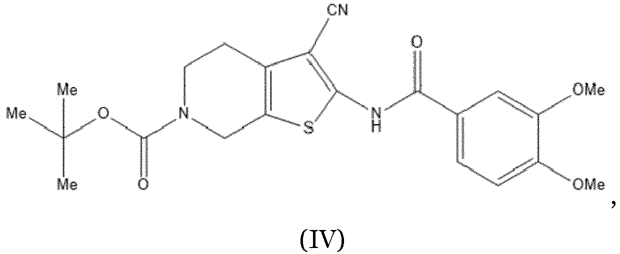

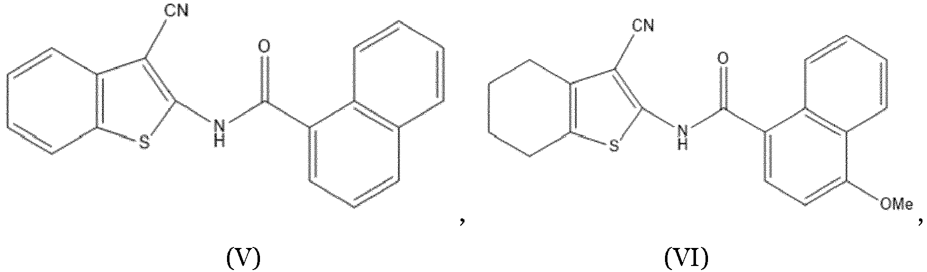

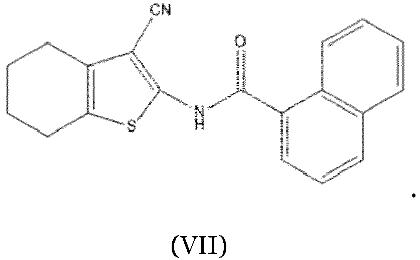

JKN-IN-5A has previously been identified as a drug for use as in the treatment of fatty liver disease, especially non-alcoholic fatty liver disease (NAFLD), due to the molecule's ability to supresses the expression levels of PKLR and lower the triglyceride (TAG) levels. The present inventors have realized that the molecular structure of JNK-IN-5A can be tuned in order to optimize the effect it has on the expression levels of PKLR and on TAG levels. Molecules according to formula (I) were proved to be active.

In one embodiment, R4 is selected form the group consisting of: 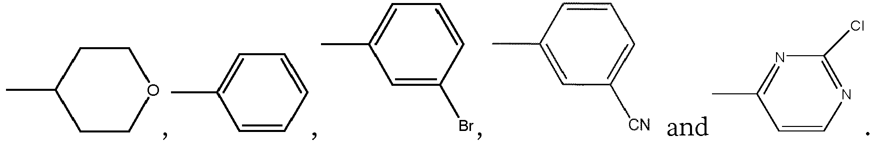 Compounds B130, B164 and B170-172 in the Examples section below are included in this embodiment and shown to be particularly active.

In one embodiment, R3 is selected form the group consisting of: 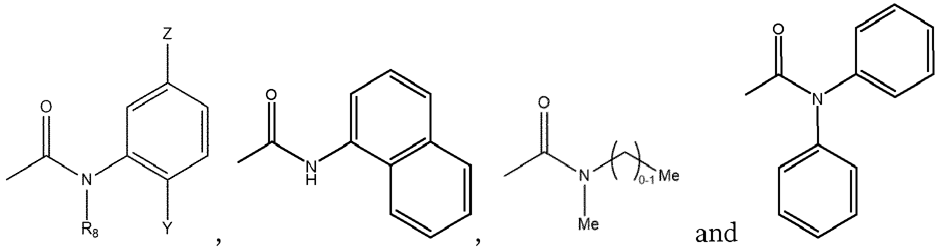 wherein:
Z is selected from the group consisting of -H , -Me and -OMe;
Y is selected from the group consisting of -H, -F, -Br, -Cl, -OMe, -OCF₃, -CF₃, - Me and -NO₂; and
R8 is selected from -H and -Me.

Compounds A117, A119, A151-A154 and A156-A164 in the Examples section below are included in this embodiment and shown to result in particularly low TAG contents.

Compounds A158 and A159 result in exceptionally low cell viability and may hence be particularly suitable for treatment of HCC. Accordingly, it may be preferred that Y is -OCF₃ or -CF₃.

Another compound of particular interest in HCC treatment is B171 shown in the Examples section below.

In one embodiment, Z is selected from the group consisting of -H , -Me and -OMe; Y is selected from the group consisting of -H, -F, -Br, -Cl, -OMe, -Me and -NO₂; and R8 is selected from -H and -Me. This embodiment may be associated with lower toxicity.

In further embodiments:
R1 is a carbonyl;
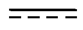 is a single bond and X is -NR₃-; and/or
R2 is

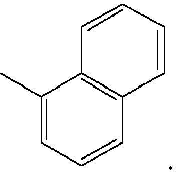

These structural features are found in the most effective compounds of the Examples section below.

The compound may have the formula (VIII)

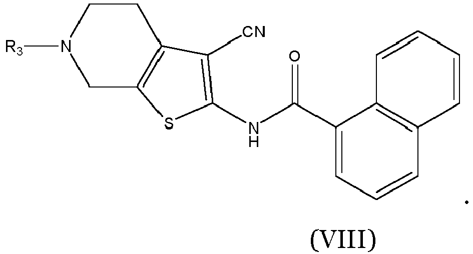

In a preferred embodiment, R3 in formula (VIII) is selected from the group consisting of 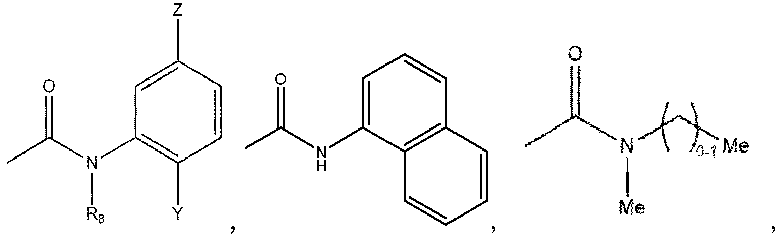 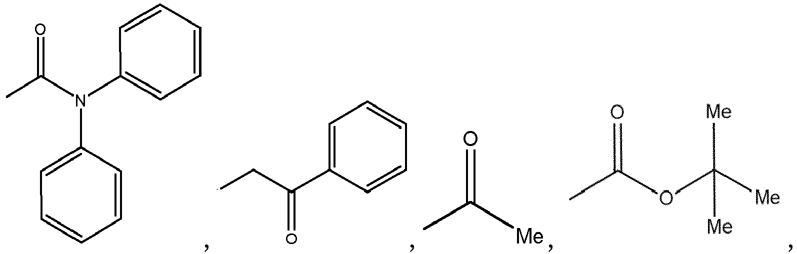 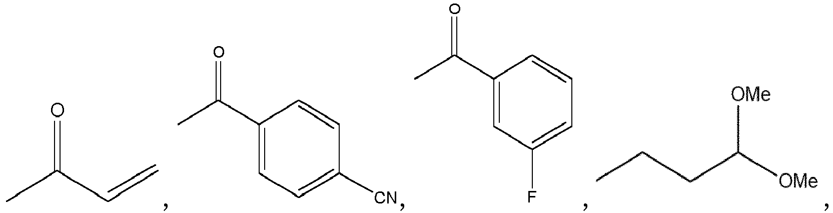 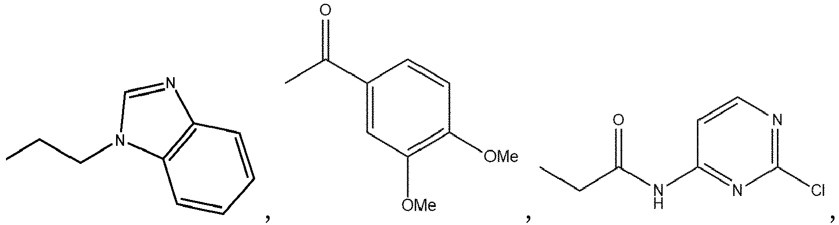 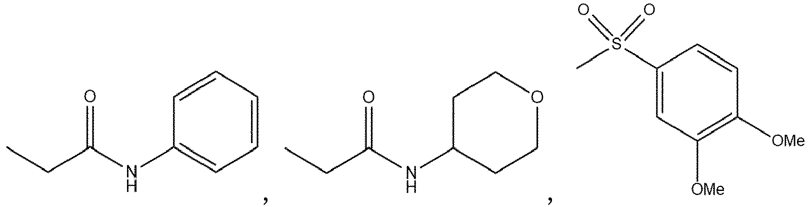 and

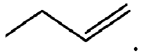

In this embodiment, one of the following applies:
Z is -H, Y is-F, -Br, -Cl, -OMe or -NO₂, and R8 is -H;
Z is -OMe, Y is -H, and R8 is -H;
Z is -Me, Y is -F, and R8 is -H; or
Z is -H, Y is -H, and R8 is -Me.

Compounds A114, A119, A117, A131-A132, A151-A154, A156-A157, A160-A164, A179, A190, A192-A193, B129-B130, B133, B172, B153, B164 and B173 in the Examples section below are included in this embodiment and exhibit a particularly good ratio between TAG content and cell viability which is suitable for the treatment of fatty liver disease.

More preferably, R3 in formula (VIII) is selected from

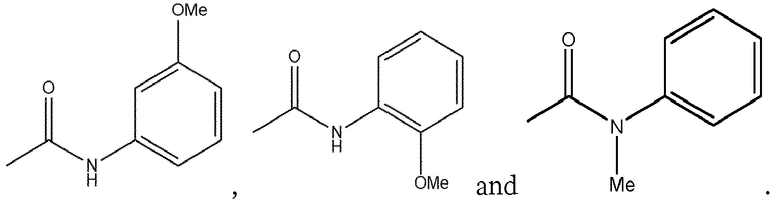

These compounds, A151, A152 and A162 in the Examples section, exhibited a low ratio between TAG content and cell viability making them particularly interesting for treatment of fatty liver disease.

The compound according to the present invention may be used as a medicament.

According to a second aspect, a pharmaceutical composition is provided comprising a compound according to the first aspect.

According to a third aspect, a compound or a pharmaceutical composition according to the first or second aspect used in a method of treatment of fatty liver disease or hepatocellular carcinoma (HCC) is provided. The fatty liver disease is preferably non-alcoholic fatty liver disease (NAFLD), which may have progressed to non-alcoholic steatohepatitis (NASH).

In one embodiment, the method of treatment comprises oral administration of the compound or pharmaceutical composition.

The method of treatment may further comprise detection of overexpression of PKLR in the liver of a subject to be treated prior to administration of the compound to the subject. Overexpression may for example be a higher level of expression than a reference level corresponding to an average level of expression in healthy liver tissue. Alternatively, overexpression may be a higher level of expression than a reference level corresponding to an average level of expression in NAFLD or HCC liver tissue.

The subject of the treatment of the first aspect is preferably a human.

As a fourth aspect of the present disclosure, there is provided a method of treatment of fatty liver disease or HCC in a subject in need thereof, said method comprising administering to the subject a therapeutically effective amount of a compound of formula (I).

The embodiments and examples of the first to third aspect apply to the fourth aspect *mutatis mutandis.*

### BRIEF DESCRIPTION OF THE DRAWINGS

Fig 1 shows a schematic view of the synthesis of N-(3-cyano-4,5,6,7-tetrahydrobenzo[b]thiophen-2-yl)-1-naphthamide analogues.
Fig 2 shows a schematic view of the synthesis of N-(3-cyano-4,5,6,7-tetrahydrobenzo[b]thiophen-2-yl)naphthalene-1-sulfonamide analogues.
Fig 3 shows a schematic view of the synthesis of triazole analogues of N-(3-cyano-4,5,6,7-tetrahydrobenzo[b]thiophen-2-yl)-1-naphthamide.
Fig 4 shows a schematic view of the synthesis of triazole analogues of N-(3-cyano-4,5,6,7-tetrahydrobenzo[b]thiophen-2-yl)naphthalene-1-sulfonamide.
Fig 5 shows a schematic view of the synthesis of 2-amino-4,5,6,7-tetrahydrobenzo[b]thiophene-3-carbonitrile analogues.
Fig 6 shows a schematic view of the synthesis of amide analogues of 2-amino-5,6,7,8-tetrahydro-4H-cyclohepta[b]thiophene-3-carbonitrile.
Fig 7 shows a schematic view of the synthesis of amide analogues of 2-aminobenzo[b]thiophene-3-carbonitrile.
Fig 8 shows a schematic view of the synthesis of analogues of aminothiophene.
Fig 9 shows a schematic view of the synthesis of tert-butyl 2-amino-3-cyano-4,7-dihydrothieno[2,3-c]pyridine-6(5H)-carboxylate analogues.
Fig 10 shows a schematic view of the synthesis of an analogue of N-(3-cyano-4,5,6,7-tetrahydrobenzo[b]thiophen-2-yl)-1-naphthamide with a linker PKL-B52.
Fig 11 shows a schematic view of the synthesis of N-(3-cyano-4,5,6,7-tetrahydrothieno[2,3-c]pyridin-2-yl)-1-naphthamide.
Fig 12 shows a schematic view of the synthesis of Analogues of N-(3-cyano-4,5,6,7-tetrahydrothieno[2,3-c] pyridin-2-yl)-1-naphthamide.
Fig 13 shows a list of the synthesized urea analogues of N-(3-cyano-4,5,6,7-tetrahydrothieno[2,3-c]pyridin-2-yl)-1-naphthamide obtained via isocyanates.
Fig 14 shows a list of the synthesized urea analogues urea analogues of N-(3-cyano-4,5,6,7-tetrahydrothieno[2,3-c]pyridin-2-yl)-1-naphthamide obtained via carbamoyl chlorides.
Fig 15 shows a list of the synthesized amide and sulfonamide analogues of N-(3-cyano-4,5,6,7-tetrahydrothieno[2,3-c]pyridin-2-yl)-1-naphthamide.
Fig 16 shows a list of the synthesized alkyl analogues of N-(3-cyano-4,5,6,7-tetrahydrothieno[2,3-c]pyridin-2-yl)-1-naphthamide.
Fig 17 shows a list of the synthesized heterocyclic ring containing analogues of N-(3-cyano-4,5,6,7-tetrahydrothieno[2,3-c] pyridin-2-yl)-1-naphthamide.
Fig 18 shows the scoring of histopathological and immunohistochemical findings in rats liver tissues.
Fig 19-24 show western blotting of PKLR after 4 days of 10 µM drug treatment. JNK and JNK-5A are abbreviations for JNK-IN-5A in these figures.
Fig 25 shows screening of potential drug candidates. Western blotting of PKLR after 2 respectively 4 days of 10 µM drug treatment. JNK is an abbreviation for JNK-IN-5A in this figure.
Fig 26-34 shows western blotting of PKLR and steatosis related pathway proteins with one week steatosis model. JNK is an abbreviation for JNK-IN-5A in this figure.
Fig 35-41 show the triglyceride level either in percent or O.D at 570nm after one week steatosis model. JNK and JNK-5A are abbreviations for JNK-IN-5A in these figures.
Fig 42-47 show the cell viability after 2 days incubation with 10 µM drug treatment. JNK and JNK-5A are abbreviations for JNK-IN-5A in these figures.
Fig 48 shows the cell viability after 4 days incubation with 10 µM drug treatment. JNK is an abbreviation for JNK-IN-5A in this figure.
Fig 49 shows the cell viability after 1 week steatosis with 10 µM drug treatment. JNK is an abbreviation for JNK-IN-5A in this figure.
Fig 50 shows TAG contents and cell viability after 1 week steatosis with 10 µM drug treatment. JNK is an abbreviation for JNK-IN-5A in this figure. TAG contents are represented by the grey staples, and the cell viabilities are represented by the black staples.
Fig 51 shows the ratio between TAG contents and cell viability. JNK is an abbreviation for JNK-IN-5A in this figure.
Fig 52 shows a schematic view of the JNK activity test using ab273417.
Fig 53 shows the JNK activity using purified JNK1, JNK2 and JNK3.
Fig 54-55 shows the protein-ligand binding results.
Fig 56 shows the ranking of tested target compounds based on cell viability.
Fig 57 shows the ranking of tested target compounds based on TAG contents.
Fig 58 shows the ranking of tested target compounds based on the ratio between TAG contents and cell viability.

### DETAILED DESCRIPTION

JNK-IN-5A has been showed to work as a drug for the treatment of fatty liver disease. However, the molecular structure needs to be tuned in order to optimize the reduction of the expression levels of PKLR and other steatosis related proteins as well as the reduction of triglyceride levels.

Herein, a compound according to the first aspect is shown to provide an improved effect in the reduction of triglyceride levels compared to JNK-IN-5A. The compound according to the first aspect or a composition according to the second aspect may be used in the treatment of fatty liver disease or HCC. The fatty liver disease may be non-alcoholic fatty liver disease (NAFLD) or may have progressed to non-alcoholic steatohepatitis (NASH).

Molecular docking studies have been performed (see Methods - Molecular docking) and based on these, a list of small molecules was synthesized. The activity, triglyceride levels and cell viability of the small molecules was determined.

### EXAMPLES

### Methods

### Rat animal studies

*In vivo biosafety studies.* Healthy Sprague Dawley male rats (12 weeks old, 250-280 g) were obtained from Atatürk University Experimental Research Center (ATADEM, Erzurum Turkey) and housed under standard environmental conditions (temperature 20 °C-25 °C, humidity 50 ± 20% and 12-hour light/dark diurnal cycle). Food and water were available ad libitum. Rats were randomly separated into one control group and two experimental groups (30 mg/kg/day BX-912 or JNK-IN-5A, n=5). BX-912 and JNK-IN-5A were dissolved in DMSO and orally administrated using oral gavage for 7 days. The control group were received the same amount of vehicle solution only.

After 7 days, rats were anaesthetized, and blood samples were collected from the abdominal aorta for biochemical and haematological analysis. The plasma was separated and stored at -80 °C for further analysis. Major organs such as heart, kidney, liver, muscle, adipose, intestine (duodenum, ileum, jejunum), pancreas, colon, and stomach were collected from all the animals for further analysis. Selected organs were fixed in 10% neutral buffered formalin for histopathological examination.

In addition to sampling for biochemical and haematological analysis, one drop of blood samples was obtained from the animals for evaluating micronucleus frequencies as genotoxicity endpoint at the end of the experiments. Three different smears of each rat were prepared using pre-coded and cold slides, then the slides airdried at room temperature, and fixed in ethanol for 10 min, and stained with Giemsa. The frequencies of micronucleated polychromatic erythrocytes (MNPCEs) were analysed according to previously suggested approach using a light microscope. The incidence of MN-PCEs was determined by scoring a total of 3000 PCE per animal and results were expressed as % MN (Solórzano-Meléndez et al., 2021).

*In vivo efficacy studies.* After 1-week acclimation period, rats were randomly separated into two groups: control group (n=5) and high sucrose group (n=20). The control group was fed with a standard diet while the others consumed high sucrose diet (HSD). Sucrose was purchased from Sigma Chemical Co. (St Louis, MO, USA) and supplied at the dose of 10% in the drink water for two weeks.

After 2 weeks, five rats in the HSD group were randomly sacrificed for conformation of NAFLD model. Then, the remaining rats in the HSD group were separated into three sub-groups (n=5): HSD group, HSD plus BX-912 group and HSD plus JNK-IN-5A group. BX-912 and JNK-IN-5A were dissolved in DMSO and were orally administrated using oral gavage for one week (30 mg/kg/day). The HSD group were treated with vehicle solution only.

At the end of the experiment, rats were sacrificed after being anesthetized. Blood samples were collected from the abdominal aorta and centrifuged at 8000 rpm for 15 min at 4 °C. The plasma was separated and stored at -80 °C until further analysis. Other internal organs, including the heart, adipose tissues, liver, kidney, muscle, intestine (duodenum, ileum, jejunum), pancreas, colon and stomach were immediately removed and then snap frozen in liquid nitrogen and stored at -80 °C; the liver was also fixed in 10% formaldehyde for histopathological examination.

The biosafety and efficacy studies were approved by The Ethics Committee of Atatürk University, and all experiments were carried out in accordance with relevant guidelines and regulations for the care and use of laboratory animals. Animal Experiments Local Ethics Committee of Atatürk University approved the experimental procedure described in this study (Approval Date: 27.08.2020; Approval Number: 42190979- 000-E.2000208344).

*Histopathological examination.* The liver tissues were fixed in 10% buffered formaldehyde solution. After fixation, the tissues were passed through graded alcohol and xylene series and embedded in paraffin blocks. 5 micrometer thick sections were taken serially from the paraffin blocks. Histopathological changes were evaluated by performing hematoxylin eosin staining on the sections taken. Sections were evaluated according to histopathological findings as none (-), mild (+), moderate (++) and severe (+++).

*Immunohistochemical examination.* After deparaffinization, the slides were immersed in antigen retrieval solution (pH 6.0) and heated in microwave for 15 min to unmask the antigens. The sections were then dipped in 3% H₂O2 for 10 min to block endogenous peroxidase activity. Protein block was dropped onto the sections, washed with PBS for 10 min. Primary antibodies (8-OH-dG, Cat No: sc-66036, Diluent Ratio: 1/100, Santa Cruz) were prepared and applied according to the usage conditions. Expose mouse and rabbit specific HRP/DAB detection IHC kit were used as follows: sections were incubated with goat anti-mouse antibody, then with streptavidin peroxidase, and finally with 3,3' diamino benzidine + chromogen. Slides were counter stained with hematoxylin. Immunoreactivity in the sections were graded as none (-), mild (+), moderate (++) and severe (+++).

### Cell culture experiments and in vitro steatosis induction

HepG2 wild type cells were purchased from genome engineering company Synthego. Cells were maintained with Roswell Park Memorial Institute growth media, RPMI, 1640 (R2405, Sigma-Aldrich) and supplemented with 10 % fetal bovine serum (FBS, F7524, Sigma-Aldrich), 1 % penicillin - streptomycin (P/S, P4333, Sigma-Aldrich). Steatosis induction media was prepared as high glucose Dulbecco's Modified Eagle Media (DMEM, D0819, Sigma-Aldrich) with 10 % FBS, 1 % P/S media supplemented with 10 µg/ml insulin (I9278, Sigma-Aldrich), and 10 µM T0901317 (T2320, Sigma-Aldrich). HepG2 cells were seeded 6x104 cells per well for 96-well plate and 1x106 cells per well for 6-well plate. HepG2 cells were incubated with steatosis induce media (SM) for one week by 3day + 2day + 2day with total three times media exchange.

### Triglyceride (TAG) measurement assay

HepG2 cells were seeded at 6x104 cells per well into 96 well plate and induced steatosis for one week with 10 µM of target drugs. After steatosis induction, cells were washed with 200 µl of phosphate buffered saline (PBS) and triglyceride contents was measured by Triglyceride Assay Kit - Quantification (ab65336, Abcam) following manufacturer's instruction. Optical density (O.D) values were detected with microplate reader at 570 nm (Hidex Sense Beta Plus).

### MTT assay measurements

The cytotoxic effects of the synthesized drugs on the HepG2 cells were tested by MTT (Thiazolyl Blue Tetrazolium Bromide) method. HepG2 cells were seeded 2x104 cells per well in 96-well plate in 200 µL growth media. After 2 days of cell seeding, 10 µM of the respective target drugs were dissolved in 0.1 % DMSO and 200 µL of the media were treated for two days. After the incubation period, 5 mg/ml MTT (M2128, Sigma-Aldrich) solution in PBS (10 µL) was added to each well. After 1 hr, the MTT solution and all media were removed from the wells. 100 µl of DMSO was added and mixed to dissolve the formazan crystals. Cell viability was analysed by measuring the absorbance of the dissolved formazan in a microplate reader at a wavelength of 570 nm with microplate reader (Hidex Sense Beta Plus).

### Western blot

To test the effect of the synthesized drugs on the expression levels of PKLR and steatosis-related proteins, HepG2 cells were seeded 2.5x105 and 5x105 per well in 6-well plate, respectively. After 4 and 7 days of 10 µM drug treatments for PKLR and steatosis assays, respectively, the cells were washed with PBS solution and then lysed with CelLytic M (C2978, Sigma-Aldrich) lysis buffer containing protease inhibitors (11836170001, Roche). The cell lysates were centrifuged at 12.000 rpm for 10 min and supernatants were collected. Protein Assay Dye Reagent (5000006, Bio-Rad) was used for the determination of protein concentration. The absorbance of the proteins was measured spectrophotometrically at 595 nm by a microplate reader (Hidex Sense Beta Plus). Protein electrophoresis was performed using Mini-PROTEAN^{®} TGXTM Precast Gels (4561086, Bio-Rad) and then the separated proteins were transferred to a Trans-Blot Turbo Mini 0.2 um PVDF Transfer Packs membrane (1704158, Bio-Rad) by using Trans-Blot^{®} TurboTM Transfer System (Bio-Rad). The membrane was blocked with 5 % skim milk for 30 min at 4 °C with gentle rocking. After blocking, the membrane was treated with primary antibodies: Anti-PKLR (HPA006653, Atlas Antibodies, Sigma-Aldrich), Anti-PKM2 (D78A4, Cell Signalling Tech.) Anti-Chrebp (ab92809, Abeam), Anti-Acetyl-CoA Carboxylase (NBP2-55439, Novus Biologicals), Anti-Fatty Acid Synthase (ab22759, Abeam) and Anti-Beta actin (ab8227, Abcam) as a control. The membrane was treated at 4 °C overnight on a rocking platform. After the treatment of primary antibodies, the membrane was washed three times with mixture of tris-buffered saline and Polysorbate 20 (TBS-T buffer, A09-7500-100, Medicago). The membrane was then treated with Goat anti-Rabbit IgG-HRP (ab205718, Abcam) as the secondary antibody. The treatment was carried out at 4 °C for 30 min with gentle rocking. The protein bands on the membrane were revealed using enhanced chemiluminescence substrate (WBLUF0500, Merck) and detected with ImageQuant^{™} LAS 500 (GE Healthcare).

### JNK enzyme activity assay

JNK activity was measured with JNK Activity Assay Kit (ab273417, Abcam). JNK activity assay was performed with purified JNK, JNK1 (PV3319, Thermo Fisher), JNK2 (PV3620, Thermo Fisher) JNK3(PV4563, Thermo Fisher). 0.3 µg of each JNK purified protein were incubated with c-Jun Protein/ATP in Kinase Assay Buffer for 1 hr followed by manufacturer's instruction with 10 µM small molecules. Catalysed substrate Phospho-c Jun (Ser 73) was detected by western blot analysis. Western blot analysis performed with JNK Activity Assay kit supplied anti-Phospho-cJun (Ser 73) Specific Antibody at 1:10,000 for overnight and Goat Anti-Rabbit HRP (ab205718, abcam) at 1:10,000 for 1 hr incubation as primary and secondary antibody.

### Cellular Thermal Shift Assay (CETSA)

HepG2 cells were trypsinized and calculated at 1×10⁶ cells into 1.5 ml micro centrifuge tube with 100 µl cell culture media. Cells were incubated in a CO₂ incubator at 37 °C for 1 hr with 10 µM of each target drug. Drug treated cells were heated at 60 °C for 3 min using a heat plate. Heated cells were lysed with Native lysis buffer (ab156035, Abcam). Thermal shifted JNK proteins were detected with western blot analysis using JNK1+2+3 antibody (Ab179461, Abcam) and band intensity was analysed with ImageJ program.

### Ranking of target molecules

HepG2 cells were seeded at 60,000 cells per well in 96well plate. Using steatosis induce media, the cells were treated with 10µM of target drugs for one week. The media was changed 3 times at day 0, day 3 and day 5. TAG assay was performed using MAK266-1KT SDS Triglyceride Quantification Colorimetric kit and cell viability analysis was performed using MTT assay.

### Molecular docking

Computational docking studies were performed using the MOE v. 2019.01 (Molecular Operating Environment, 2019). The protein structure with the PDB ID code: 2O2U was prepared using the protein preparation module in MOE by the addition of missing atoms and residues, correction of bond order and formal charge and adjustment of tautomer (Angell et al., 2007). System was protonated using the protonate 3D algorithm and generalized Born volume integral (GB/VI) was used as the electrostatics function, with a value of 80 as dielectric constant. The electrostatics and van der Waals cut off were set as 10 and 15 Å, respectively. Protein was charged and minimized with the application of AMBER10:EHT force field implemented in a MOE software.

All of the compounds were built using ChemBioDraw Ultra 14.0, charged and minimized by MMFF94x force field, along with the adjustment of hydrogens and lone pairs (Halgren et al., 1996). The dataset was subjected to energy minimization using default RMS gradient of 0.1 kcal/mol/Å2. Following protonation and minimization, the compounds were saved in the MOE database format.

The active site was defined to include all atoms within 5 Å of cognate ligand. Before execution of docking, benchmarking of different combination of scoring and placement methods in MOE Dock was performed to find the most suitable combination of algorithm and scoring functions for our target protein. In this study, induced fit docking procedure along with Triangle Matcher algorithm as placement method and LondonDG as initial scoring and GBVI/WSA dG as re-scoring method presented the most reliable results. For each compound, 10 individual docking runs were conducted and 100 conformations were generated. The bestranked solution of each compound was further assessed for protein-ligand binding analysis.

### Synthesis procedure of target compounds

### General synthesis procedure

All reactions were performed using oven-dried glassware and under an inert atmosphere (nitrogen) unless otherwise stated. All reagents and dry solvents were obtained from the commercial supplier Sigma-Aldrich and used without further purification. Organic solutions were concentrated under reduced pressure on a Heidolph rotary evaporator. Reactions were monitored by liquid chromatographymass spectrometry (LC-MS, Thermo Fisher TSQ Series, Athena C18-WP,100 Å, 2.1×50 mm, 3 µm); Water:MeOH (0.01 formic acid)) or by Thin-Layer Chromatography (TLC) using silica gel pre-coated aluminium plates (Kieselgel 60, 254, E. Merck, Germany). The chromatograms were visualized using ultraviolet light (254 and 366 nm) and stained with vanillin dips or aqueous potassium permanganate solution. ¹H NMR spectra were recorded on Advance Bruker 500 MHz spectrometer in CDCl₃ and DMSO-d6. ¹³C NMR spectra were recorded in deuterated solvents on Bruker spectrometer at 126 MHz, with the central peak of the deuterated solvent as the internal standard. The ¹H NMR spectra are reported as δ/ppm downfield from tetramethyl silane (multiplicity, number of protons, coupling constant J/Hz). The ¹³C NMR spectra are reported as δ/ppm. All chemical shifts are reported in parts per million (ppm) relative to the residual solvent peak. The following abbreviations are used to denote signal patterns: (s) singlet, (d) doublet, (t) triplet, (q) quartet, (m) multiplet, and (br) broad unless otherwise stated. Flash-column chromatography was performed on PuriFlash XS 520 Plus Flash chromatography system (Interchim) with built-in UV-detector, ELSD-detector and fraction collector with Interchim silica gel columns.

### Amines and acyl chlorides reactions (General procedure A)

To a solution of the selected amine in acetonitrile (0.1 M), triethylamine (1.2 equiv.) and the selected acylchloride (1 to 1.2 equiv.) was added. The reaction mixture was stirred at room temperature for 1 h, concentrated, mounted on silica and purified by flash chromatography to afford the desired amides.

### Amide coupling (General procedure B)

To a solution of carboxylic acid (1 equiv.), 1-Ethyl-3-(3-dimethylaminopropyl)carbodiimide (EDCl, 1.2 equiv.) and HOBt (1.2 equiv.) in DMF (0.1-0.3 M) and triethylamine or DIPEA (2 equiv.) were added at 0°C. The mixture was stirred for 10 min at room temperature. Then the desired amine (1.2 equiv.) was added, and the reaction mixture was stirred at room temperature for 6-16 h. The residue was poured onto ice and then extracted with ethyl acetate. The combined organic extracts were washed with brine, dried over magnesium sulphate and evaporated to dryness under reduced pressure, mounted on silica and purified by flash chromatography to afford the desired product.

### Ester hydrolysis (General procedure C)

To a solution of the selected ethyl ester in water/THF (1:1, 0.1 M), LiOH (2-3 equiv.) was added. The reaction mixture was stirred at room temperature for 1 h. Reaction mixtures were concentrated to approximately half their volume on a rotavap, acidified to pH 1-2 by the addition of HCl (aq.,1 M), and filtered to afford the desired carboxylic acids. Compounds were dissolved, mounted on silica and purified by flash chromatography to afford the desired products.

### Alkylation of secondary amines (General procedure D)

A solution of secondary amine (1 equiv.), potassium carbonate (1.2 equiv.) and TBAB (0.01 equiv.) in THF or DMF was stirred for 30 min at room temperature. Then Alkyl Bromide (2-3 equiv.) was added, and the reaction mixture was stirred at room temperature for 48 h. The residue was partitioned between water and ethyl acetate and the aqueous phase was extracted with further ethyl acetate. The combined organic extracts were washed with brine, dried over magnesium sulphate and evaporated to dryness under reduced pressure to afford the desired product.

### Azide preparation (General procedure E)

A solution of alkyl bromide (1 equiv.) and sodium azide (2 equiv.) in EtOH/H2O (2:1) was stirred at room temperature for 2h. The mixture was evaporated under reduced pressure then extracted with diethyl ether and water. The combined organic extracts were dried over magnesium sulphate and evaporated to dryness under reduced pressure to give the desired product.

### Copper(I)-catalyzed alkyne-azide cycloaddition (CuAAC) reactions (General procedure F)

To a solution of alkyne (1 equiv.) and copper sulphate (0.75 equiv.) in dichloromethane (DCM)/H₂O (2:1), sodium ascorbate (0.25 equiv.) was added. The mixture was stirred at room temperature for 20 min. Then appropriate azide (1.5 equiv.) was added and reaction was stirred for further 6-16 h. The mixture was then extracted with DCM and water. The combined organic extracts were dried over magnesium sulphate and evaporated to dryness under reduced pressure to yield the desired products. Compounds were purified by flash chromatography.

### Boc group deprotection (General procedure G)

To a solution of protected amine in DCM, trifluoroacetic acid (TFA) (DCM: TFA 4:1) was added in the presence of nitrogen. The mixture was stirred at room temperature for 1 hour. The reaction progress and results were confirmed by TLC and LC/MS. When the reaction was completed, after concentration under reduced pressure, ether or ethanol was added and crystallized to obtain the target compounds.

### Synthesis of target compounds

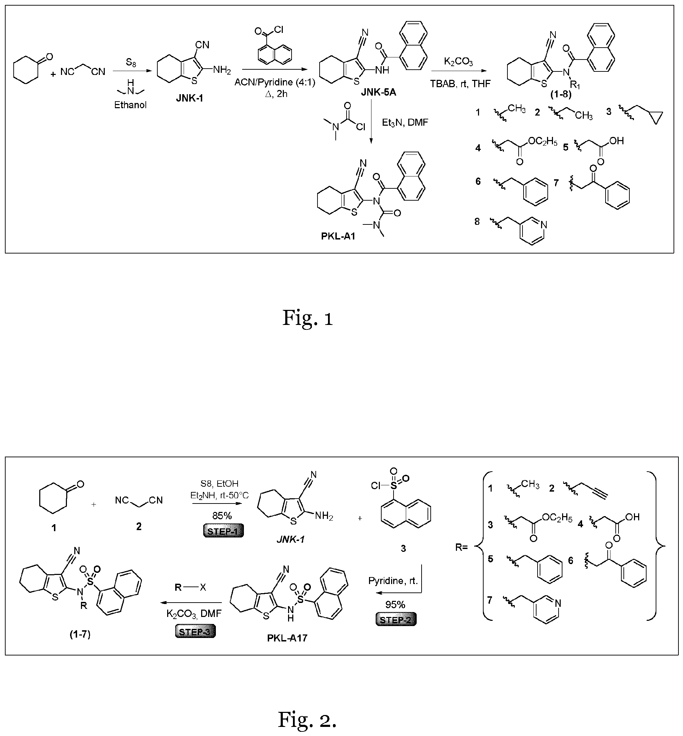

A schematic view of the synthesis of N-(3-cyano-4,5,6,7-tetrahydrobenzo[b]thiophen-2-yl)-1-naphthamide analogues can be seen in Fig 1 and the synthesis procedure for the specific compounds follows below.

### Synthesis of 2-amino-4,5,6,7-tetrahydrobenzofblthio-phene-3-carbonitrile (H):

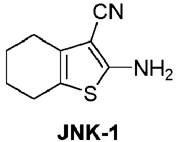

The compound was synthesized according to the literature procedure [4]. To a stirred solution of cyclohexanone (10.4 ml, 0.10 mol) in ethanol (300 ml) were added sulphur (3.52 g, 0.11 mol), malononitrile (6.60 g, 0.10 mol) and diethylamine (10.30 ml, 0.10 mol). The reaction mixture was heated at 50 °C for 3 hours. The mixture was allowed to cool to room temperature and the solvent was removed under reduced pressure. The residue was partitioned between water and ethyl acetate and the aqueous phase was extracted with additional ethyl acetate. The combined organic extracts were washed with brine, dried over magnesium sulphate and evaporated to dryness under reduced pressure to give the desired product (16.7g, 94%) as a brown solid.

### Synthesis ofN-(3-cyano-4,5,6,7-tetrahydrobenzo[b]thiophen-2-yl)-1-naphthamide (JNK-5A)

A solution of (aminothiophene, JNK-1) (1 g, 5.61 mmol) in ACN (5 ml) with 1-naphthoyl chloride (1 mL, 6.73 mmol) was stirred together at 80°C under an inert atmosphere for 2 h. The reaction was monitored by TLC and LCMS/MS. The mixture was then evaporated to dryness and chromatographed over silica gel using a gradient of hexane/EtOAc (9:1 to 8:2). The product fractions were pooled and evaporated to afford the title compound.

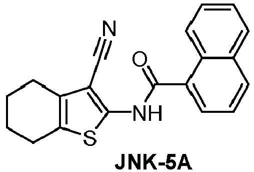

### Synthesis of N-(3-cyano-4,5,6,7-tetrahydrobenzo[b]thiophen-2-yl)-N-(prop-2-yn-1-yl)-1-naphthamide (PKL-A65)

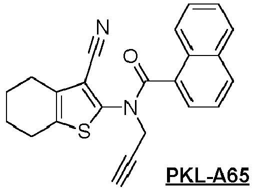

Following general procedure D, JNK-5A (500 mg, 1.5 mmol), was reacted with propargyl bromide solution (0.013ml, 1.8 mmol), to afford the title compound as a yellow liquid after column chromatography (EtOAc in Hexane : 0-10%).

¹H NMR (500 MHz, CDCl₃) δ 7.98 (d, J = 8.4 Hz, 1H), 7.75 (d, J = 7.2 Hz, 3H), 7.48 (dt, J = 34.2, 7.3 Hz, 3H), 7.32 (s, 1H), 4.72 (s, 0H), 2.42 (s, 4H), 2.33 (s, 1H), 1.65 (s, 4H). ¹³C NMR (126 MHz, CDCl₃) δ 135.16, 133.23, 132.31, 129.26, 127.20, 126.29, 125.57, 124.99, 124.75, 124.14, 124.05, 123.54, 112.27, 73.22, 66.95, 28.67, 23.50, 23.13, 21.62, 20.66, 18.71.

### Synthesis of Ethyl 2-(N-(3-cyano-4,5,6,7-tetrahydrobenzo[b]thiophen-2-yl)-1-naphthamido) acetate (PKL-A3)

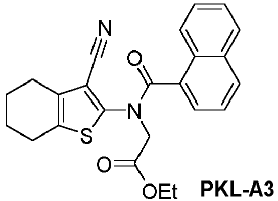

Following general procedure D, JNK-5A (100 mg, 0.30 mmol) reacted with ethyl bromoacetate solution (0.040 ml, 0.36 mmol) to afford 90 mg (71 %) of the title compound as a transparent liquid after column chromatography (EtOAc in Hexane: 0-20%).

¹H NMR (500 MHz, CDCl₃) δ 8.12 (d, *J* = 8.5 Hz, 1H), 7.76 - 7.69 (m, 2H), 7.52 (t, *J* = 7.7 Hz, 1H), 7.44 (dt, *J* = 15.1, 7.3 Hz, 2H), 7.29 (t, *J* = 7.8 Hz, 1H), 4.63 (d, *J* = 6.2 Hz, 2H), 4.26 (q, *J* = 7.3 Hz, 2H), 2.34 (d, *J* = 35.6 Hz, 3H), 1.30 (t, *J* = 7.2 Hz, 2H). 13C NMR (126 MHz, CDCl₃) δ 168.32, 150.31, 136.38, 133.84, 133.26, 132.33, 130.28, 130.16, 128.04, 127.23, 126.54, 125.63, 124.91, 124.50, 113.22, 108.87, 68.22, 61.91, 61.07, 52.11, 24.37, 24.06, 22.59, 21.66, 14.20.

### Synthesis of N-benzyl-N-[3-cyano-4,5,6,7-tetrahydrobenzo[b]thiophen-2-yl)-i-naphthamide (PKL-A4)

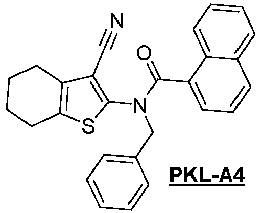

Following general procedure D, JNK-5A (100 mg, 0.30 mmol) reacted with benzyl bromide (0.35 ml, 0.36 mmol) to afford 116 mg (91 %) of the title compound as a transparent liquid after column chromatography (EtOAc in Hexane: 0-20%).

¹H NMR (500 MHz, CDCl₃) δ 8.00 (d, J = 8.5 Hz, 1H), 7.79 - 7.69 (m, 2H), 7.55 - 7.39 (m, 4H), 7.32 - 7.22 (m, 5H), 5.17 (s, 2H), 2.32 (s, 4H), 1.59 (s, 4H). ¹³C NMR (126 MHz, CDCl3) δ 135.48, 133.96, 133.37, 132.89, 130.05, 128.81, 128.71, 128.30, 128.24, 128.15, 127.22, 126.52, 125.23, 125.06, 124.55, 29.71, 24.39, 24.09, 22.63, 21.67.

### Synthesis of 2-(N-(3-cyano-4,5,6,7-tetrahydrobenzo[b]thiophen-2-yl)-1-naphthamidolacetic acid (PKL-A2)

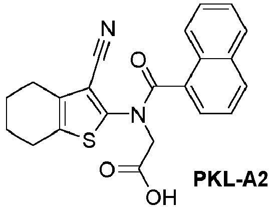

Following general procedure C, JNK-5A (350 mg, 0.83 mmol reacted with lithium hydroxide (70.19 mg, 1.67 mmol) to afford 255 mg (78 %) of the title compound as a yellow solid after column chromatography (EtOAc in Hexane: 0-20%).

¹H NMR (500 MHz, CDCl₃) δ 8.08 (d, *J* = 8.4 Hz, 1H), 7.79 - 7.69 (m, 3H), 7.47 (ddd, *J* = 27.0, 15.1, 7.2 Hz, 5H), 7.34 - 7.25 (m, 2H), 2.38 (s, 2H), 2.32 (s, 2H), 1.60 (s, 4H). ¹³C NMR (126 MHz, CDCl₃) δ 176.25, 172.48, 135.50, 132.93, 132.23, 131.00, 129.27, 127.04, 126.27, 125.55, 124.49, 124.04, 123.45, 50.89, 28.68, 23.36, 23.03, 21.55, 20.62, 19.73.

### Synthesis of N-(3-cyano-4,5,6,7-tetrahydrobenzo[b]thiophen-2-yl)-N-(dimethylcarbamoyl)-1-naphthamide (PKL-A1)

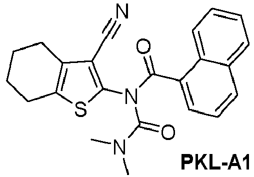

Following general procedure A, JNK-5A (100 mg, 0.30 mmol) reacted with N-methycarbamoyl chloride (29.4 ul, 0.30 mmol) to afford 90 mg (74 %) of the title compound as yellow crystals after column chromatography (EtOAc in Hexane: 0-20%).

¹H NMR (500 MHz, CDCl₃) δ 9.15 (d, *J* = 8.7 Hz, 1H), 7.90 (d, *J* = 8.1 Hz, 1H), 7.84 - 7.75 (m, 2H), 7.62 (dd, *J* = 8.6, 6.9 Hz, 1H), 7.44 (dt, *J* = 29.1, 7.6 Hz, 2H), 3.25 (s, 3H), 2.89 (s, 3H), 2.65 (dt, *J* = 16.3, 5.6 Hz, 5H), 1.80 (q, *J* = 7.5 Hz, 4H). ¹³C NMR (126 MHz, CDCl₃) δ 151.30, 150.33, 149.50, 136.08, 134.16, 134.10, 132.87, 130.96, 129.10, 128.57, 128.54, 128.08, 126.56, 126.35, 124.51, 114.71, 109.77, 37.07, 36.88, 25.01, 24.18, 23.07, 22.12.

### Synthesis of N-(3-cyano-4,5,6,7-tetrahydrobenzo[b]thiophen-2-yl)-N-(2-oxo-2-phenylethyl)-1-naphthamide (PKL-B26)

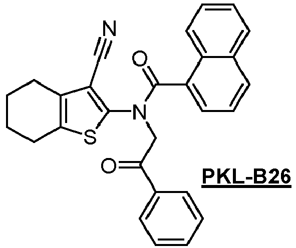

Following general procedure D, JNK-5A (100mg, 0.30 mmol), reacted with 2-Bromoacetophenone (0.35 ml, 0.36 mmol) to afford 85 mg (63 %) of the title compound as a liquid after column chromatography (EtOAc in Hexane: 0-20%).

¹H NMR (500 MHz, CDCl₃) δ 8.27 (d, *J* = 8.4 Hz, 1H), 7.98 (d, *J* = 7.7 Hz, 2H), 7.71 (dd, *J* = 8.2, 4.0 Hz, 2H), 7.60 - 7.50 (m, 3H), 7.44 (dt, *J* = 8.1, 4.1 Hz, 3H), 7.29 (dd, *J* = 8.2, 7.1 Hz, 1H), 5.38 (s, 2H), 2.35 (s, 3H), 2.26 (s, 3H), 1.55 (p, *J* = 3.2 Hz, 4H). ¹³C NMR (126 MHz, CDCl₃) δ 191.36, 170.11, 149.85, 135.59, 133.56, 133.08, 132.52, 132.25, 131.62, 129.35, 129.00, 127.89, 127.14, 126.93, 126.26, 125.50, 124.88, 123.85, 123.47, 112.47, 107.71, 55.99, 23.28, 22.98, 21.66, 21.63, 21.52, 20.63.

### Synthesis of N-(3-cyano-4,5,6,7-tetrahydrobenzo[b]thiophen-2-yl)-N-(2-oxo-2-phenylethyl)-1-naphthamide(PKL-B29)

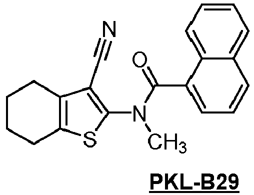

Following general procedure D, JNK-5A (100 mg, 0.30 mmol), reacted with iodomethane (22.47 µL, 0.36 mmol) to afford 86 mg (83 %) of the title compound as an oil after column chromatography (EtOAc in Hexane: 0-20%).

### Synthesis of N-(3-cyano-4,5,6,7-tetrahydrobenzo[b]thiophen-2-yl)-N-ethyl-1-naphthamide(PKL-B30)

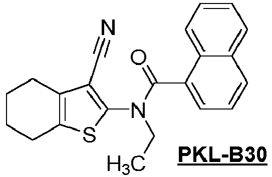

Following general procedure D, JNK-5A (100 mg, 0.30 mmol), reacted with iodoethane (26.76 µL, 0.36 mmol) to afford 70 mg (65 %) of the title compound as an oil after column chromatography (EtOAc in Hexane : 0-20%).

### Synthesis of N-(3-cyano-4,5,6,7-tetrahydrobenzo[b]thiophen-2-yl)-N-(pyridin-3-ylmethyl)-1-naphthamide (PKL-B32)

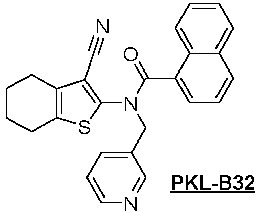

Following general procedure D, JNK-5A (100 mg, 0.30 mmol), reacted with 3-(chloromethyl) pyridine hydrochloride (42.21 mg, 0.36 mmol) to afford 70 mg (55 %) of the title compound as an oil after column chromatography (EtOAc in Hexane: 0-20%).

¹H NMR (500 MHz, CDCl₃) δ 8.52 (d, *J* = 4.9 Hz, 1H), 8.48 - 8.41 (m, 1H), 7.95 (d, *J* = 8.4 Hz, 1H), 7.87 - 7.82 (m, 1H), 7.74 (d, *J* = 8.4 Hz, 2H), 7.50 (t, *J* = 7.7 Hz, 1H), 7.43 (t, *J* = 7.4 Hz, 2H), 7.28 (s, 2H), 5.16 (s, 2H), 2.32 (d, *J* = 18.9 Hz, 5H), 1.60 (s, 5H). ¹³C NMR (126 MHz, CDCl₃) δ 170.75, 150.11, 149.70, 137.01, 135.97, 134.29, 134.27, 133.37, 132.45, 131.63, 130.31, 130.00, 128.30, 127.34, 126.60, 125.21, 125.12, 124.51, 123.85, 113.16, 109.10, 31.89, 24.40, 24.08, 22.70, 22.54.

A schematic view of the synthesis of N-(3-cyano-4,5,6,7-tetrahydrobenzo[b]thiophen-2-yl)naphthalene-1-sulfonamide analogues can be seen in Fig. 2 and the synthesis procedure for the specific compounds follows below.

### Synthesis of N-(3-cyano-4,5,6,7-tetrahydrobenzo[b]thiophen-2-yl)naphthalene-1-sulfonamide (PKL-A17)

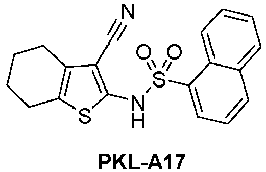

An oven-dried 100 mL round-bottom flask was charged with intermediate JNK-1 (1 eq), 1-sulfonapthoyl chloride (1.2 equiv.) and pyridine as solvent and stirred under an N₂ atmosphere for 12 h. Reaction was monitored by TLC analysis. The solvent was removed under reduced pressure when the reaction was finished. Purification was performed by silica column chromatography (hand column) over silica gel eluting with a gradient of hexane/EtOAc (8:2 to 7:3). The product fractions were pooled and evaporated to afford the title compound with a yield of 80%.

¹H NMR (500 MHz, CDCl₃) δ 7.74 (d, J = 8.4 Hz, 1H), 7.37 (d, J = 8.2 Hz, 1H), 7.21 (dd, J = 5.3, 7.8 Hz, 2H), 6.81 (dt, J = 7.2, 22.9 Hz, 2H), 6.73 (t, J = 7.8 Hz, 1H), 1.62 - 1.54 (m, 4H), 0.74 (dq, J = 5.7, 6.6,11.3 Hz, 4H). ¹³C NMR (126 MHz, CDCl₃) δ 140.19, 139.08, 138.95, 138.25, 135.18, 134.33, 133.45, 132.75, 132.32, 129.76, 129.51, 129.48, 118.46, 29.02, 28.86, 27.54, 26.60.

### Synthesis of N-(3-cyano-4,5,6,7-tetrahydrobenzo[b]thiophen-2-yl)-N-(prop-2-yn-1-yl)naphthalene-1-sulfonamide (PKL-A53)

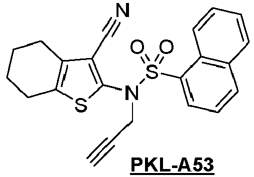

Potassium carbonate (514 mg,3.72 mmol) and propargyl bromide (0.754 mL, 7.44 mmol) was added to a solution of PKL-A17 (207 mg,0.744 mmol) in DMF (0.07 M) in an oven dried round bottom flask. The mixture was heated to 60 °C and the reaction mixture was poured into ice water. After cooling to room temperature, the mixture was extracted with EtOAc (3 times) and the combined organic layers were washed with 1 N HCl (1 time) and 1 M LiCl (3 times). The mixture was dried over Na₂SO₄ and concentrated. The residue was purified by column chromatography using silica and a gradient of hexane/EtOAc (3:1 to 2:1) to obtain the desired product.

¹H NMR (500 MHz, DMSO) δ 8.43 (d, J = 8.6 Hz, 2H), 8.27 (dd, J = 1.1, 7.5 Hz, 1H), 8.21 - 8.17 (m, 1H), 7.78 - 7.70 (m, 3H), 4.63 (d, J = 2.4 Hz, 2H), 3.41 (d, J = 2.6 Hz, 1H), 2.69 (t, J = 5.4 Hz, 2H), 2.53 (d, J = 5.1 Hz, 2H), 1.80 (dd, J = 4.1, 7.0 Hz, 4H). ¹³C NMR (126 MHz, DMSO) δ 162.79, 144.93, 138.29, 136.47, 134.42, 134.21, 132.07, 132.01, 129.63, 128.91, 128.53, 127.67, 125.10, 124.71, 113.04, 111.62, 78.47, 77.75, 42.91, 24.67, 24.12, 22.73, 21.85.

### Synthesis of ethyl N-(3-cyano-4,5,6,7-tetrahydrobenzo[b]thiophen-2-yl)-N-(naphthalen-1-ylsulfonyl) glycinate (PKL-A43)

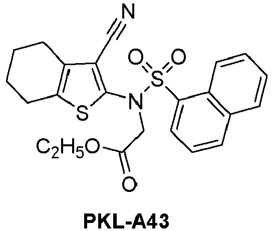

Potassium carbonate (45 mg,0.32 mmol) ethyl bromoacetate solution (0.0374 mL, 0.32 mmol) and TBAB as a Catalyst (17 mg, 0.05 mmol) were added to a solution of JNK-1 (100 mg,0.27 mmol) in 7 ml dry THF in an oven dried round bottom flask. The reaction mixture is stirred overnight. The reaction mixture was then poured into ice water. After cooling to room temperature, the mixture was extracted with EtOAc (3 times). The EtOAc layer was dried over Na₂SO₄ and concentrated. The residue was purified by column chromatography using silica to obtain the title compound.

¹H NMR (500 MHz, DMSO) δ 8.40 (t, J = 7.9 Hz, 2H), 8.24 (dd, J = 1.1, 7.5 Hz, 1H), 8.18 (dd, J = 1.7, 7.8 Hz, 1H), 7.76 - 7.68 (m, 3H), 4.67 (s, 2H), 4.04 (q, J = 7.1 Hz, 2H), 2.75 - 2.64 (m, 2H), 2.50 - 2.42 (m, 2H), 1.86 - 1.73 (m, 4H), 1.13 (t, J = 7.1 Hz, 3H). ¹³C NMR (126 MHz, DMSO) δ 168.10, 145.87, 138.14, 136.25, 134.54, 134-04, 132.88, 131.56, 129.60, 128.79, 128.60, 127.54, 125.09, 124.65, 112.78, 111.32, 61.68, 55.38, 53.82, 24.58, 24.08, 22.75, 21.86, 14.21.

### Synthesis of N-(3-cyano-4,5,6,7-tetrahydrobenzo[b]thiophen-2-yl)-N-methylnaphthalene-i-sulfon- amide (PKL-A66)

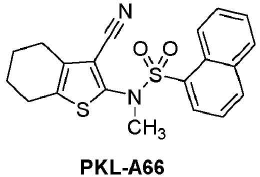

Potassium carbonate (514 mg ,3.72 mmol) and methyl iodide (0.754 mL, 7.44 mmol) was added to a solution of JNK-1 (207 mg,0.744 mmol) in DMF (0.07 M) in an oven dried round bottom flask. The reaction mixture was stirred for 30 min and poured into ice water. After cooling to room temperature, the mixture was extracted with EtOAc (3 times) and the combined organic layers were washed with 1 N HCl (1 time) and 1 M LiCl (3 times). The mixture was dried over Na₂SO₄ and concentrated. The residue was purified by column chromatography using silica to obtain title compound.

¹H NMR (500 MHz, CDCl₃) δ 8.48 (dd, J = 3.6, 6.9 Hz, 1H), 8.18 (dd, J = 1.6, 7.5 Hz, 1H), 8.07 (d, J = 8.3 Hz, 1H), 7.89 - 7.84 (m, 1H), 7.55 - 7.46 (m, 3H), 3.28 - 3.22 (m, 3H), 2.58 (t, J = 6.1 Hz, 2H), 2.44 - 2.37 (m, 2H), 1.75 (tt, J = 5.8, 14.3 Hz, 4H). ¹³C NMR (126 MHz, CDCl₃) δ 135.07, 134.49, 133.43, 133.37, 130.68, 130.42, 128.07, 127.83, 127.20, 126.03, 123.99, 123.09, 39.27, 23.73, 23.13, 21.84, 20.87.

### Synthesis of N-benzyl-N-(3-cyano-4,5,6,7-tetrahydrobenzo[b]thiophen-2-yl)naphthalene-1-sulfon-amide (PKL-A44)

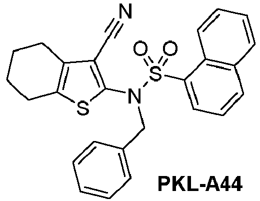

Potassium carbonate (514 mg,3.72 mmol) and benzyl bromide (0.754 mL, 7.44 mmol) were added to a solution of JNK-1 (207 mg,0.744 mmol) in DMF (0.07 M) in an oven dried round bottom flask. The reaction mixture was stirred for overnight and poured into ice water. After cooling to room temperature, the mixture was extracted with EtOAc (3 times) and the combined organic layers were washed with 1 N HCl (1 time) and 1 M LiCl (3 times). The mixture was dried over Na₂SO₄ and concentrated. The residue was purified by column chromatography using silica to obtain title compound.

¹H NMR (500 MHz, DMSO) δ 8.46 (dd, J = 8.4, 15.6 Hz, 2H), 8.34 (dd, J = 1.1, 7.4 Hz, 1H), 8.20 (dd, J = 1.4, 8.2 Hz, 1H), 7.82 - 7.68 (m, 3H), 7.39 - 7.30 (m, 3H), 7.26 (dd, J = 1.9, 7.6 Hz, 2H), 4.84 (s, 2H), 2.61 - 2.57 (m, 2H), 2.42 (t, J = 5.5 Hz, 2H), 1.74 (p, J = 6.1 Hz, 4H). ¹³C NMR (126 MHz, DMSO-H6) δ 170.82, 145.85, 137.48, 136.35, 135.14, 134.50, 134.25, 132.06, 131.93, 129.65, 128.99, 128.81, 128.71, 128.67, 128.62, 128.50, 127.68, 127.08, 126.88, 125.26, 124.71, 113.19, 111.28, 60.23, 56.48, 24.55, 24.05, 22.67, 21.75, 21.23, 14.55.

### Synthesis of N-(3-cyano-4,5,6,7-tetrahydrobenzo[b]thiophen-2-yl)-N-(2-oxo-2-phenylethyl) naphthalene-1-sulfonamide (PKL-A61)

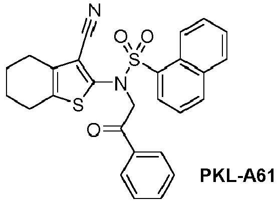

Potassium carbonate (514 mg,3.72 mmol) and 2-bromoacetophenone (0.754 mL, 7.44 mmol) were added to a solution of JNK-1 (207 mg,0.744 mmol) in DMF (0.07 M) in an oven dried round bottom flask. The reaction mixture was stirred for 30 min and poured into ice water. After cooling to room temperature, the mixture was extracted with EtOAc (3 times) and the combined organic layers were washed with 1 N HCl (1 time) and 1 M LiCl (3 times). The mixture was dried over Na₂SO₄ and concentrated. The residue was purified by column chromatography using silica to obtain title compound.

¹H NMR (500 MHz, CDCl₃) δ 8.27 (d, *J* = 8.4 Hz, 1H), 7.98 (d, *J* = 7.7 Hz, 2H), 7.71 (dd, *J* = 8.2, 4.0 Hz, 2H), 7.60 - 7.50 (m, 3H), 7.44 (dt, *J* = 8.1, 4.1 Hz, 3H), 7.29 (dd, *J* = 8.2, 7.1 Hz, 1H), 5.38 (s, 2H), 2.35 (s, 3H), 2.26 (s, 3H), 1.55 (p, *J* = 3.2 Hz, 4H). ¹³C NMR (126 MHz, CDCl₃) δ 191.36, 170.11, 149.85, 135.59, 133.56, 133.08, 132.52, 132.25, 131.62, 129.35, 129.00, 127.89, 127.14, 126.93, 126.26, 125.50, 124.88, 123.85, 123.47, 112.47, 107.71, 55.99, 23.28, 22.98, 21.66, 21.63, 21.52, 20.63.

### Synthesis of N-(3-cyano-4,5,6,7-tetrahydrobenzo[b]thiophen-2-yl)-N-(pyridin-3-ylmethyl) naphthalene-1-sulfonamide (PKL-A58)

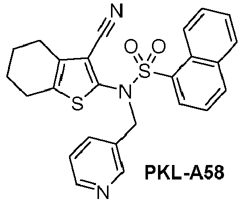

Potassium carbonate (514 mg, 3.72 mmol) and 2-chloropyridine (0.754 mL, 7.44 mmol) were added to a solution of JNK-1 (207 mg, 0.744 mmol) in DMF (0.07 M) in an oven dried round bottom flask. The reaction mixture was stirred for 30 min and poured into ice water. After cooling to room temperature, the mixture was extracted with EtOAc (3 times) and the combined organic layers were washed with 1 N HCl (1 time) and 1 M LiCl (3 times). The mixture was dried over Na₂SO₄ and concentrated. The residue was purified by column chromatography using silica to obtain title compound.

¹H NMR (500 MHz, CDCl₃) δ 8.52 (d, *J* = 4.9 Hz, 1H), 8.48 - 8.41 (m, 1H), 7.95 (d, *J* = 8.4 Hz, 1H), 7.87 - 7.82 (m, 1H), 7.74 (d, *J* = 8.4 Hz, 2H), 7.50 (t, *J* = 7.7 Hz, 1H), 7.43 (t, *J* = 7.4 Hz, 2H), 7.28 (s, 2H), 5.16 (s, 2H), 2.32 (d, *J* = 18.9 Hz, 5H), 1.60 (s, 5H). ¹³C NMR (126 MHz, CDCl₃) δ 170.75, 150.11, 149.70, 137.01, 135.97, 134.29, 134.27, 133.37, 132.45, 131.63, 130.31, 130.00, 128.30, 127.34, 126.60, 125.21, 125.12, 124.51, 123.85, 113.16, 109.10, 31.89, 24.40, 24.08, 22.70, 22.54.

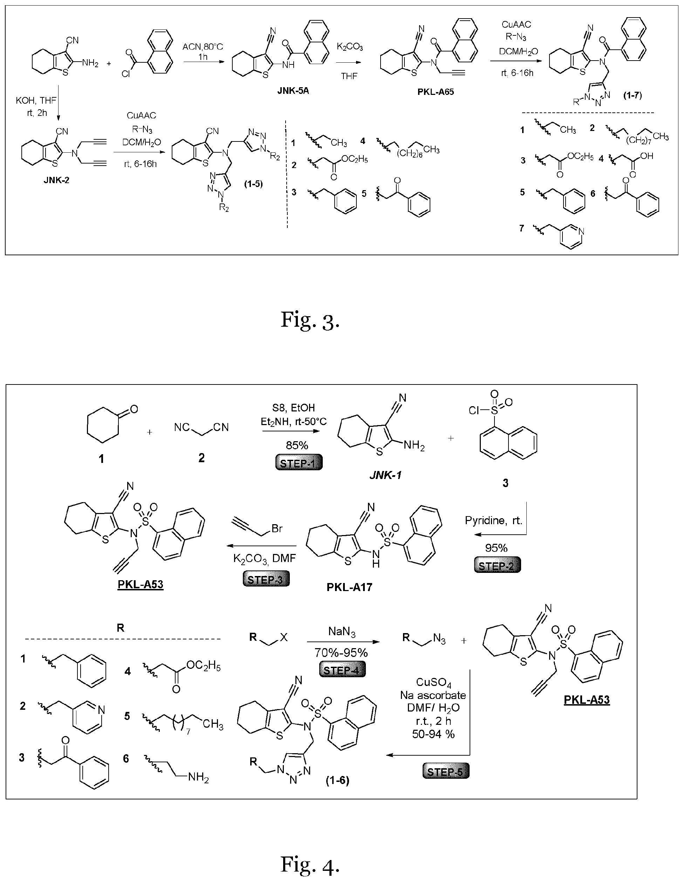

A schematic view of the synthesis of triazole analogues of N-(3-cyano-4,5,6,7-tetrahydrobenzo[b]thiophen-2-yl)-1-naphthamide can be seen in Fig. 3 and the synthesis procedure for the specific compounds follows below.

### Synthesis of N-(3-cyano-4,5,6,7-tetrahydrobenzo[b]thiophen-2-yl)-N-(prop-2-yn-1-yl)-1-naphthamide (PKL-A65)

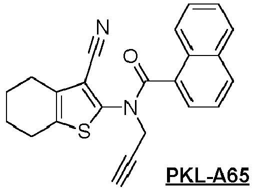

Following general procedure D, JNK-5A (500mg, 1,5 mmol), was reacted with propargyl bromide solution (0,013ml, 1,8 mmol), to afford 480 mg (81%) of the title compound as a yellow liquid after column chromatography ( EtOAc in Hexane : 0-10%). ¹H NMR (500 MHz, CDCl₃) δ 7.98 (d, J = 8.4 Hz, 1H), 7.75 (d, J = 7.2 Hz, 3H), 7.48 (dt, J = 34.2, 7.3 Hz, 3H), 7.32 (s, 1H), 4.72 (s, oH), 2.42 (s, 4H), 2.33 (s, 1H), 1.65 (s, 4H). ¹³C NMR (126 MHz, CDCl₃) δ 135.16, 133.23, 132.31, 129.26, 127.20, 126.29, 125.57, 124.99, 124.75, 124.14, 124.05, 123.54, 112.27, 73.22, 66.95, 28.67, 23.50, 23.13, 21.62, 20.66, 18.71.

### Synthesis of N-((1-benzyl-1H-1,2,3-triazol-4-yl)methyl)-N-(3-cyano-4,5,6,7-tetrahydro benzo[b] thiophen-2-yl)-1-naphthamide (PKL-B4)

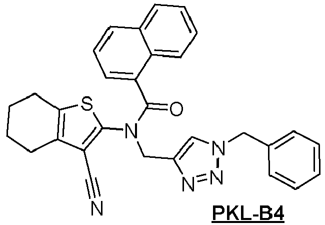

Following general procedure D, PKL-A65 (90mg, 0,24 mmol), reacted with benzyl azide (38,8 mg, 0,29 mmol) to afford 75 mg (61 %) of the title compound as a white solid after column chromatography (EtOAc in Hexane : 0-40%). ¹H NMR (500 MHz, CDCl₃) δ 7.81 (s, 1H), 7.72 (d, J = 7.4 Hz, 2H), 7.43 - 7.38 (m, 2H), 7.33 (d, J = 6.9 Hz, 3H), 7.25 (d, J = 16.4 Hz, 3H), 7.19 (t, J = 2.3 Hz, 1H), 5.51 (s, 2H), 5.16 (s, 2H), 2.45 - 2.20 (m, 3H), 1.59 (s, 4H). ¹³C NMR (126 MHz, CDCl₃) δ 169.68, 142.24, 135.10, 133.56, 133.09, 132.28, 131.42, 129.11, 128.94, 128.13, 127.76, 127.17, 127.09, 126.08, 125.44, 124.14, 123.47, 112.21, 53.28, 23.43, 23.04, 21.55, 20.59.

### Synthesis of N-[3-cyano-4,5,6,7-tetrahydrobenzo[b]thiophen-2-yl)-N-[(1-(2-oxo-2-phenyl ethyl)-1H-1,2,3-triazol-4-yl)methyl)-1-naphthamide (PKL-B31)

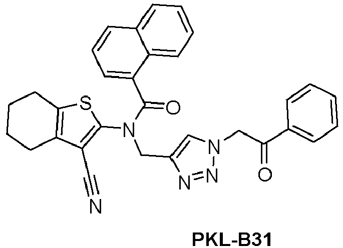

Following general procedure D, PKL-A65 (100mg, 0.30 mmol), reacted with 2-azido-1-phenylethan-1-one (52.2 mg, 0.32 mmol) to afford 95 mg (66 %) of the title compound as a white solid after column chromatography (EtOAc in Hexane: o-40%).

¹H NMR (500 MHz, CDCl₃) δ 7.95 (d, J = 7.8 Hz, 3H), 7.72 (d, J = 8.2 Hz, 2H), 7.60 (d, J = 7.4 Hz, 1H), 7.53 - 7.39 (m, 5H), 7.29 (s, 1H), 5.84 (s, 2H), 5.22 (s, 2H), 2.33 (s, 4H), 1.59 (s, 4H). ¹³C NMR (126 MHz, CDCl₃) δ 189.98, 170.85, 143.19, 136.27, 134.60, 134-11, 133.99, 133.31, 132.59, 130.09, 129.22, 128.43, 128.15, 127.26, 126.52, 126.10, 125.37, 125.06, 124.53, 113.35, 109.57, 55.63, 31.60, 24.48, 24.10, 22.67, 22.59.

### Synthesis of ethyl2-(4-((N-(3-cyano-4,5,6,7-tetrahydrobenzo[b]thiophen-2-yl)-1-naphth amido) methyl)-1H-1,2,3-triazol-1-yl)acetate (PKL-B28)

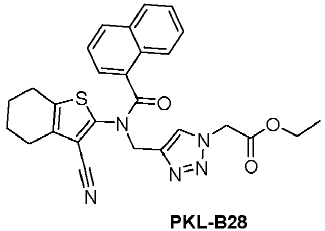

Following general procedure D, PKL-A65 (200mg, 0.54 mmol), reacted with ethyl 2-azidoacetate (76.6 mg, 0.59 mmol) to afford 235 mg (88 %) of the title compound as a white solid after column chromatography (EtOAc in Hexane : 0-50%).

¹H NMR (500 MHz, CDCl₃) δ 7.99 (s, 1H), 7.91 (d, J = 8.4 Hz, 1H), 7.72 (d, J = 8.2 Hz, 2H), 7.50 - 7.40 (m, 3H), 7.28 (s, 1H), 5.21 (s, 2H), 5.14 (s, 2H), 4.23 (q, J = 7.2 Hz, 2H), 2.32 (s, 4H), 1.58 (d, J = 9.9 Hz, 5H), 1.25 (t, J = 7.2 Hz, 3H). ¹³C NMR (126 MHz, CDCl₃) δ 171.17, 170.81, 166.13, 149.60, 143.30, 136.21, 134.11, 133.32, 132.50, 130.15, 130.02, 128.18, 127.19, 126.50, 125.57, 125.28, 125.13, 124.51, 113.29, 62.47, 60.41, 51.02, 24.46, 24.09, 22.58, 21.62, 14.13.

### Synthesis of 2-(4-((N-(3-cyano-4,5,6,7-tetrahydrobenzo[b]thiophen-2-yl)-1-naphthamido )methyl)-1H-1,2,3-triazol-1-yl)acetic acid (PKL-B33)

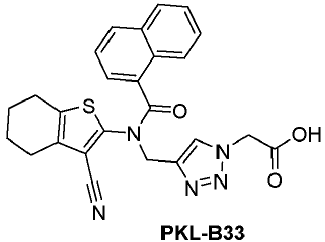

Following general procedure C, PKL-B28 (80 mg, 0.16 mmol), reacted with Lithium hydroxide (13.44 mg, 0.32 mmol) to afford 49 mg (65 %) of the title compound as a yellow solid after column chromatography (EtOAc in Hexane: 0-20%).

¹H NMR (500 MHz, CDCl₃) δ 8.13 (s, 1H), 7.88 (d, *J* = 8.4 Hz, 1H), 7.73 (t, *J =* 7.4 Hz, 2H), 7.49 - 7.39 (m, 4H), 7.28 (t, *J =* 7.7 Hz, 1H), 5.23 (s, 2H), 5.12 (s, 3H), 2.31 (s, 6H), 1.58 (s, 6H). ¹³C NMR (126 MHz, CDCl₃) δ 171.51, 167.97, 136.49, 134.23, 133.28, 131.99, 130.40, 129.89, 128.24, 127.32, 126.62, 125.24, 125.16, 124.50, 50.79, 24.45, 24.06, 22.55, 21.58.

### Synthesis of 2-(4-((N-(3-cyano-4,5,6,7-tetrahydrobenzo[b]thiophen-2-yl)-1-naphthamido )methyl)-1H-1,2,3-triazol-1-yl)acetic acid (PKL-B34)

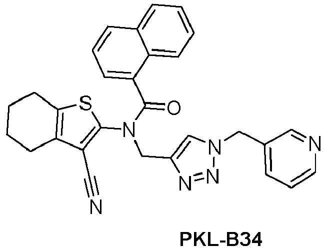

Following general procedure D, PKL-A65 (100 mg, 0.30 mmol), reacted with 3-(azidomethyl) pyridine (43.4 mg, 0.32 mmol) to afford 97 mg (71 %) of the title compound as a white solid after column chromatography (EtOAc in Hexane: o-40%).

¹H NMR (500 MHz, CDCl₃) δ 8.60 (s, 1H), 7.87 - 7.79 (m, 1H), 7.76 - 7.68 (m, 2H), 7.55 (t, *J* = 9.4 Hz, 1H), 7.46 - 7.38 (m, 3H), 7.31 - 7.23 (m, 2H), 7.18 (d, *J =* 8.8 Hz, 1H), 5.53 (s, 2H), 5.16 (s, 2H), 2.31 (d, *J* = 21.9 Hz, 4H), 1.59 (s, 5H). ¹³C NMR (126 MHz, CDCl₃) δ 170.81, 170.77, 150.20, 149.45, 149.14, 143.62, 136.24, 135.79, 134.16, 133.32, 132.34, 130.75, 130.23, 128.23, 127.15, 126.53, 125.21, 125.13, 124.49, 51.72, 46.67, 24.47, 24.06, 22.56, 21.59.

### N-(3-cyano-4,5,6,7-tetrahydrobenzo[b]thiophen-2-yl)-N-((1-decyl-1H-1,2,3-triazol-4-yl)methyl)-1-naphthamide(PKL-B35)

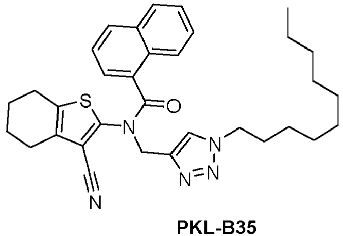

Following general procedure D, PKL-A65 (100mg, 0.39 mmol), reacted with azidodecane (79.27 mg, 0.43 mmol) to afford 140 mg (57 %) of the title compound as a white solid after column chromatography (EtOAc in Hexane : 0-40%).

¹H NMR (500 MHz, CDCl₃) δ 7.91 (d, *J =* 8.2 Hz, 1H), 7.83 (s, 1H), 7.73 (d, *J* = 8.1 Hz, 2H), 7.44 (dq, *J* = 15.1, 7.4 Hz, 3H), 7.29 (s, 1H), 5.18 (s, 2H), 4.32 (t, *J =* 7.0 Hz, 2H), 2.32 (s, 4H), 1.87 (s, 2H), 1.59 (s, 2H), 1.27 (s, 4H), 1.24 - 1.13 (m, 11H), 0.81 (t, *J =* 6.8 Hz, 3H). ¹³C NMR (126 MHz, CDCl₃) δ 170.75, 142.74, 136.11, 134.07, 133.33, 132.51, 130.15, 129.97, 128.22, 127.10, 126.47, 125.21, 124.51, 77.28, 77.03, 76.77, 50.53, 31.87, 30.30, 29.51, 29.44, 29.28, 29.03, 26.51, 24.47, 24.07, 22.68, 22.58, 21.62, 14.12.

### Synthesis of 2-(di(prop-2-yn-1-yl)amino)-4,5,7,6-tetrahydrobenzo[b]thiophene-3-carbonitrile (JNK-2)

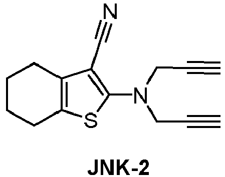

A solution of JNK-1 (1 g, 5.61 mmol), potassium hydroxide (346.23 mg, 6.17 mmol) and TBAB (Catalytic amount 0.2 equiv.) in THF was stirred for 30 min at room temperature. Then propargyl bromide solution (0.051ml, 6.73 mmol) was added, and the reaction mixture was stirred at room temperature for 48 h. The residue was partitioned between water and ethyl acetate and the aqueous extracted with further ethyl acetate. The combined organic extracts were washed with brine, dried over magnesium sulphate and evaporated to dryness under reduced pressure to give the desired product as a yellow liquid.

### Synthesis of 2-(bis((1-(2-oxo-2-phenylethyl)-1H-1,2,3-triazol-4-yl)methyl) amino) -4,5,6,7-tetrahydro- benzo[b]thiophene-3-carbonitrile (PKL-B86)

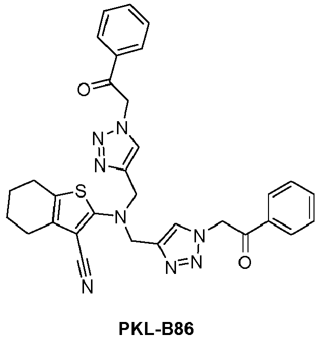

Following general procedure D, JNK-2 (100 mg, 0.39 mmol), reacted with 2-azido-1-phenylethan-1-one (30.74 mg, 0.43 mmol) to afford 110 mg (49 %) of the title compound as a white solid after column chromatography (EtOAc in Hexane : 0-60%).

¹H NMR (500 MHz, CDCl₃) δ 7.81 (d, *J* = 1.3 Hz, 2H), 7.79 (d, *J* = 1.4 Hz, 2H), 7.65 (s, 2H), 7.59 - 7.54 (m, 2H), 7.42 - 7.37 (m, 4H), 5.80 (s, 4H), 4.83 (s, 4H), 2.43 (dd, *J* = 6.5, 4.2 Hz, 4H), 1.75 - 1.67 (m, 4H). ¹³C NMR (126 MHz, CDCl₃) δ 190.38, 162.52, 134.51, 133.88, 133.77, 129.13, 128.02, 125.38, 121.28, 87.03, 55.62, 49.30, 24.62, 24.14, 23.25, 22.17.

### Synthesis of 2-(bis((1-benzyl-1H-1,2,3-triazol-4-yl)methyl)amino)-4,5,6,7-tetrahydrobenzo [b] thiophene -3-carbonitrile (PKL-B60)

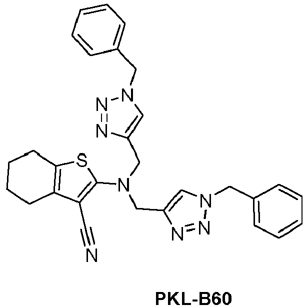

Following general procedure D, JNK-2 (200 mg, 0.78 mmol), reacted with (azidomethyl)benzene (125.64 mg, 0.94 mmol)) to afford 220 mg (54 %) of the title compound as a white solid after column chromatography (EtOAc in Hexane: 0-60%).

¹H NMR (500 MHz, CDCl₃) δ 7.81 (d, *J* = 1.3 Hz, 2H), 7.79 (d, *J* = 1.4 Hz, 2H), 7.65 (s, 2H), 7.59 - 7.54 (m, 2H), 7.42 - 7.37 (m, 4H), 5.80 (s, 4H), 4.83 (s, 4H), 2.43 (dd, *J* = 6.5, 4.2 Hz, 4H), 1.75 - 1.67 (m, 4H). ¹³C NMR (126 MHz, CDCl₃) δ 190.38, 162.52, 134.51, 133.88, 133.77, 129.13, 128.02, 125.38, 121.28, 87.03, 55.62, 49.30, 24.62, 24.14, 23.25, 22.17.

### Synthesis of diethyl 2,2'-((((3-cyano-4,5,6,7-tetrahydrobenzo[b]thiophen-2-yl)azanediyl)bis (methylene))bis(1H-1,2,3-triazole-4,1-diyl))diacetate (PKL-B40)

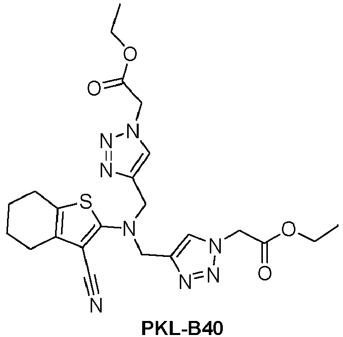

Following general procedure D, JNK-2 (200 mg, 0.78 mmol), reacted with ethyl 2-azidoacetate (111.68 mg, 0.86 mmol) to afford 230 mg (57 %) of the title compound as a white solid after column chromatography (EtOAc in Hexane : 0-60%).

¹H NMR (500 MHz, CDCl₃) δ 7.67 (s, 2H), 5.08 (s, 4H), 4.75 (s, 4H), 4.18 (q, *J* = 7.1 Hz, 4H), 2.43 (dt, *J* = 11.4, 5.6 Hz, 4H), 1.70 (dq, *J* = 11.9, 6.5, 5.9 Hz, 4H), 1.22 (t, *J* = 7.1 Hz, 7H). ¹³C NMR (126 MHz, CDCl₃) δ 166.32, 162.47, 143.59, 133.78, 124.84, 121.55, 117.53, 87.70, 62.44, 50.90, 48.79, 24.60, 24.14, 23.22, 22.15, 14.04.

### Synthesis of 2-(bis((1-decyl-1H-1,2,3-triazol-4-yl)methyl)amino)-4,5,6,7-tetrahydro benzo[b] thiophene-3-carbonitrile (PKL-B85)

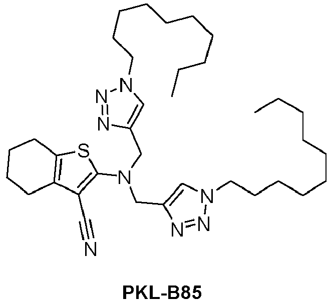

Following general procedure D, JNK-2 (100 mg, 0.39 mmol), reacted with azidodecane (79.27 mg, 0.43 mmol) to afford 140 mg (57 %) of the title compound as a white solid after column chromatography (EtOAc in Hexane: 0-40%).

¹H NMR (500 MHz, CDCl₃) δ 7.65 (s, 2H), 4.67 (s, 4H), 4.26 (t, *J* = 7.3 Hz, 4H), 2.43 (dt, *J =* 11.9, 5.6 Hz, 4H), 1.82 (t, *J =* 7.3 Hz, 4H), 1.76 - 1.66 (m, 4H), 1.30 - 1.22 (m, 11H), 1.18 (s, 20H), 0.80 (t, *J* = 6.8 Hz, 7H). ¹³C NMR (126 MHz, CDCl₃) δ 161.65, 141.75, 132.76, 122.23, 120.35, 116.55, 86.76, 49.44, 47.25, 30.83, 29.27, 28.67, 28.45, 28.37, 28.23, 28.23, 27.96, 25.46, 23.57, 23.10, 22.21, 21.64, 21.13, 13.08.

A schematic view of the synthesis of triazole analogues of N-(3-cyano-4,5,6,7-tetrahydrobenzo[b]thiophen-2-yl)naphthalene-1-sulfonamide can be seen in Fig. 4 and the synthesis procedure for the specific compounds follows below.

### General Procedure for the Synthesis of N-(3-cyano-4,5,6,7-tetrahydrobenzo[b]thiophen-2-yl)-N-[prop-2-yn-1-yl)naphthalene-1-sulfonamide (PKL-A53)

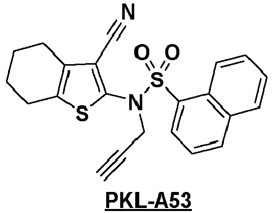

Add 514 mg (3.72 mmol) K₂CO₃, 0.754 mL (1.50 g, 7.44 mmol) propargyl bromide to a solution of 207 mg (0.744 mmol) PKL-A17 in DMF (0.07 M) in an oven dried round bottom flask. Heat the mixture to 60 °C. Pour the reaction mixture into ice water. After cooling to room temperature, extract the mixture with EtOAc (3×). Wash the combined organic layers with 1 N HCl (1×) and 1 M LiCl (3×). Dry the mixture over Na₂SO₄ and concentrate it. Purify the residue by column chromatography using silica and hexanes/EtOAc 3:1 → 2:1 to obtain desired product or extract with ether/ water, combine ether layer and concentrate the mixture. Purify the residue by column chromatography using silica and hexane/EtOAc 3:1 → 2:1 to obtain the desired product.

¹H NMR (500 MHz, DMSO) δ 8.43 (d, J = 8.6 Hz, 2H), 8.27 (dd, J = 1.1, 7.5 Hz, 1H), 8.21 - 8.17 (m, 1H), 7.78 - 7.70 (m, 3H), 4.63 (d, J = 2.4 Hz, 2H), 3.41 (d, J = 2.6 Hz, 1H), 2.69 (t, J = 5.4 Hz, 2H), 2.53 (d, J = 5.1 Hz, 2H), 1.80 (dd, J = 4.1, 7.0 Hz, 4H). ¹³C NMR (126 MHz, DMSO) δ 162.79, 144.93, 138.29, 136.47, 134.42, 134.21, 132.07, 132.01, 129.63, 128.91, 128.53, 127.67, 125.10, 124.71, 113.04, 111.62, 78.47, 77.75, 42.91, 24.67, 24.12, 22.73, 21.85.

### General Procedure for the Synthesis of azides of heterocycles, alkyl and benzyl compounds

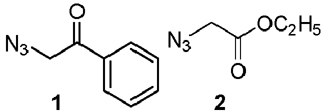

Method A: A round bottom flask was charged with alpha-halogenated heterocycles (6.5 mmol) in acetonitrile (20 mL) at room temperature. Sodium azide (19.4 mmol) was added to the stirring solution for 4 h. Upon completion, cold water was added and the reaction mixture was extracted with EtOAc (2×20 mL). The organic layers were then dried using anhydrous MgSO₄, filtered, and concentrated to obtain corresponding azides with good yield.

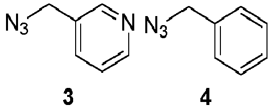

Method B: To a stirred solution of 10.0 mmol benzyl halides in 100 mL of acetone/H₂O 4:1 (v/v) was added 15.0 mmol (0.98 g) of sodium azide. The reaction mixture was stirred at room temperature for 24 h. After, the reaction was extracted with Et₂O (3 × 50 mL), dried over Na₂SO₄, and concentrated under reduced pressure to give benzyl azides as pale yellow oils.

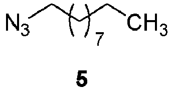

Method C: To a stirred solution of 10.0 mmol alkyl halides in 20 mL of DMF, 12.0 mmol (0.78 g) of sodium azide was added. The reaction mixture was stirred at 70 °C for 24 h in an oil bath. After, the reaction was extracted with Et₂O (3 × 50 mL), dried over Na₂SO₄, and concentrated under reduced pressure to give alkyl azides as pale yellow oil.

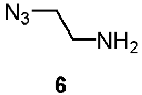

(Method D): To a stirred solution of 10.0 mmol alkyl amine halides in 5 mL of H₂O, 12.0 mmol (0.78 g) of sodium azide was added. The reaction mixture was stirred at 70 °C for 12 h in an oil bath. After, the reaction was extracted with Et₂O (3 × 50 mL), dried over Na₂SO₄, and concentrated under reduced pressure to give alkyl azides as pale yellow oil.

### General Procedure for the Synthesis of triazole analogues of N-(3-cyano-4,5,6,7-tetrahydrobenzo [b]thiophen-2-yl)naphthalene-1-sulfonamide

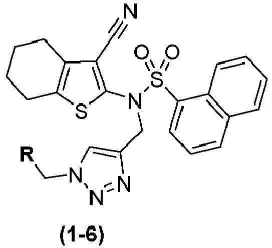

To a solution of PKL-A53 (0.2 g, 0.97 mmol) in mixture of DMF: water (3: 2, v/v, 5 mL), a stoichiometric amount of corresponding acetophenone and heterocyclic azides (1.0 mmol) was added. After addition of copper sulphate (0.48 mmol) and sodium L-ascorbate (0.73 mmol), reaction mixture was left for stirring at room temperature for 12 h. On complete utilization of starting material, reaction mixture was adsorbed directly on silica, and subjected to purification by column chromatography to obtain desired products in 65-94% yields (Eluent: hexane: ethyl acetate: 8.0: 2.0 - 1.0: 9.0).

### Synthesis of N-((1-benzyl-1H-1,2,3-triazol-4-yl)methyl)-N-(3-cyano-4,5,6,7-tetrahydrobenzo[b] thiophen-2-yl)naphthalene-1-sulfonamide (PKL-A55)

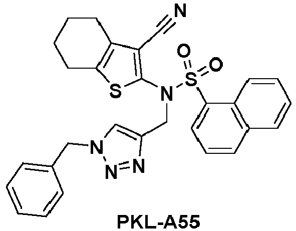

Following general procedure D, N-(3-cyano-4,5,6,7-tetrahydrobenzo[b]thiophen-2-yl)-*N*-(prop-2-yn-1-yl)naphthalene-1-sulfonamide (PKL-A53) reacted with benzyl azide to afford (70 %) of the title compound as a white solid after column chromatography (EtOAc in Hexane : 0-70%).

¹H NMR (500 MHz, CDCl₃) δ 8.60 (dd, J = 3.5, 6.6 Hz, 1H), 8.24 (dd, J = 1.7, 7.5 Hz, 1H), 8.13 (d, J = 8.2 Hz, 1H), 7.95 (dd, J = 3.5, 6.6 Hz, 1H), 7.60 (ddd, J = 1.9, 4.4, 8.4 Hz, 3H), 7.53 (t, J = 7.9 Hz, 1H), 7.39 - 7.34 (m, 3H), 7.23 (t, J = 4.4 Hz, 2H), 5.48 (s, 2H), 4.95 (s, 2H), 2.61 - 2.53 (m, 2H), 2.41 (t, J = 6.1 Hz, 2H), 1.80 - 1.70 (m, 4H). ¹³C NMR (126 MHz, CDCl₃) δ 143.96, 141.81, 136.72, 134.62, 133.39, 131.42, 130.44, 128.08, 127.95, 127.74, 127.46, 127.09, 126.17, 123.93, 123.09, 122.77, 111.66, 110.77, 53.27, 47.76, 28.68, 23.77, 23.07, 21.70, 20.75.

### Synthesis of N-(3-cyano-4,5,6,7-tetrahydrobenzo[b]thiophen-2-yl)-N-((1-(pyridin-3-ylmethyl)-1H-1,2,3-triazol-4-yl)methyl)naphthalene-1-sulfonamide(PKL-A64)

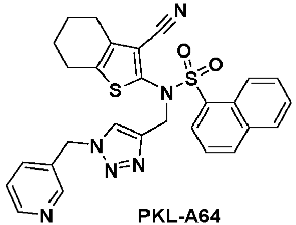

Following general procedure D, *N*-(3-cyano-4,5,6,7-tetrahydrobenzo[b]thiophen-2-yl)-*N*-(prop-2-yn-1-yl)naphthalene-1-sulfonamide (PKL-A53) reacted with 3-(azidomethyl)pyridine to afford (65%) of the title compound as a brown solid after column chromatography (EtOAc in Hexane : 50-100%).

¹H NMR (500 MHz, CDCl₃) δ 8.67 - 8.54 (m, 3H), 8.24 (d, J = 7.3 Hz, 1H), 8.13 (d, J = 8.2 Hz, 1H), 7.95 (dt, J = 2.2, 5.7 Hz, 1H), 7.65 - 7.59 (m, 3H), 7.58 - 7.51 (m, 2H), 7.32 (d, J = 6.6 Hz, 1H), 5.51 (s, 2H), 4.95 (d, J = 1.7 Hz, 2H), 2.57 (t, J = 6.1 Hz, 2H), 2.42 (t, J = 6.0 Hz, 2H), 1.76 (dq, J = 5.4, 19.1 Hz, 4H). ¹³C NMR (126 MHz, CDCl₃) δ 170.14, 149.20, 148.17, 143.92, 142.25, 136.82, 134.77, 134.67, 133.48, 133.38, 131.36, 130.50, 127.99, 127.72, 127.50, 126.21, 123.91, 123.12, 122.92, 122.88, 111.72, 110.88, 59.38, 50.65, 47.79, 23.78, 23.08, 21.69, 20.74, 13.18.

### Synthesis of N-(3-cyano-4,5,6,7-tetrahydrobenzo[b]thiophen-2-yl)-N-1-(2-oxo-2-phenylethyl)-1H-1,2,3-triazol-4-yl)methyl)naphthalene-1-sulfonamide (PKL-A54)

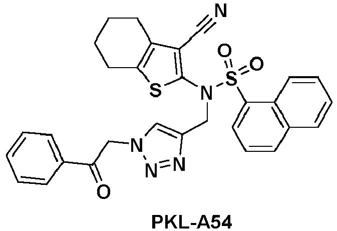

Following general procedure D, *N*-(3-cyano-4,5,6,7-tetrahydrobenzo[b]thiophen-2-yl)-*N*-(prop-2-yn-1-yl)naphthalene-1-sulfonamide (PKL-A53) reacted with 2-azido-1-phenylethan-1-one to afford (80%) of the title compound as a white solid after column chromatography (EtOAc in Hexane : 0-70%).

¹H NMR (500 MHz, CDCl₃) δ 8.60 (dd, J = 3.5, 6.6 Hz, 1H), 8.24 (dd, J = 1.7, 7.5 Hz, 1H), 8.13 (d, J = 8.2 Hz, 1H), 7.95 (dd, J = 3.5, 6.6 Hz, 1H), 7.60 (ddd, J = 1.9, 4.4, 8.4 Hz, 3H), 7.53 (t, J = 7.9 Hz, 1H), 7.39 - 7.34 (m, 3H), 7.23 (t, J = 4.4 Hz, 2H), 5.48 (s, 2H), 4.95 (s, 2H), 2.6₁ - 2.53 (m, 2H), 2.41 (t, J = 6.1 Hz, 2H), 1.80 - 1.70 (m, 4H). ¹³C NMR (126 MHz, CDCl₃) δ 189.98, 143.96, 141.81, 136.72, 134.62, 133.39, 131.42, 130.44, 128.08, 127.95, 127.74, 127.46, 127.09, 126.17, 123.93, 123.09, 122.77, 111.66, 110.77, 53.27, 47.76, 28.68, 23.77, 23.07, 21.70, 20.75.

### Synthesis of ethyl 2-(4-((N-(3-cyano-4,5,6,7-tetrahydrobenzo[b]thiophen-2-yl)naphthalene-1-sulfonamido)methyl)-1H-1,2,3-triazol-1-yl)acetate (PKL-A73)

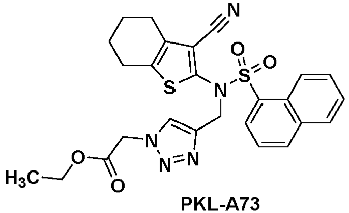

Following general procedure D, *N*-(3-cyano-4,5,6,7-tetrahydrobenzo[b]thiophen-2-yl)-*N*-(prop-2-yn-1-yl)naphthalene-1-sulfonamide (PKL-A53) reacted with ethyl 2-azidoacetate to afford (89%) of the title compound as a white solid after column chromatography (EtOAc in Hexane : 0-70%).

¹H NMR (500 MHz, CDCl₃) δ 8.63 (d, J = 8.3 Hz, 1H), 8.27 (d, J = 7.3 Hz, 1H), 8.15 (d, J = 8.2 Hz, 1H), 7.96 (d, J = 8.0 Hz, 1H), 7.86 (s, 1H), 7.64 (t, J = 8.3 Hz, 2H), 7.55 (t, J = 7.8 Hz, 1H), 5.12 (s, 2H), 5.00 (s, 2H), 4.27 (q, J = 7.1 Hz, 2H), 2.58 (t, J = 5.9 Hz, 2H), 2.43 (t, J = 6.0 Hz, 2H), 1.81 - 1.70 (m, 4H), 1.30 (t, J = 7.2 Hz, 3H).¹³C NMR (126 MHz, CDCl₃) δ 164.97, 144.03, 141.91, 136.75, 134.66, 133.46, 133.42, 131.43, 130.49, 127.98, 127.80, 127.50, 126.18, 124.41, 123.94, 123.13, 111.71, 110.71, 61.44, 49.97, 47.72, 23.78, 23.09, 21.70, 20.76, 13.07, 0.00.

### Synthesis of N-(3-cyano-4,5,6,7-tetrahydrobenzo[b]thiophen-2-yl)-N-((1-decyl-1H-1,2,2-triazol-4-yl)methyl)naphthalene-1-silfonamide (PKL-A48-1)

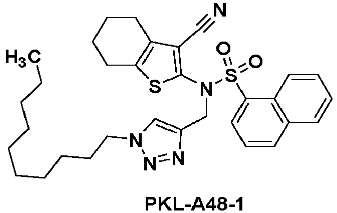

Following general procedure D, *N*-(3-cyano-4,5,6,7-tetrahydrobenzo[b]thiophen-2-yl)-*N*-(prop-2-yn-1-yl)naphthalene-1-sulfonamide (PKL-A53) reacted with 1-azidodecane to afford (88%) of the title compound as a white solid after column chromatography (EtOAc in Hexane : 0-65%).

¹H NMR (500 MHz, CDCl₃) δ 8.64 (d, J = 8.2 Hz, 1H), 8.26 (d, J = 7.3 Hz, 1H), 8.15 (d, J = 8.1 Hz, 1H), 7.97 (d, J = 7.8 Hz, 1H), 7.67 (s, 1H), 7.66 - 7.60 (m, 2H), 7.55 (t, J = 7.8 Hz, 1H), 4.97 (s, 2H), 4.30 (t, J = 7.2 Hz, 2H), 2.57 (t, J = 6.0 Hz, 2H), 2.43 (t, J = 6.0 Hz, 2H), 1.86 (t, J = 7.2 Hz, 2H), 1.82 - 1.69 (m, 4H), 1.26 (s, 15H), 0.88 (t, J = 6.8 Hz, 3H). ¹³C NMR (126 MHz, CDCl₃) δ 144.06, 141.39, 136.71, 134.62, 133.40, 131.49, 130.46, 127.98, 127.80, 127.49, 126.19, 123.98, 123.12, 122.56, 111.74, 110.80, 49.50, 47.96, 30.85, 29.14, 28.48, 28.40, 28.26, 27.96, 25.41, 23.78, 23.09, 21.70, 21.66, 20.76, 13.09.

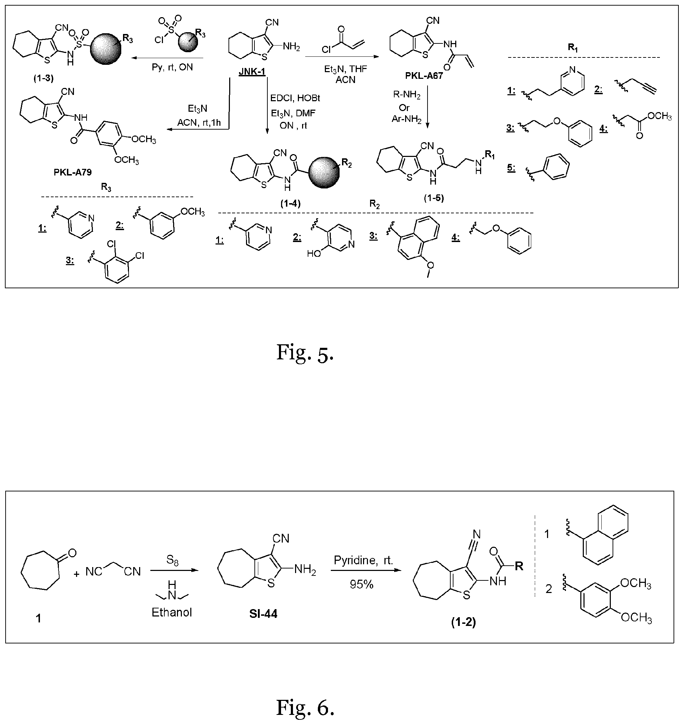

A schematic view of the synthesis of analogues of 2-amino-4,5,6,7-tetrahydrobenzo[b]thiophene-3-carbonitrile can be seen in Fig. 5 and the synthesis procedure for the specific compounds follows below.

### General Procedure for the Synthesis of N-(2-cyano-4,5,6,7-tetrahydrobenzo[b]thiophen-2-yl) acrylamide (PKL-A67)

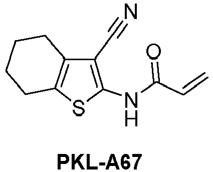

To the solution of JNK-1 (1 equiv.) in THF or ACN (3-5 mL) Et₃N (1.1 equiv.) was added at 0 °C and left at room temperature for 1.5 h. After that, add acroyl chloride (1.1 equiv.) dropwise to the reaction mixture at 0 °C and stir at room temperature overnight. The reaction mixture was quenched with cold NH₄Cl solution and extracted with EtOAc. The combined organic layers were washed with brine, dried (Na₂SO₄), and concentrated under reduced pressure. Purification by column chromatography (3:1 hexanes/EtOAc) afforded desired compound in 75% yield.

¹H NMR (500 MHz, CDCl₃) δ 8.57 (s, 1H), 6.55 (d, J = 16.8 Hz, 1H), 6.39 - 6.30 (m, 1H), 5.92 (d, J = 10.2 Hz, 1H), 2.66 (d, J = 5.4 Hz, 2H), 2.60 (d, J = 5.4 Hz, 2H), 1.86 - 1.82 (m, 4H). ¹³C NMR (126 MHz, CDCl₃) δ 160.75, 145.51, 130.05, 129.58, 127.98, 127.48, 113.48, 22.95, 22.05, 21.08.

### General Procedure for the Synthesis of Michael adducts of N-(3-cyano-4,5,6,7-tetrahydro benzo[b]thiophen-2-yl)acrylamide (1-5)

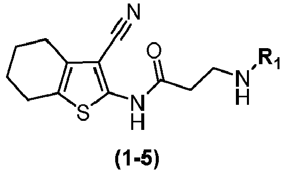

To the solution of A (1 equiv.) in ACN (3-5 mL) Et₃N (1.1 equiv.) was added at 0 °C and left at room temperature for 1.5 h. After that, add the corresponding amine (1.1 equiv.) dropwise to the reaction mixture at 0 °C and stir at room temperature overnight. The reaction mixture was quenched with cold NH₄Cl solution and extracted with EtOAc. The combined organic layers were washed with brine, dried (Na₂SO₄), and concentrated under reduced pressure. Purification by column chromatography (3:1 hexanes/EtOAc) afforded desired compound in good yield.

### Synthesis of N-(3-cyano-4,5,6,7-tetrahydrobenzo[b]thiophen-2-yl)-3-(phenylamino)propanamide (PKL-A29)

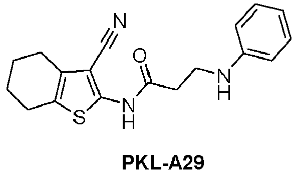

Following general procedure for the Synthesis of Michael adducts, *N*-(3-cyano-4,5,6,7-tetrahydrobenzo[*b*]thiophen-2-yl) acrylamide (PKL-A67) reacted with aniline to afford (80 %) of the title compound as a white solid after column chromatography (EtOAc in Hexane: 0-85%).

¹H NMR (500 MHz, CDCl₃) δ 9.56 (s, 1H), 7.23 (t, J = 7.7 Hz, 2H), 6.82 (t, J = 7.3 Hz, 1H), 6.77 (d, J = 8.0 Hz, 2H), 3.58 (t, J = 5.9 Hz, 2H), 2.77 (t, J = 5.9 Hz, 2H), 2.65 - 2.60 (m, 2H), 2.56 (t, J = 5.8 Hz, 2H), 1.82 (dp, J = 3.6, 4.6, 11.2 Hz, 5H). ¹³C NMR (126 MHz, CDCl₃) δ 167.59, 145.54, 145.37, 129.82, 128.43, 127.28, 118.34, 113.41, 113.28, 92.45, 38.96, 33.96, 22.92, 22.06, 21.08.

### Synthesis of N-(3-cyano-4,5,6,7-tetrahydrobenzo[b]thiophen-2-yl)-3-(prop-2-yn-1-ylamino)propanamide (PKL-A69)

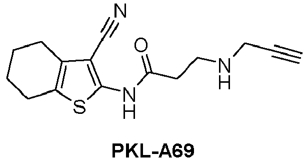

Following general procedure for the synthesis of Michael adducts, *N*-(3-cyano-4,5,6,7-tetrahydrobenzo[*b*]thiophen-2-yl) acrylamide (PKL-A67) reacted with prop-2-yn-1-amine to afford (82 %) of the title compound as a white solid after column chromatography (EtOAc in Hexane: 0-75%).

¹H NMR (500 MHz, CDCl₃) δ 12.54 (s, 1H), 3.63 (d, J = 2.4 Hz, 2H), 3.15 - 3.11 (m, 2H), 2.64 - 2.55 (m, 7H), 2.29 (t, J = 2.4 Hz, 1H), 1.81 (tdd, J = 2.3, 3.8, 8.8 Hz, 5H). ¹³C NMR (126 MHz, CDCl₃) δ 168.30, 146.38, 129.67, 126.68, 113.73, 92.16, 79.21, 71.85, 42.09, 36.09, 32.87, 22.99, 22.96, 22.12, 21.16.

### Synthesis of N-(3-cyano-4,5,6,7-tetrahydrobenzo[b]thiophen-2-yl)-3-((2-(pyridin-3-yl)ethyl)amino )propanamide (PKL-A68)

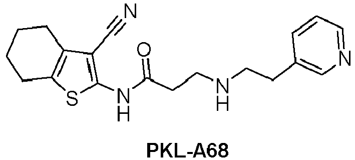

Following general procedure for the Synthesis of Michael adducts, *N*-(3-cyano-4,5,6,7-tetrahydrobenzo[*b*]thiophen-2-yl) acrylamide (PKL-A67) reacted with 2-(pyridin-3-yl)ethan-1-amine to afford (72 %) of the title compound as a white solid after column chromatography (EtOAc in Hexane : 20-95%).

¹H NMR (500 MHz, DMSO) δ 8.47 (s, 1H), 8.41 (d, J = 4.8 Hz, 1H), 7.67 (d, J = 7.8 Hz, 1H), 7.31 (dd, J = 4.8, 7.8 Hz, 1H), 3.17 (s, 2H), 3.00 - 2.90 (m, 4H), 2.86 (t, J = 7.4 Hz, 2H), 2.63 (t, J = 5.5 Hz, 2H), 2.57 (d, J = 4.9 Hz, 2H), 1.75 (s, 4H). ¹³C NMR (126 MHz, DMSO) δ 170.36, 150.28, 148.29, 147.96, 136.63, 135.28, 130.75, 127.10, 123.92, 115.15, 92.44, 79.70, 79.43, 79.17, 23.95, 23.84, 23.13, 22.23.

### Synthesis of methyl (3-((3-cyano-4,5,6,7-tetrahydrobenzo[b]thiophen-2-yl)amino)-3-oxopropyl)glycinate (PKL-A72)

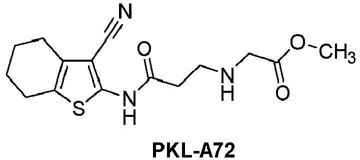

Following general procedure for the Synthesis of Michael adducts, *N*-(3-cyano-4,5,6,7-tetrahydrobenzo[*b*]thiophen-2-yl) acrylamide (PKL-A67) reacted with methyl glycinate to afford (75%) of the title compound as a white solid after column chromatography (EtOAc in Hexane : 0-85%).

¹H NMR (500 MHz, CDCl₃) δ 12.64 (s, 1H), 3.76 (s, 3H), 3.59 (s, 2H), 3.07 - 3.01 (m, 2H), 2.62 (td, J = 2.9, 5.0, 5.8 Hz, 2H), 2.58 - 2.54 (m, 4H), 1.81 (ddq, J = 2.5, 3.0, 4.6, 6.8 Hz, 4H). ¹³C NMR (126 MHz, CDCl₃) δ 170.96, 168.23, 146.33, 129.68, 126.64, 113.76, 92.19, 51.10, 48.46, 43.49, 33.46, 22.99, 22.96, 22.13, 21.17.

### Synthesis of N-(3-cyano-4,5,6,7-tetrahydrobenzo[b]thiophen-2-yl)-3-((2-phenoxyethyl)amino) propanamide (PKL-A95)

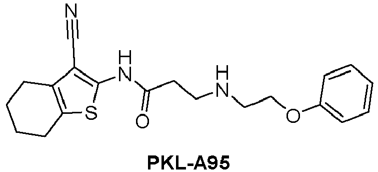

Following general procedure for the Synthesis of Michael adducts, *N*-(3-cyano-4,5,6,7-tetrahydrobenzo[*b*]thiophen-2-yl) acrylamide (PKL-A67) reacted with 2-phenoxyethan-1-amine to afford (74 %) of the title compound as a white solid after column chromatography (EtOAc in Hexane : 0-85%).

¹H NMR (500 MHz, CDCl₃) δ 12.86 (s, 1H), 7.22 (d, J = 7.7 Hz, 2H), 6.87 (dd, J = 7.9, 16.8 Hz, 3H), 4.21 (t, J = 4.9 Hz, 2H), 3.08 (t, J = 4.9 Hz, 2H), 3.01 (t, J = 5.7 Hz, 2H), 2.54 - 2.44 (m, 5H), 1.73 (q, J = 7.2 Hz, 4H). ¹³C NMR (126 MHz, CDCl₃) δ 168.61, 157.52, 146.46, 129.67, 128.48, 126.58, 119.98, 113.98, 113.49, 92.03, 65.35, 47.24, 43.62, 33.38, 22.96, 22.93, 22.12, 21.18.

### Synthesis of N-(3-cyano-4,5,6,7-tetrahydrobenzo[b]thiophen-2-yl)-3,4-dimethoxybenzamide (PKL-A79)

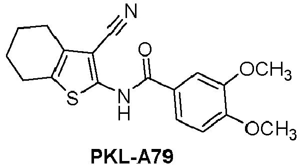

Following general procedure A for the synthesis of PKL-A79, 2-amino-4,5,6,7-tetrahydrobenzo[b]thiophene-3-carbonitrile (JNK-1) reacted with Dimethoxy Benzoyl chloride to afford (70%) of the title compound as a white solid after column chromatography (EtOAc in Hexane : 0-40%). ¹H NMR (500 MHz, CDCl₃) δ 8.78 (s, 1H), 7.55 - 7.50 (m, 1H), 7.44 (dt, J = 1.4, 8.5 Hz, 1H), 6.95 (d, J = 8.3 Hz, 1H), 3.97 (d, J = 2.5 Hz, 6H), 2.67 (t, J = 5.6 Hz, 2H), 2.63 (t, J = 5.7 Hz, 2H), 1.85 (q, J = 7.0 Hz, 4H). ¹³C NMR (126 MHz, CDCl₃) δ 162.87, 153.02, 149.42, 146.91, 130.82, 128.43, 124.13, 119.78, 114.49, 110.76, 110.40, 77.16, 56.08, 56.03, 53.32, 24.40, 24.01, 23.88.

### Synthesis of N-(3-cyano-4,5,6,7-tetrahydrobenzo[b]thiophen-2-yl)-2-phenoxyacetamide (PKL-A78)

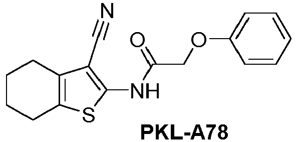

Following general procedure B, To a solution of 2-phenoxyacetic acid (1 equiv.), EDCl (1.2 equiv.) and HOBt (1.2 equiv.) in DMF (0.1-0.3 M) Triethylamine (1.5 equiv.) were added at 0°C. The mixture was stirred for 30 min at room temperature. Then, 2-amino-4,5,6,7-tetrahydrobenzo[b]thiophene-3-carbonitrile (JNK-1) (1.2 equiv.) was added, and the reaction mixture was stirred at room temperature for 36 h, to afford 65% of the title compound as white solid after column chromatography (EtOAc in Hexane : 0-40%).

¹H NMR (500 MHz, CDCl₃) δ 9.34 (s, 1H), 7.36 (t, J = 7.8 Hz, 2H), 7.08 (t, J = 7.4 Hz, 1H), 7.02 (d, J = 8.1 Hz, 2H), 4.72 (s, 2H), 2.64 (dt, J = 5.5, 21.1 Hz, 4H), 1.84 (q, J = 7.4 Hz, 5H). ¹³C NMR (126 MHz, CDCl₃) δ 165.32, 156.91, 145.32, 131.65, 130.40, 129.89, 129.41, 123.25, 115.28, 114.23, 95.31, 77.16, 67.17, 24.43, 24.39, 23.46, 22.47.

### Synthesis of N-(3-cyano-4,5,6,7-tetrahydrobenzo[b]thiophen-2-yl)-3-hydroxyisonicotinamide (PKL-A102)

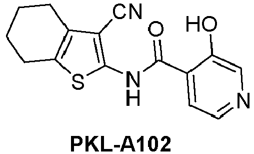

Following general procedure B, To a solution of 3-hydroxyisonicotinic acid (1 equiv.), EDCl (1.2 equiv.) and HOBt (1.2 equiv.) in DMF (0.1-0.3 M) Triethylamine (1.5 equiv.) were added at 0°C. The mixture was stirred for 30 min at room temperature. Then, 2-amino-4,5,6,7-tetrahydrobenzo[b]thiophene-3-carbonitrile (JNK-1) (1.2 equiv.) was added, and the reaction mixture was stirred at room temperature for 36 h, to afford 52 % of the title compound as a white solid after column chromatography (EtOAc in Hexane : 0-95%).

¹H NMR (500 MHz, DMSO) δ 11.74 (s, 1H), 7.29 (s, 1H), 7.07 (d, J = 5.0 Hz, 1H), 6.82 (d, J = 4.9 Hz, 1H), 1.77 - 1.67 (m, 4H), 0.91 (dq, J = 2.0, 3.1, 7.0 Hz, 4H). ¹³C NMR (126 MHz, DMSO) δ 165.61, 157.97, 140.47, 136.55, 129.91, 126.13, 125.37, 122.67, 121.13, 117.02, 92.42, 39.52, 24.33, 24.14, 23.49, 22.61.

### Synthesis of N-[2-cyano-4,5,6,7-tetrahydrobenzo[b]thiophen-2-yl) nicotinamide (PKL-A103)

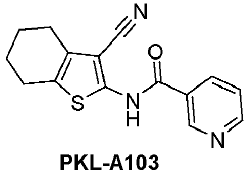

Following general procedure B, to a solution of nicotinic acid (1 equiv.), EDCl (1.2 equiv.) and HOBt (1.2 equiv.) in DMF (0.1-0.3 M) Triethylamine (1.5 equiv.) were added at 0°C. The mixture was stirred for 30 min at room temperature. Then, 2-amino-4,5,6,7-tetrahydrobenzo[b]thiophene-3-carbonitrile (JNK-1) (1.2 equiv.) was added, and the reaction mixture was stirred at room temperature for 48 h, to afford 45% of the title compound as a white solid after column chromatography (EtOAc in Hexane: 0-85%).

¹H NMR (500 MHz, DMSO) δ 11.17 (s, 1H), 8.25 (d, J = 2.3 Hz, 1H), 7.97 (dd, J = 1.9, 4.8 Hz, 1H), 7.46 (dt, J = 2.1, 7.9 Hz, 1H), 6.77 (dd, J = 4.9, 7.9 Hz, 1H), 1.76 - 1.71 (m, 4H), 1.02 - 0.92 (m, 4H). ¹³C NMR (126 MHz, DMSO) δ 164.41, 153.26, 149.62, 136.59, 131.98, 129.28, 128.93, 123.97, 114.59, 96.42, 29.49, 24.09, 23.93, 23.06, 22.17.

### Synthesis of N-(3-cyano-4,5,6,7-tetrahydrobenzo[b]thiophen-2-yl) pyridine-3-sulfonamide (PKL-A82)

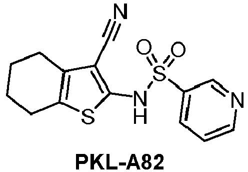

Following general procedure A for the synthesis of PKL-A82, 2-amino-4,5,6,7-tetrahydrobenzo[b]thiophene-3-carbonitrile (JNK-1) reacted with pyridine-3-sulfonyl chloride to afford (70%) of the title compound as a white solid after column chromatography (EtOAc in Hexane: 0-40%).

¹H NMR (500 MHz, CDCl₃) δ 9.03 (d, J = 2.2 Hz, 1H), 8.87 (d, J = 4.9 Hz, 1H), 8.21 - 8.17 (m, 1H), 7.52 (dd, J = 4.8, 8.1 Hz, 1H), 5.30 (s, 1H), 2.65 (t, J = 6.1 Hz, 2H), 2.51 (t, J = 6.1 Hz, 2H), 1.86 - 1.76 (m, 4H). ¹³C NMR (126 MHz, CDCl₃) δ 153.26, 147.31, 142.07, 134.17, 133.92, 133.06, 132.73, 123.14, 111.90, 103.11, 23.46, 23.12, 21.76, 20.74.

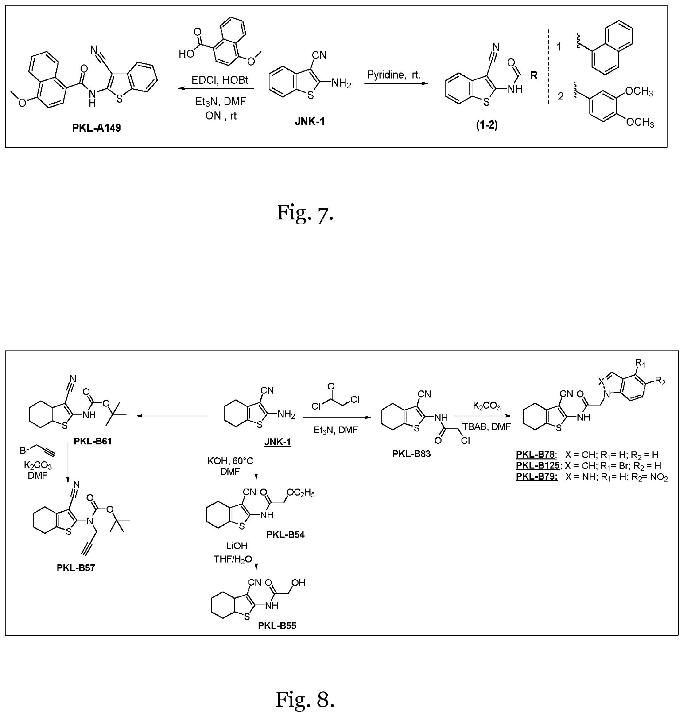

A schematic view of the synthesis of analogues of aminothiophene can be seen in Fig. 8 and the synthesis procedure for the specific compounds follows below.

### Synthesis of 2-chloro-N-(3-cyano-4,7,6,7-tetrahydrobenzo[b]thiophen-2-yl)acetamide(PKL-B83)

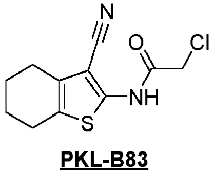

To solution of aminothiophene JNK-1 (200 mg, 1,12 mmol) in DMF, Triethylamine (0.17 mL, 1,23 mmol) was added dropwise to the reaction mixture at 0°C. The mixture was stirred for 30 min at room temperature. Then chloro acethyl chloride (0.1 mL, 1,23 mmol) was added, and the reaction mixture was stirred at room temperature for 1h. The residue was poured to ice, and then extracted with Chloroform. The combined organic extracts were washed with brine, dried over magnesium sulphate and evaporated to dryness under reduced pressure to afford 230 mg (81%) of the desired product as a brown solid.

¹H NMR (500 MHz, CDCl₃) δ 9.22 (s, 1H), 4.21 (s, 2H), 2.59 (ddd, *J* = 7.8, 5.3, 1.8 Hz, 2H), 2.54 (td, *J* = 6.0, 1.8 Hz, 2H), 1.81 - 1.73 (m, 5H). ¹³C NMR (126 MHz, CDCl₃) δ 162.80, 145.03, 131.40, 129.46, 113.79, 95.15, 42.03, 24.03, 23.98, 23.03, 22.04.

### Synthesis of representative molecule PKL-B78

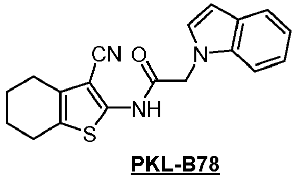

Following general procedure D, PKL-B83 (80 mg, 0.31 mmol), was reacted with indole (36.79 mg, 0.31 mmol), to afford the title compound as a white solid after column chromatography ( EtOAc in Hexane : 0 - 40%).'

¹H NMR (500 MHz, CDCl₃) δ 8.04 (s, 1H), 7.64 (dt, J = 7.9, 1.0 Hz, 1H), 7.25 - 7.20 (m, 2H), 7.13 (ddd, *J* = 8.0, 6.5, 1.5 Hz, 1H), 7.10 (d, *J* = 3.3 Hz, 1H), 6.66 (dd, *J* = 3.2, 0.8 Hz, 1H), 4.95 (s, 2H), 2.55 - 2.51 (m, 2H), 2.42 (d, *J* = 1.8 Hz, 2H), 1.70 (ddddd, *J* = 15.3, 7.8, 6.0, 3.6, 1.7 Hz, 4H). ¹³C NMR (126 MHz, CDCl₃) δ 165.43, 144.93, 136.15, 131.24, 129.20, 129.05, 127.68, 123.14, 121.88, 120.94, 113.23, 108.87, 104.85, 95.15, 49.55, 23.99, 23.91, 23.00, 21.99.

### Synthesis of representative molecule PKL-B125

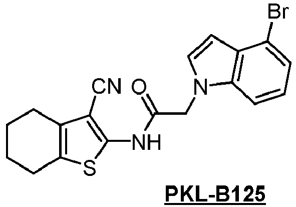

A solution of 4-Bromoindole (0.64 mL, 0.51 mmol), potassium hydroxide (34,34 mg, 0.61 mmol) and TBAB (Catalytic amount 0.02equiv.) in THF was stirred for 30 min at room temperature. Then PKL-B83 (129.9 mg, 0,51 mmol) was added, and the reaction mixture was stirred at room temperature for 18h. The residue was filtered and evaporated. The solid obtained was washed with water and then ethanol, to afford the desired product as a white solid.

¹H NMR (500 MHz, DMSO) δ 12.08 (s, 1H), 7.58 (d, *J* = 3.2 Hz, 1H), 7.52 (d, *J* = 8.2 Hz, 1H), 7.32 (d, *J* = 7.5 Hz, 1H), 7.13 (t, *J* = 7.9 Hz, 1H), 6.50 (dd, *J* = 3.2, 0.8 Hz, 1H), 5.34 (s, 2H), 2.61 (tt, *J* = 4.9, 2.3 Hz, 4H), 1.80 (p, *J* = 2.9 Hz, 4H). ¹³C NMR (126 MHz, DMSO) δ 166.75, 137.43, 131.57, 131.26, 128.85, 127.76, 122.95, 122.44, 114.84, 113.99, 110.12, 101.31, 93.37, 49.15, 23.99, 23.80, 23.06, 22.17.

### Synthesis of tert-butyl (3-cyano-4,5,6,7-tetrahydrobenzo[b]thiophen-2-yl)carbamate (PKL-B61)

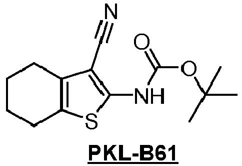

Di-tert-butyl decarbonate (673.41 mg; 3.09 mmol) was added to a stirred solution of aminothiophene (500mg, 3.09 mmol) and Triethylamine 0.4 mL; 3.37) in THF/H2O (4:1). The mixture was stirred for 16 hours at room temperature. Concentrate then add H2O then acidified the mixture to PH=2 with HCl (1M). Aquous layer was extracted with EtOAc (x4) and combined and washed with saturated NaHCO₃ and Brine, dry using NaSO₄, Filter and concentrate.

¹H NMR (500 MHz, CDCl₃) δ 7.38 (s, 1H), 2.57 - 2.51 (m, 2H), 2.49 (t, *J* = 5.7 Hz, 2H), 1.75 (hept, *J* = 3.4 Hz, 4H), 1.46 (s, 9H). ¹³C NMR (126 MHz, CDCl₃) δ 150.29, 147.56, 129.99, 125.94, 113.49, 82.02, 27.12, 22.97, 22.89, 22.10, 21.08.

### Synthesis of tert-butyl (3-cyano-4,5,6,7-tetrahydrobenzo[b]thiophen-2-yl)(prop-2-yn-1-yl) carbamate (PKL-B57)

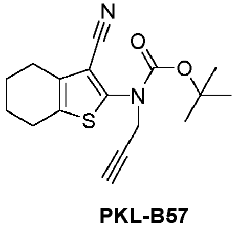

A solution of PKL-B61 (100mg, 0,36 mmol), potassium carbonate (59,58 mg, 0.43 mmol) and TBAB (Catalytic amount 0.2equiv.) in THF was stirred for 30 min at room temperature. Then propargyl bromide solution (0,032ml, 0,43 mmol) was added, and the reaction mixture was stirred at room temperature for 48h. The residue was partitioned between water and ethyl acetate and the aqueous extracted with further ethyl acetate. The combined organic extracts were washed with brine, dried over magnesium sulphate and evaporated to dryness under reduced pressure to give the desired product as a yellow liquid.

¹H NMR (500 MHz, CDCl₃) δ 4.36 (d, *J* = 2.4 Hz, 2H), 2.61 (t, *J* = 5.8 Hz, 2H), 2.56 (t, *J* = 5.8 Hz, 2H), 2.25 (d, *J* = 2.6 Hz, 1H), 1.78 (q, *J* = 7.3, 6.4 Hz, 4H), 1.42 (s, 9H). ¹³C NMR (126 MHz, CDCl₃) δ 152.61, 149.48, 134.26, 133.67, 113.65, 83.15, 78.25, 73.51, 28.04, 24.68, 24.31, 22.97, 22.00.

### Synthesis of ethyl (3-cyano-4,5,6,7-tetrahydrobenzo[b]thiophen-2-yl)glycinate (PKL-B54)

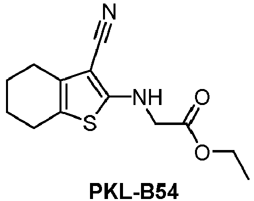

A solution of aminothiophene JNK-1 (100mg, 0,39 mmol), potassium hydroxide (37,77 mg, 0.43 mmol) and TBAB (Catalytic amount 0.2equiv.) in Acetone/H₂O (4:1) was stirred for 30 min at room temperature. Then Bromo ethyl acetate (68 µl, 0,43 mmol) was added, and the reaction mixture was stirred at 60°C for 18h. The residue was partitioned between water and ethyl acetate and the aqueous extracted with further ethyl acetate. The combined organic extracts were washed with brine, dried over magnesium sulphate and evaporated to dryness under reduced pressure, mounted on silica and purified by flash chromatography to afford the desired product as a white solid.

¹H NMR (500 MHz, CDCl₃) δ 5.17 (s, 1H), 4.20 (q, *J* = 7.1 Hz, 2H), 3.91 (s, 2H), 2.44 (q, *J* = 5.3 Hz, 4H), 1.72 (ddtd, *J* = 14.0, 8.6, 6.1, 2.3 Hz, 4H), 1.24 (t, *J* = 7.2 Hz, 3H). 13C NMR (126 MHz, CDCl₃) δ 169.15, 161.23, 133.23, 119.80, 115.74, 86.26, 61.92, 48.39, 24.47, 24.16, 23.34, 22.10, 14.15.

### Synthesis of (3-cyano-4,5,6,7-tetrahydrobenzo[b]thiophen-2-yl)glycine (PKL-B55)

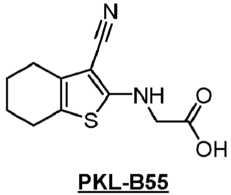

To a solution of PKL-B54 (200 mg; 0.75 mmol) in THF/H₂O (2:1) was added LiOH,H₂O (47.62 mg, 1.13 mmol) and the reaction mixture was stirred at room temperature for 1h. The reaction was quenched with 0.5M aqueous solution of HCl and extracted with EtOAc (20mLx3). The combined organic layers were washed with brine, dried over MgSO₄ and filtered. The solvents were removed under reduced pressure to yield the desired acid.

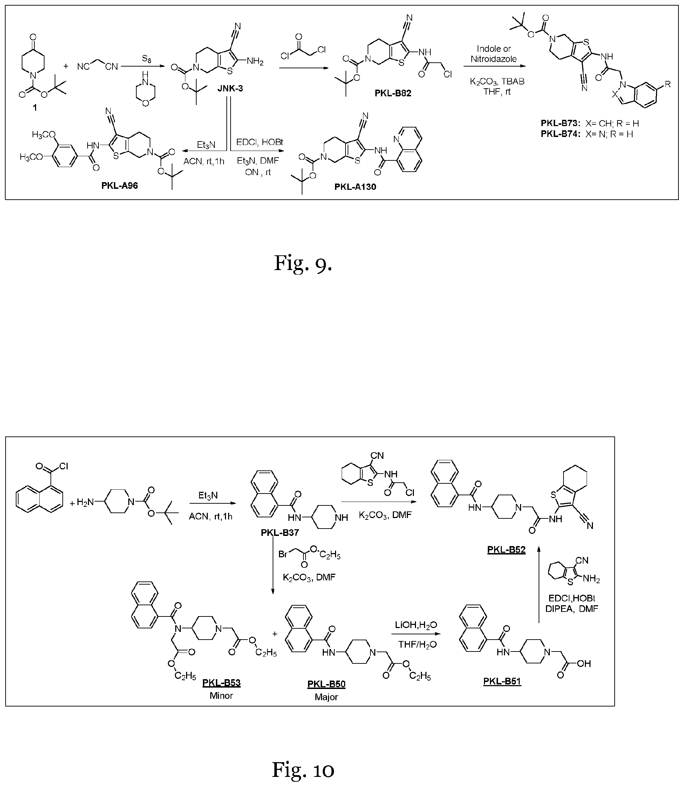

A schematic view of the synthesis of analogues of N-(3-cyano-4,5,6,7-tetrahydrobenzo[b]thiophen-2-yl)-1-naphthamide with a linker PKL-B52 can be seen in Fig. 9 and the synthesis procedure for the specific compounds follows below.

### Synthesis of N-(piperidin-4-yl)-1-naphthamide (PKL-B37)

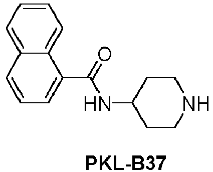

A solution of tert-butyl 4-aminopiperidine-1-carboxylate (1 g, 4.99 mmol), and naphtaloyl chloride (0,1 mL, 5.49 mmol) in Acetonitrile was stirred for 2h at 80°C. Then reaction mixture was cooled and filtered, the reaction was monitored by TLC and LCMSMS, the compound was directly deprotected during the reaction. The liquid part was evaporated to dryness under reduced pressure to give the desired product as a white solid.

### Synthesis of ethyl 2-(4-(1-naphthamido)piperidin-1-yl)acetate PKL-B50 and ethyl 2-(4-(N-(2-ethoxy-2-oxoethyl)-1-naphthamido)piperidin-1-yl)acetate (PKL-B53)

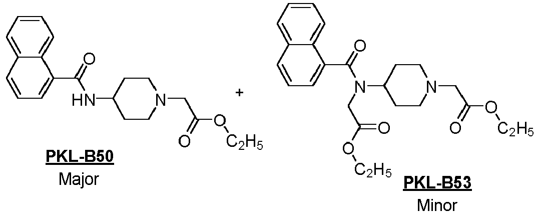

A solution of PKL-B37 (100mg, 0.39 mmol), potassium carbonate (59.77 mg, 0.43 mmol) and TBAB (Catalytic amount 0.2equiv.) in DMF was stirred for 30 min at 60°C. Then Bromo ethyl acetate (47 µl, 0,43 mmol) was added, and the reaction mixture was stirred at room temperature for 48h. The residue was partitioned between water and ethyl acetate and the aqueous extracted with further ethyl acetate. The combined organic extracts were washed with brine, dried over magnesium sulphate and evaporated to dryness under reduced pressure, mounted on silica and purified by flash chromatography to afford the desired products both as transparent liquids.

### Synthesis of 2-(4-(1-naphthamido)piperidin-1-yl)acetic acid (PKL-B51)

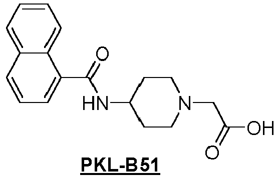

To a solution of PKL-B50 (200 mg; 0.58 mmol) in THF/H₂O (2:1) was added LiOH,H₂O (29.58 mg, 0.70 mmol) and the reaction mixture was stirred at room temperature for 30 min. The reaction was quenched with 0.5M aqueous solution of HCl and extracted with EtOAc (20 mLx3). The combined organic layers were washed with brine, dried over MgSO₄ and filtered. The solvents were removed under reduced pressure to yield the desired acid as a white solid.

### Synthesis of N-(1-(2-((3-cyano-4,5,6,7-tetrahydrobenzo[b]thiophen-2-yl)amino)-2-oxoethyl) piperidin-4-yl)-1-naphthamide (PKL-B52)

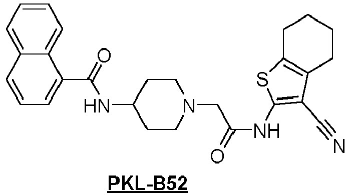

This compound was prepared using two different methods.

Method 1: Following general procedure D, PKL-B82 (60.09 mg, 0.25 mmol), was reacted with PKL-B37 (60 mg, 0.23 mmol), to afford 70 mg (63%) of the title compound as a white solid after column chromatography (EtOAc in Hexane: 0-50%).

Method 2: To solution of PKL-B51 (60 mg, 0.19 mmol), EDCl (65.74 mg, 0.19 mmol) and HOBt (38.93 mg, 0.45 mmol) in DMF, triethyl amine (40.16 µL, 0.29 mmol) was added at 0°C. The mixture was stirred for 30 min at room temperature. Then aminothiophene (34,24 mg, 0,19 mmol) was added, and the reaction mixture was stirred at room temperature for 16h. The residue was poured to ice, and then extracted with ethyl acetate. The combined organic extracts were washed with brine, dried over magnesium sulphate and evaporated to dryness under reduced pressure to afford 76 mg (84%) the desired product as a white solid.

¹H NMR (500 MHz, CDCl₃) δ 10.40 (s, 1H), 8.23 - 8.20 (m, 1H), 7.84 (dt, *J* = 8.3, 1.1 Hz, 1H), 7.81 - 7.78 (m, 1H), 7.53 (dd, *J* = 7.0, 1.2 Hz, 1H), 7.51 - 7.43 (m, 2H), 7.39 (dd, *J* = 8.2, 7.0 Hz, 1H), 3.18 (s, 2H), 2.91 - 2.83 (m, 2H), 2.57 (td, *J* = 5.9, 1.9 Hz, 2H), 2.50 (ddd, *J* = 12.7, 8.3, 6.3 Hz, 4H), 2.14 (dd, *J* = 13.0, 3.8 Hz, 2H), 1.79 - 1.71 (m, 5H), 1.68 (dd, *J* = 12.0, 3.5 Hz, 2H). ¹³C NMR (126 MHz, CDCl₃) δ 169.12, 167.20, 146.47, 134.37, 133.71, 130.77, 130.66, 130.10, 128.35, 128.33, 127.16, 126.44, 125.28, 124.95, 124.73, 114.34, 93.79, 60.55, 53.07, 46.33, 32.64, 31.60, 24.09, 24.02, 23.11, 22.11.

A schematic view of the synthesis of amide analogues of 2-amino-5,6,7,8-tetrahydro-4H-cyclohepta[b]thiophene-3-carbonitrile can be seen in Fig. 6 and the synthesis procedure for the specific compounds follows below.

### Synthesis of 2-amino-5,6,7,8-tetrahydro-4H-cyclohepta[b]thiophene-3-carbonitrile (SI-44)

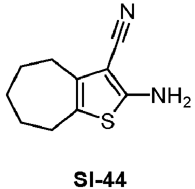

The title compound, was synthesized by preparing a mixture of cycloheptanone (1.0 g, 1 equiv.), malononitrile (0.66 g, 0.63 ml, 1 equiv.), sulphur S₈ (0.32 g, 1 equiv.) and ethanol (5 ml). The obtained mixture was stirred at 45 - 50 °C. Once the temperature was obtained, diethylamine (1.02 ml) was added dropwise until the sulphur completely dissolved. The obtained solid was filtered, washed with ethanol and recrystallized from benzene. Yield 61% ; m.p. 125-126 °C (lit.21 125 °C).

¹H NMR (500 MHz, CDCl₃) δ 4.49 (s, 2H, NH₂), 2.63-2.59 (m, 4H), 1.85-1.81 (m, 2H), 1.68-1.63 (m, 4H).

### General Procedure for the Synthesis of amide analogues of 2-amino-5,6,7,8-tetrahydro-4H-cyclohepta[b]thiophene-3-carbonitrile (1-2)

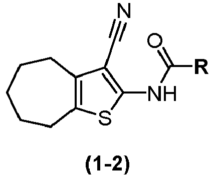

An oven-dried 100 mL round-bottom flask was charged with intermediate compound SI-44 (1 equiv.), dimethoxy benzoyl chloride/1-naphthoyl chloride (1.2 equiv.) and pyridine as solvent and stirred under an N₂ atmosphere for 12h. Reaction was monitored by TLC analysis. The solvent was later removed under reduced pressure. Purification was performed by Silica column chromatography to afford desired product.

### Synthesis of N-(3-cyano-5,6,7,8-tetrahydro-4H-cyclohepta[b]thiophen-2-yl)-1-naphthamide (PKL-A56)

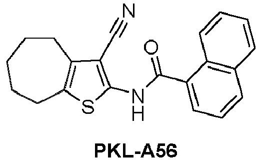

Following general procedure A for the synthesis of PKL-A56, 2-amino-5,6,7,8-tetrahydro-4H-cyclohepta[b]thiophene-3-carbonitrile (SI-44) reacted with 1-naphthoyl chloride to afford (78 %) of the title compound as a white crystal solid after column chromatography (EtOAc in Hexane : 0-30%).

¹H NMR (500 MHz, CDCl₃) δ 8.69 (s, 1H), 8.40 (d, J = 8.3 Hz, 1H), 8.05 (d, J = 8.2 Hz, 1H), 7.93 (d, J = 7.9 Hz, 1H), 7.80 (d, J = 7.1 Hz, 1H), 7.65 - 7.52 (m, 3H), 2.78 (td, J = 5.1, 8.6 Hz, 4H), 1.90 (p, J = 5.6 Hz, 2H), 1.71 (dp, J = 5.5, 16.4 Hz, 4H). ¹³C NMR (126 MHz, CDCl₃) δ 164.33, 143.21, 134.91, 132.82, 131.50, 131.43, 129.96, 129.20, 127.60, 126.92, 125.94, 124.97, 123.97, 123.64, 113.74, 95.63, 31.03, 28.20, 28.08, 27.04, 26.33.

### Synthesis of N-[3-cyano-5,6,7,8-tetrahydro-4H-cyclohepta[b]thiophen-2-yl)-3,4-dimethoxy-benzamide (PKL-A135)

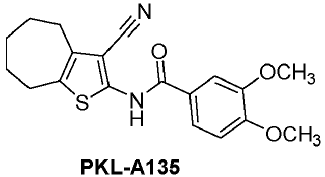

Following general procedure A for the synthesis of PKL-A135, 2-amino-5,6,7,8-tetrahydro-4H-cyclohepta[b]thiophene-3-carbonitrile (SI-44) reacted with Dimethoxy Benzoyl chloride to afford (71%) of the title compound as a white solid after column chromatography (EtOAc in Hexane: 0-40%).

¹H NMR (500 MHz, CDCl₃) δ 8.72 (s, 1H), 7.52 (d, J = 2.1 Hz, 1H), 7.43 (dd, J = 2.1, 8.4 Hz, 1H), 6.94 (d, J = 8.4 Hz, 1H), 3.97 (d, J = 2.6 Hz, 6H), 2.79 - 2.73 (m, 4H), 1.88 (dq, J = 5.7, 8.8 Hz, 2H), 1.73 - 1.65 (m, 4H). ¹³C NMR (126 MHz, CDCl₃) δ 163.01, 153.11, 149.54, 144.78, 135.73, 132.18, 124.25, 119.84, 115.03, 110.85, 110.53, 96.18, 56.20, 56.14, 32.04, 29.18, 29.13, 28.05, 27.35.

A schematic view of the synthesis of amide analogues of 2-aminobenzo[b]thiophene-3-carbonitrile can be seen in Fig. 7 and the synthesis procedure for the specific compounds follows below.

### Synthesis of N-(3-cyanobenzo[b]thiophen-2-yl)-4-methoxy-1-naphthamide (PKL-A149)

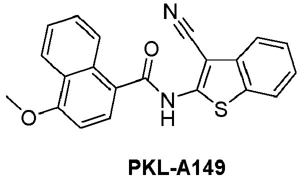

Following general procedure B, to a solution of carboxylic acid (1 equiv.), EDCl (1.2 equiv.) and HOBt (1.2 equiv.) in DMF (0.1-0.3 M), triethylamine (1.5 equiv.) were added at 0°C. The mixture was stirred for 30 min at room temperature. Then, 2-aminobenzo[b]thiophene-3-carbonitrile (1.2 equiv.) was added, and the reaction mixture was stirred at room temperature for 6-16 h, to afford 72 % of the title compound as a white solid after column chromatography (EtOAc in Hexane: 0-20%).

¹H NMR (500 MHz, DMSO) δ 11.74 (s, 1H), 7.52 (d, J = 8.5 Hz, 1H), 7.47 (dd, J = 1.3, 8.4 Hz, 1H), 7.24 (d, J = 8.0 Hz, 1H), 7.15 (d, J = 8.1 Hz, 1H), 6.91 (d, J = 7.9 Hz, 1H), 6.86 (ddd, J = 1.5, 6.8, 8.5 Hz, 1H), 6.79 (ddd, J = 1.3, 6.8, 8.1 Hz, 1H), 6.73 (td, J = 1.1, 7.6 Hz, 1H), 6.63 (td, J = 1.2, 7.2, 7.7 Hz, 1H), 6.33 (d, J = 8.2 Hz, 1H), 3.26 (s, 3H). ¹³C NMR (126 MHz, DMSO) δ 167.73, 158.11, 134.87, 133.39, 131.73, 129.99, 128.51, 126.68, 126.46, 125.33, 125.21, 123.28, 122.44, 120.38, 114-33, 103.70, 88.79, 56.65.

### General Procedure for the Synthesis of amide analogues of 2-aminobenzo[b]thiophene-3-carbonitrile (1-2)

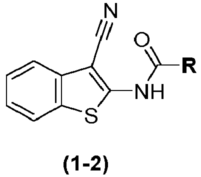

An oven-dried 100 mL round-bottom flask was charged with 2-aminobenzo[b]thiophene-3-carbonitrile (1 equiv.), Dimethoxy Benzoyl chloride/1-Naphthoyl chloride (1.2 equiv.) and pyridine as solvent, stir under an N₂ atmosphere for 3h. Reaction was monitored by TLC analysis. Later solvent was removed under reduced pressure. Purification was performed by (hand column) Silica column chromatography to afford desired product.

### Synthesis of N-(3-cyanobenzo[b]thiophen-2-yl)-1-naphthamide (PKL-A31)

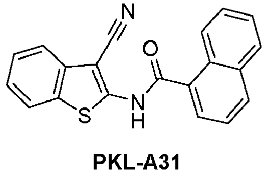

Following general procedure for the synthesis of amide analogues of 2-aminobenzo[b]thiophene-3-carbonitrile, 90 % of the title compound was afforded as a white solid after column chromatography (EtOAc in Hexane : 0-40%). ¹H NMR (500 MHz, CDCl₃) δ 9.04 (s, 1H), 8.49 - 8.44 (m, 1H), 8.10 (d, J = 8.3 Hz, 1H), 7.98 - 7.94 (m, 1H), 7.90 (dd, J = 1.2, 7.2 Hz, 1H), 7.86 (d, J = 8.0 Hz, 1H), 7.83 - 7.79 (m, 1H), 7.69 - 7.63 (m, 1H), 7.60 (dd, J = 7.2, 8.3 Hz, 2H), 7.54 - 7.50 (m, 1H), 7.43 (ddd, J = 1.2, 7.3, 8.3 Hz, 1H). ¹³C NMR (126 MHz, CDCl3) δ 165.92, 150.79, 133.61, 133.15, 128.78, 128.26, 127.16, 126.36, 126.28, 125.22, 124.90, 124.68, 122.42, 120.90, 113.92.

### Synthesis of N-(3-cyanobenzo[b]thiophen-2-yl)-3,4-dimethoxybenzamide (PKL-A142)

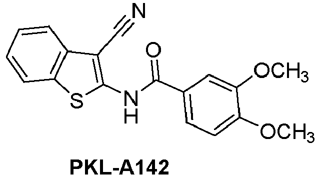

Following general procedure for the synthesis of amide analogues of 2-aminobenzo[b]thiophene-3-carbonitrile, afford 85 % of the title compound as a white solid after column chromatography (EtOAc in Hexane : 0-40%). ¹H NMR (500 MHz, CDCl₃) δ 9.01 (s, 1H), 7.73 (ddt, J = 0.9, 7.9, 12.8 Hz, 2H), 7.51 (d, J = 2.2 Hz, 1H), 7.48 - 7.40 (m, 2H), 7.33 (ddd, J = 1.2, 7.2, 8.3 Hz, 1H), 6.92 (d, J = 8.4 Hz, 1H), 3.92 (d, J = 1.4 Hz, 6H). ¹³C NMR (126 MHz, CDCl₃) δ 163.58, 151.36, 149.68, 133.73, 133-59, 126.20, 125.03, 123.68, 122.37, 120.75, 120.30, 114.19, 110.99, 110.61, 88.50, 56.26, 56.20.

A schematic view of the synthesis of analogues of tert-butyl 2-amino-3-cyano-4,7-dihydrothieno[2,3-c]pyridine-6(5H)-carboxylate can be seen in Fig. 10 and the synthesis procedure for the specific compounds follows below.

### Synthesis of tert-butyl 2-amino-3-cyano-4,7-dihydrothieno[2,3-c]puridine-6(5H)-carboxylate (JNK-3)

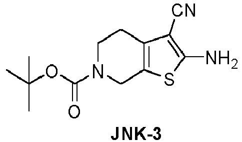

1-Boc-4-piperidone (9 mmol), malononitrile (9 mmol) and sulphur (9 mmol) were dissolved in ethanol (50ml). The mixture was heated to 50 °C and, once the solubilization of sulphur was complete, morpholine (9 mmol) was added dropwise. The reaction mixture was kept at 50°C until the absence of starting material was confirmed by TLC and LCMS/MS (usually between I and 3 hours). Once cooled to room temperature, the desired aminothiophene precipitated, was filtered, and washed with ethanol until the mother liquor became colorless. ¹H NMR (500 MHz, CDCl₃) δ 4.71 (s, 2H), 4.29 (s, 2H), 3.59 (t, J = 5.8 Hz, 2H), 2.52 (d, J = 5.7 Hz, 2H), 1.41 (s, 9H). ¹³C NMR (126 MHz, CDCl₃) δ 160.86, 154.59, 114.85, 88.20, 80.42, 42.59, 42.04, 41-37, 28.42, 24.73.

### Synthesis of tert-butyl 2-(2-chloroacetamido)-3-cyano-4,5,6,7-tetrahydrobenzo[b]thiophene-6-carboxylate (PKL-B82)

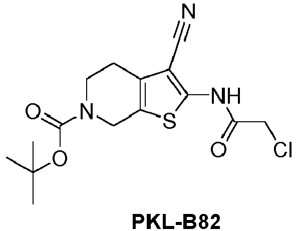

To solution of Amine (200 mg, 0,71 mmol) in DMF, Triethylamine (0.11 mL, 0,78 mmol) was added dropwise to the reaction mixture at 0°C. The mixture was stirred for 30 min at room temperature. Then Chloro acethyl chloride (0.11 mL, 0,79 mmol) was added, and the reaction mixture was stirred at room temperature for 1h. The residue was poured to ice, and then extracted with Chloroform. The combined organic extracts were washed with brine, dried over magnesium sulphate and evaporated to dryness under reduced pressure to give the desired product as a yellow solid.

### Synthesis of tert-butyl 2-(2-(1H-indol-1-yl)acetamido)-3-cyano-4,7-dihydrothieno[2,3-c]pyridine-6(5H)-carboxylate (PKL-B73)

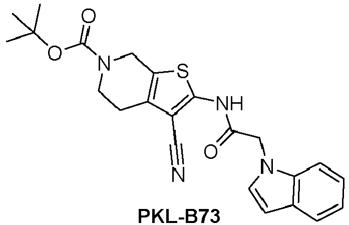

Following general procedure D, PKL-B82 (80.0 mg, 0,22 mmol), was reacted with indole (26.41 mg, 0,22 mmol), to afford 82 mg (83%) of the title compound as a white solid after column chromatography ( EtOAc in Hexane : 0-50%).¹H NMR (500 MHz, CDCl₃) δ 8.41 (s, 1H), 7.63 (d, *J* = 7.8 Hz, 1H), 7.24 - 7.17 (m, 3H), 7.12 (ddd, *J* = 8.0, 6.7, 1.4 Hz, 1H), 7.09 (d, *J* = 3.2 Hz, 1H), 6.64 (d, *J* = 3.2 Hz, 1H), 4.94 (s, 2H), 4.39 (s, 2H), 3.57 (t, *J* = 5.9 Hz, 2H), 2.52 (t, *J* = 5.8 Hz, 2H), 1.39 (s, 10H). ¹³C NMR (126 MHz, CDCl₃) δ 165.72, 154.49, 146.35, 136.18, 129.02, 127.80, 123.06, 121.83, 120.90, 112.93, 108.87, 104.66, 94.34, 80.62, 49.37, 31.60, 28.40, 22.67, 14.14.

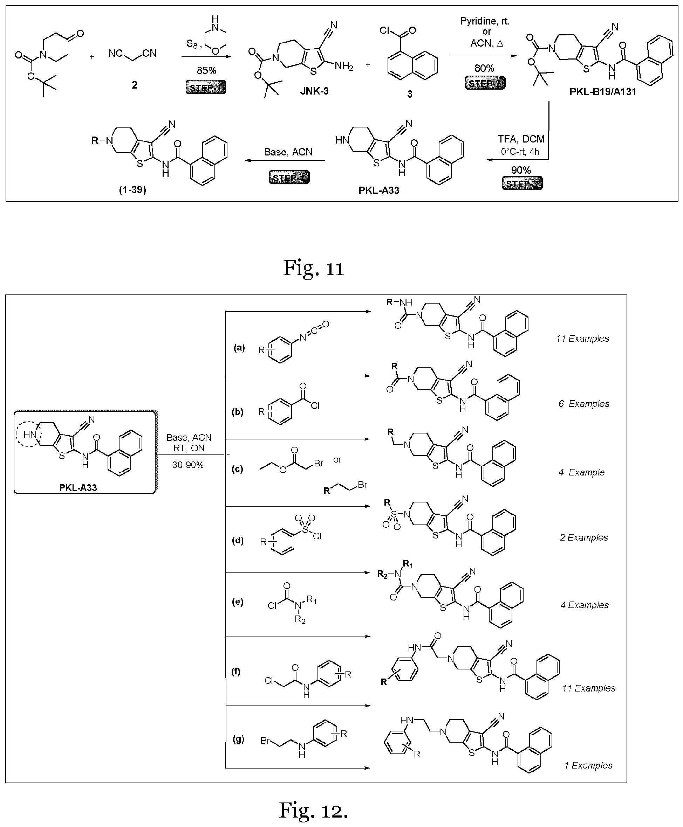

A schematic view of the synthesis of N-(3-cyano-4,5,6,7-tetrahydrothieno[2,3-c]pyridin-2-yl)-1-naphthamide can be seen in Fig. 11-17 and the synthesis procedure for the specific compounds follows below.

### Synthesis of N-(3-cyano-4.5.6.7-tetrahydrothienor[2.3-c]pyridin-2-yl)-1-naphthamide (PKL-B19/A131)

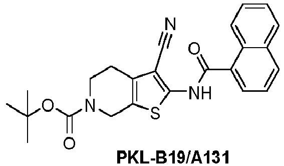

This synthesis can be performed using acetonitrile (ACN) (i) or pyridine as solvent (ii) (step 2 in Fig. 11).
(i): A solution of JNK-3 (1 g, 3.58 mmol) in ACN (5 ml) with 1-naphthoyl chloride (1 mL, 3.94 mmol) was stirred together at 80 °C under an inert atmosphere for 1h. The reaction was monitored by TLC and LCMS/MS.
(ii): A solution of JNK-3 (1 g, 3.58 mmol) in pyridine (3 ml) with 1-naphthoyl chloride (1 mL, 3.94 mmol) was stirred together at room temperature under an inert atmosphere overnight. The reaction was monitored by TLC and LCMS/MS. The mixture was then evaporated to dryness and chromatographed over silica gel using with a gradient of hexane/EtOAc (9:1 to 7:3). The product fractions were collected and evaporated to afford 80% of the title compound.

¹H NMR (500 MHz, CDCl₃) δ 8.03 (dd, J = 1.1, 8.3 Hz, 2H), 7.76 (dd, J = 1.1, 7.1 Hz, 2H), 7.54 - 7.49 (m, 2H), 7.45 (ddd, J = 1.4, 6.8, Hz, 2H), 7.42 - 7.35 (m, 4H), 7.00 - 6.95 (m, 2H), 4.65 (s, 2H), 3.78 (s, 2H), 2.87 (d, J = 5.7 Hz, 2H), 1.51 (s, 9H). ¹³C NMR (126 MHz, CDCl₃) δ 171.38, 146.00, 132.98, 132.21, 131.69, 129.46, 128.10, 127.60, 126.96, 126.68, 124.75, 123.97, 113.08, 80.80, 28.43, 24.74.

### Synthesis of N-(3-cyano-4,5,6,7-tetrahydrothieno[2,3-c]pyridin-2-yl)-1-naphthamide (PKL-A33)

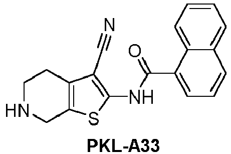

Following the general procedure G, PKL-B19/A131 (1 g, 2.31 mmol) in methylene chloride (DCM, 5.5 mL), reacted with TFA (1 mL) to afford 90% the desired product as a white solid after column chromatography using methanol in EtOAc: 95-5%.

### General Procedure for the Synthesis of N-functionalization of N(3-cyano-4,5,6,7-tetrahydrothieno[2,3-c]pyridin-2-yl)-1-naphthamide (1-24)

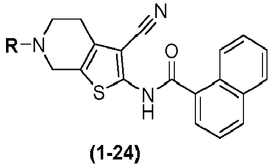

To a solution of the PKL-A33 in acetonitrile (0.1 M), triethylamine (1.2 equiv.) and the selected acylchloride/isocyanate/carbamoyl chlorides/alkyl halide/benzoyl chlorides/sulfonyl chloride (1 to 1.2 equiv.) was added. The reaction mixture was stirred at room temperature for overnight, concentrated, mounted on silica and purified by flash chromatography to afford the desired compounds.

### Synthesis of representative molecule PKL-A152

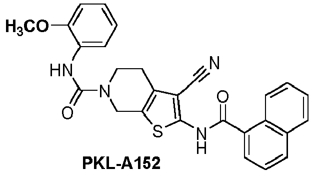

Following step 1-3 (see Fig. 11), to obtain N-(3-cyano-4,5,6,7-tetrahydrothieno[2,3-c]pyridin-2-yl)-1-naphthamide.

First step, 1-Boc-4-piperidone (9 mmol), malononitrile (9 mmol) and sulphur (9 mmol) were dissolved in ethanol (50ml). The mixture was heated to 50 °C and, once the solubilization of sulphur was complete, morpholine (9 mmol) was added dropwise. The reaction mixture was kept at 50 °C until the absence of starting material was confirmed by TLC and LCMS/MS (usually between 1 and 3 hours). Once cooled to room temperature, the desired aminothiophene precipitated, was filtered, and washed with ethanol until the mother liquor became colorless.

Second step can be performed using acetonitrile (ACN) (i) or pyridine as solvent (ii) as mentioned above.
(i) A solution of JNK-3 (1 g, 3.58 mmol) in ACN (5 ml) with 1-naphthoyl chloride (1 mL, 3.94 mmol) was stirred together at 80 °C under an inert atmosphere for 1 h. The reaction was monitored by TLC and LCMS/MS.
(ii) A solution of JNK-3 (1 g, 3.58 mmol) in pyridine (3 ml) with 1-Naphthoyl chloride (1mL, 3.94 mmol) was stirred together at room temperature under an inert atmosphere overnight. The reaction was monitored by TLC and LCMS/MS.

The mixture was then evaporated to dryness and chromatographed over silica gel eluting using a gradient of hexane/EtOAc (9:1 to 7:3). The product fractions were collected and evaporated to afford the title compound.

Third step, to a solution of protected amine in methylene chloride (DCM), trifluoroacetic acid (TFA) (DCM: TFA 4:1) was added in the presence of nitrogen. The mixture was stirred at room temperature for 4 hours. The reaction progress and results were confirmed by TLC and LCMS/MS. When the reaction was completed, the reaction mixture was concentration under reduced pressure, and crystallized by adding ether or ethanol to obtain the target compound PKL-A33.

Step 4, to a solution of the PKL-A33 in acetonitrile (0.1 M), Triethylamine (1.2 equiv.) and the 1-isocyanato-2-methoxybenzene (1.2 equiv.) was added. The reaction mixture was stirred at room temperature for 2 hours, concentrated, mounted on silica and purified by flash chromatography to afford the desired compounds.

¹H NMR (500 MHz, DMSO) δ 12.43 (s, 1H), 8.18 - 8.12 (m, 2H), 8.09 - 8.03 (m, 1H), 7.91 (s, 1H), 7.84 (dd, J = 1.2, 7.1 Hz, 1H), 7.69 - 7.62 (m, 3H), 7.58 (dd, J = 1.6, 7.9 Hz, 1H), 7.09 - 7.00 (m, 2H), 6.89 (td, J = 1.7, 7.5 Hz, 1H), 4.65 (d, J = 1.8 Hz, 2H), 3.83 (s, 3H), 3.80 (t, J = 5.7 Hz, 2H), 2.72 (t, J = 5.7 Hz, 2H). ¹³C NMR (126 MHz, DMSO) δ 167.30, 155.51, 151.30, 147.67, 133.53, 132.19, 131.62, 131.02, 130.21, 128.97, 128.70, 127.89, 127.28, 127.01, 125.84, 125.39, 125.19, 124.50, 123.94, 120.63, 114.28, 111.53, 94-75, 56.14, 43.04, 41.62, 24.27.

### Synthesis of representative molecule PKL-A157

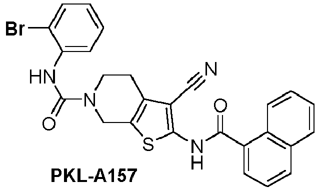

Following steps 1-3 (see Fig. 11), to obtain N-(3-cyano-4,5,6,7-tetrahydrothieno[2,3-c]pyridin-2-yl)-1-naphthamide.

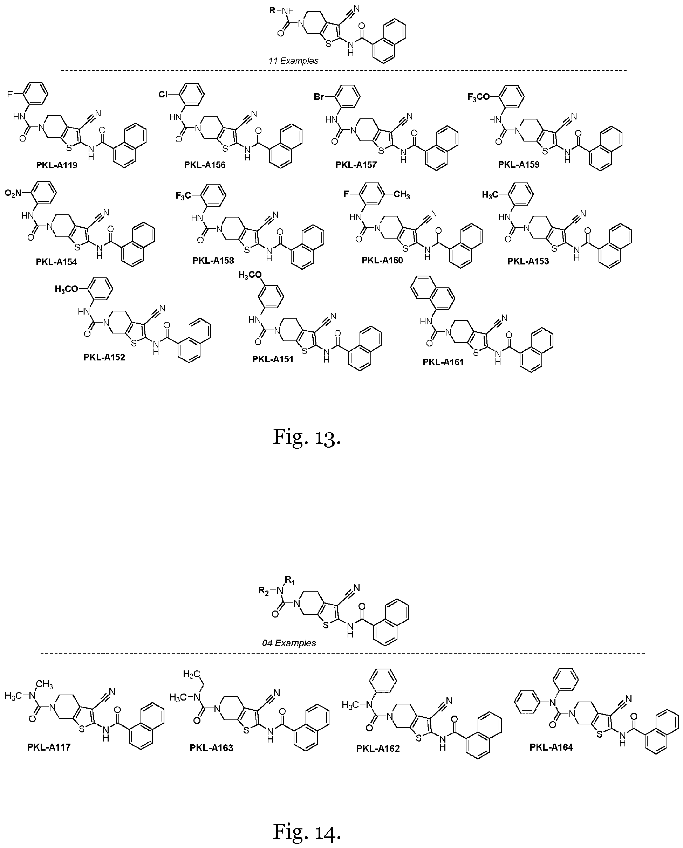

Step 4, to a solution of the PKL-A33 in acetonitrile (0.1 M), triethylamine (1.2 equiv.) and 1-bromo-2-isocyanatobenzene (1.2 equiv.) was added. The reaction mixture was stirred at room temperature for 2 hours, concentrated, mounted on silica and purified by flash chromatography to afford the desired compounds (see Fig. 13).

¹H NMR (500 MHz, DMSO) δ 12.43 (s, 1H), 8.45 (s, 1H), 8.18 - 8.11 (m, 2H), 8.09 - 8.04 (m, 1H), 7.84 (dd, J = 1.2, 7.1 Hz, 1H), 7.68 - 7.61 (m, 4H), 7.48 (dd, J = 1.6, 8.0 Hz, 1H), 7.36 (td, J = 1.5, 7.7 Hz, 1H), 7.12 (td, J = 1.7, 7.7 Hz, 1H), 4.67 (d, J = 1.7 Hz, 2H), 3.83 (t, J = 5.7 Hz, 2H), 2.74 (t, J = 5.7 Hz, 2H). ¹³C NMR (126 MHz, DMSO) δ 167.31, 155.54, 147.66, 138.19, 133.53, 132.91, 132.18, 131.63, 131.01, 130.22, 128.98, 128.60, 128.37, 127.90, 127.29, 127.07, 127.02, 125.40, 125.20, 120.58, 114.28, 94.81, 43.05, 41-78, 24.30.

### Synthesis of representative molecule PKL-A158

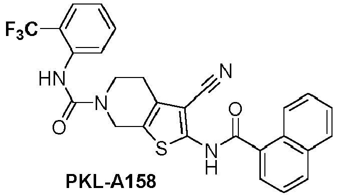

Following steps 1-3 (see Fig. 11), to obtain N-(3-cyano-4,5,6,7-tetrahydrothieno[2,3-c]pyridin-2-yl)-1-naphthamide.

Step 4, to a solution of the PKL-A33 in acetonitrile (1 equiv., 0.1 M), triethylamine (1.2 equiv.) and 1-isocyanato-2-(trifluoromethyl) benzene (1.2 equiv.) was added. The reaction mixture was stirred at room temperature for 2 hours, concentrated, mounted on silica and purified by flash chromatography to afford the desired compounds (see Fig. 13).

¹H NMR (500 MHz, DMSO) δ 12.43 (s, 1H), 8.51 (s, 1H), 8.15 (t, J = 8.0 Hz, 2H), 8.08 - 8.04 (m, 1H), 7.84 (dd, J = 1.2, 7.0 Hz, 1H), 7.71 (dd, J = 1.5, 7.8 Hz, 1H), 7.64 (qdd, J = 2.1, 7.0, 10.5 Hz, 4H), 7.44 (dd, J = 7.8, 11.2 Hz, 2H), 4.64 (t, J = 1.7 Hz, 2H), 3.81 (t, J = 5.7 Hz, 2H), 2.71 (t, J = 5.7 Hz, 2H). ¹³C NMR (126 MHz, DMSO) δ 166.36, 155.42, 132.56, 132.29, 130.65, 130.44, 129.97, 129.25, 128.00, 126.92, 126.32, 126.04, 125.80, 124.42, 124.24, 42.03, 40.80, 39.52, 23.18.

### Synthesis of representative molecule PKL-A164

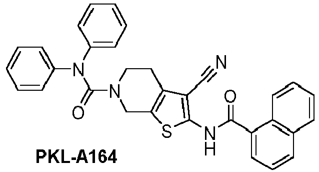

Following steps 1-3 (see Fig. 11), to obtain N-(3-cyano-4,5,6,7-tetrahydrothieno[2,3-c]pyridin-2-yl)-1-naphthamide.

Step 4, to a solution of the PKL-A33 in acetonitrile (1 eqiuv., 0.1 M), Triethylamine (1.2 equiv.) and diphenylcarbamic chloride (1.2 equiv.) was added. The reaction mixture was stirred at room temperature for 6 hours, concentrated, mounted on silica and purified by flash chromatography to afford the desired compounds (see Fig. 14).

¹H NMR (500 MHz, DMSO) δ 11.57 (s, 1H), 7.32 (dt, *J* = 1.0, 8.3 Hz, 1H), 7.30 - 7.27 (m, 1H), 7.24 - 7.20 (m, 1H), 6.99 (dd, *J* = 1.2, 7.2 Hz, 1H), 6.84 - 6.76 (m, 3H), 6.56 - 6.50 (m, 4H), 6.38 - 6.32 (m, 2H), 6.25 - 6.20 (m, 4H), 3.63 (d, *J* = 1.8 Hz, 2H), 2.79 (t, *J* = 5.6 Hz, 2H), 1.46 (t, *J* = 5.7 Hz, 2H). ¹³C NMR (126 MHz, DMSO) δ 170.81, 167.28, 159.63, 145.05, 133.52, 132.17, 131.61, 130.62, 130.19, 129.80, 128.97, 127.89, 127.26, 127.01, 125.55, 125.38, 125.28, 125.17, 114.18, 60.23, 44.13, 43.41.

### Synthesis of representative molecule PKL-A162

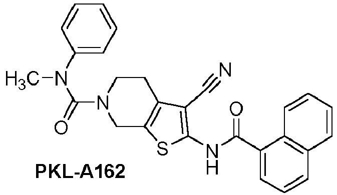

Following steps 1-3 (see Fig. 11), to obtain N-(3-cyano-4,5,6,7-tetrahydrothieno[2,3-c]pyridin-2-yl)-1-naphthamide.

Step 4, to a solution of the PKL-A33 in acetonitrile (1 equiv., 0.1 M), triethylamine (1.2 equiv.) and methyl(phenyl)carbamic chloride (1.2 equiv.) was added. The reaction mixture was stirred at room temperature for 6 hours, concentrated, mounted on silica and purified by flash chromatography to afford the desired compounds (see Fig. 14).

¹H NMR (500 MHz, DMSO) δ 11.55 (s, 1H), 7.31 (d, J = 8.2 Hz, 1H), 7.27 (d, J = 7.3 Hz, 1H), 7.24 - 7.18 (m, 2H), 6.98 (d, J = 7.2 Hz, 1H), 6.82 - 6.76 (m, 4H), 6.56 (t, J = 7.9 Hz, 2H), 6.38 (d, J = 7.7 Hz, 2H), 6.35 (d, J = 7.3 Hz, 1H), 3.45 (s, 2H), 2.60 (d, J = 5.8 Hz, 2H), 2.31 (d, J = 3.5 Hz, 3H). ¹³C NMR (126 MHz, DMSO) δ 167.29, 160.80, 146.67, 133.51, 131.59, 130.67, 130.19, 129.97, 128.96, 127.87, 127.25, 126.99, 125.37, 125.19, 125.04, 124.14, 44.38, 43-36, 23.75, 14.56.

### Synthesis of representative molecule PKL-A163

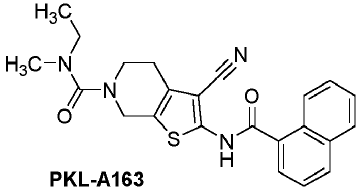

Following steps 1-3 (see Fig. 11), to obtain N-(3-cyano-4,5,6,7-tetrahydrothieno[2,3-c]pyridin-2-yl)-1-naphthamide.

Step 4, to a solution of the PKL-A33 in acetonitrile (1 equiv., 0.1 M), triethylamine (1.2 equiv.) and the methyl(ethyl)carbamic chloride (1.2 equiv.) was added. The reaction mixture was stirred at room temperature for 6 hours, concentrated, mounted on silica and purified by flash chromatography to afford the desired compounds.

¹H NMR (500 MHz, DMSO) δ 11.55 (s, 1H), 7.32 (dd, J = 1.2, 8.3 Hz, 1H), 7.31 - 7.27 (m, 1H), 7.24 - 7.21 (m, 1H), 6.99 (dd, J = 1.2, 7.1 Hz, 1H), 6.84 - 6.77 (m, 3H), 3.47 (d, J = 1.8 Hz, 2H), 2.61 (t, J = 5.6 Hz, 2H), 2.34 (q, J = 7.1 Hz, 2H), 1.97 (s, 3H), 1.88 - 1.84 (m, 2H), 0.26 (t, J = 7.1 Hz, 3H). ¹³C NMR (126 MHz, DMSO) δ 167.27, 163.74, 133.51, 132.23, 131.58, 130.90, 130.20, 128.96, 127.87, 127.23, 127.00, 125.38, 125.18, 45.42, 44.65, 44.26, 35.54, 24.12, 12.88.

### Synthesis of representative molecule PKL-A179

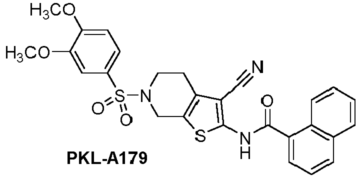

Following steps 1-3 (see Fig. 11), to obtain N-(3-cyano-4,5,6,7-tetrahydrothieno[2,3-c]pyridin-2-yl)-1-naphthamide PKL-A33.

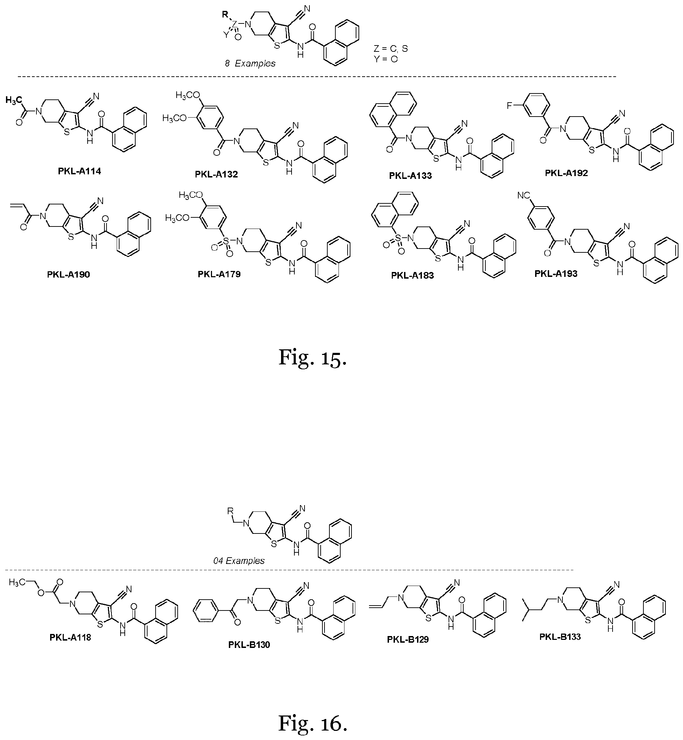

Step 4, to a solution of PKL-A33 in acetonitrile (1 equiv., 0.1 M), triethylamine (1.2 equiv.) and 3,4-dimethyl sulfonyl chloride (1.2 equiv.) were added. The reaction mixture was stirred at room temperature for 3 hours, concentrated, mounted on silica and purified by flash chromatography to afford the desired compounds (see Fig. 15).

¹H NMR (500 MHz, DMSO) δ 11.59 (s, 1H), 7.31 (dd, *J* = 1.1, 8.3 Hz, 1H), 7.27 (dd, *J* = 1.5, 8.2 Hz, 1H), 7.24 - 7.21 (m, 1H), 6.98 (dd, *J* = 1.2, 7.1 Hz, 1H), 6.83 - 6.77 (m, 4H), 6.60 (dd, *J* = 2.2, Hz, 1H), 6.38 (d, *J* = 2.2 Hz, 1H), 6.33 (d, *J =*

Hz, 1H), 3.50 (d, *J* = 1.8 Hz, 2H), 3.01 (d, *J* = 7.2 Hz, 6H), 2.61 (t, *J* = 5.9 Hz, 2H), 1.77 (t, *J =* 5.9 Hz, 2H). ¹³C NMR (126 MHz, DMSO) δ 167.31, 153.09, 149.21, 147.95, 133^{.}51, 132.12, 131.62, 130.18, 130.11, 128.97, 128.59, 127.90, 127.28, 127.02, 125.38, 125.14, 123.67, 121.64, 114.06, 111.88, 110.25, 94.15, 56.40, 56.32, 44.67, 43.29, 23.79.

### Synthesis of representative molecule PKL-A114

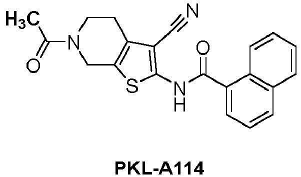

Following steps 1-3 (see Fig 11), to obtain N-(3-cyano-4,5,6,7-tetrahydrothieno[2,3-c]pyridin-2-yl)-1-naphthamide.

Step 4, to a solution of the PKL-A33 in acetonitrile (0.1 M), triethylamine (1.2 equiv.) and acetic anhydride (1.2 equiv.) were added. The reaction mixture was stirred at room temperature for 4 hours, concentrated, mounted on silica and purified by flash chromatography to afford the desired compounds (see Fig. 15).

¹H NMR (500 MHz, DMSO) δ 11.60 (d, J = 13.1 Hz, 1H), 7.35 - 7.29 (m, 2H), 7.25 - 7.22 (m, 1H), 7.01 (dd, J = 1.2, 7.1 Hz, 1H), 6.82 (dddd, J = 1.5, 5.1, 6.6, 9.6 Hz, 3H), 3.88 - 3.77 (m, 2H), 2.93 (dt, J = 5.8, 13.6 Hz, 2H), 1.95 - 1.75 (m, 2H), 1.29 (d, J = 17.6 Hz, 3H). ¹³C NMR (126 MHz, DMSO) δ 169.31, 147.71, 133.52, 132.15, 131.63, 131.12, 130.81, 130.20, 128.97, 127.89, 127.29, 127.01, 125.53, 125.38, 125.18, 114.27, 55.39, 44.56, 43.28, 24.89, 21.76.

### Synthesis of representative molecule PKL-A118

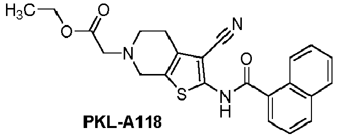

Following steps 1-3 (see Fig. 11), to obtain N-(3-cyano-4,5,6,7-tetrahydrothieno[2,3-c]pyridin-2-yl)-1-naphthamide PKL-A33.

Step 4, to a solution of the PKL-A33 in acetonitrile (1 equiv., 0.1 M), triethylamine (1.2 equiv.) and ethyl bromo acetate (2.0 equiv.) were added. The reaction mixture was stirred at room temperature for 4 hours, concentrated, mounted on silica and purified by flash chromatography to afford the desired compounds (see Fig. 16).

¹H NMR (500 MHz, DMSO) δ 8.89 (s, 1H), 8.41 (dd, J = 1.2, 8.4 Hz, 1H), 8.05 (dt, J = 1.1, 8.3 Hz, 1H), 7.96 - 7.91 (m, 1H), 7.82 (dd, J = 1.2, 7.0 Hz, 1H), 7.61 - 7.52 (m, 3H), 4.23 (q, J = 7.1 Hz, 2H), 3.83 (t, J = 1.8 Hz, 2H), 3.45 (s, 2H), 2.99 (t, J = 5.8 Hz, 2H), 2.78 (t, J = 5.7 Hz, 2H), 1.30 (s, 3H). ¹³C NMR (126 MHz, DMSO) δ 165.47, 160.70, 142.53, 129.10, 127.95, 125.98, 125.47, 124.68, 123.92, 123.29, 122.26, 121.43, 120.21, 119.89, 109.22, 88.85, 56.17, 53.17, 45.52, 44.65, 24.96, 19.32, 9.52, - 3.72.

### Preparation of intermediate 1-(2-bromoethyl)-1H-benzo[d]imidazole

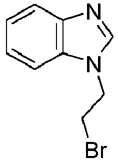

A solution of benzimidazol (200 mg, 1.69 mmol), potassium hydroxide (104.48 mg, 1.86 mmol) and tetrabutylammonium bromide (TBAB, catalytic amount 0.02 equiv.) in THF was stirred for 30 min at room temperature. Then dibromoethane (0.5 mL, 1.69 mmol) was added, and the reaction mixture was stirred at room temperature for 18 h. The residue was partitioned between water and ethyl acetate and the aqueous extracted with further ethyl acetate. The combined organic extracts were washed with brine, dried over magnesium sulphate and evaporated to dryness under reduced pressure to afford 250mg (65 %) the desired product as a white solid.

### N-(6-(2-(1H-benzo[d]imidazol-1-yl)ethyl)-3-cyano-4,5,6,7-tetrahydrothieno[2,3-c]pyridin-2-yl)-1-naphthamide (PKL- B153)

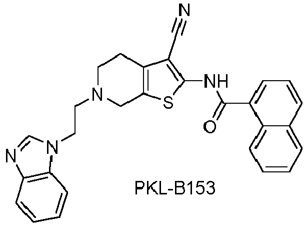

Following steps 1-3 (see Fig. 11), to obtain N-(3-cyano-4,5,6,7-tetrahydrothieno[2,3-c]pyridin-2-yl)-1-naphthamide.

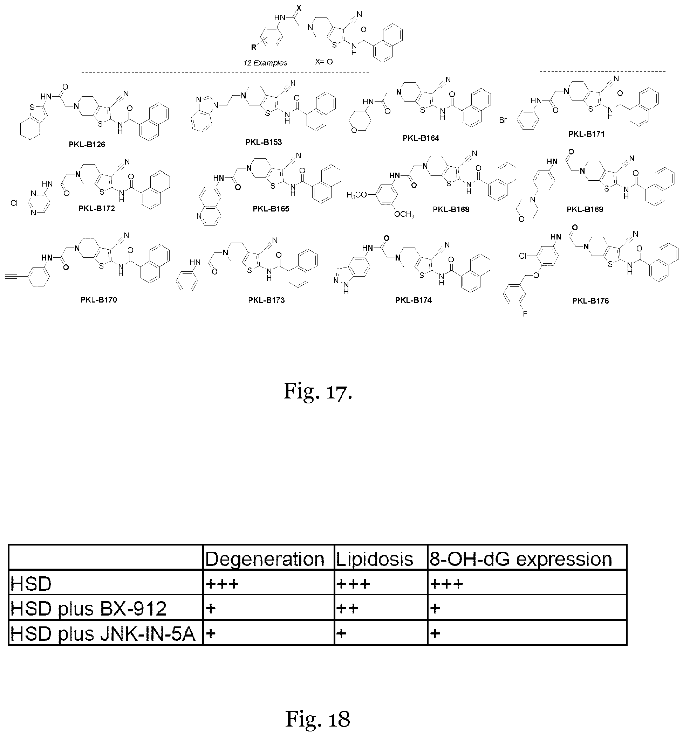

Step 4, following general procedure D, PKL-A33 ((100 mg, 2.99 mmol), was reacted with 1-(2-bromoethyl)-1H-benzo[d]imidazole (67.51 mL, 2.99 mmol), to afford 95 mg (66 %) of the title compound as a yellow solid after column chromatography (MeOH in DCM : 0-10%) (see Fig. 17). ¹H NMR (500 MHz, DMSO) δ 8.31 (s, 1H), 8.13 (d, *J* = 8.4 Hz, 1H), 8.08 - 8.04 (m, 1H), 7.92 (d, *J* = 7.0 Hz, 1H), 7.75 - 7.58 (m, 6H), 7.29 (dt, *J* = 32.7, 7.4 Hz, 2H), 4.51 (t, *J* = 6.3 Hz, 2H), 3.70 (s, 2H), 2.99 (t, *J* = 6.2 Hz, 2H), 2.89 (t, *J* = 5.7 Hz, 2H), 2.63 (d, *J* = 5.6 Hz, 2H).¹³C NMR (126 MHz, DMSO) δ 144.87, 143.78, 134.40, 133.61, 131.92, 130.90, 130.55, 129.76, 128.78, 127.36, 127.22, 126.66, 125.87, 125.38, 122.64, 121.81, 121.73, 119.83, 111.01, 110.87, 55-90, 51.06, 49.55, 42.37, 24.11.

### Preparation of intermediate 2-chloro-N-(3-cyano-4,5,6,7-tetrahydrobenzo[b]thiophen-2-yl) acetamide (PKL-B83)

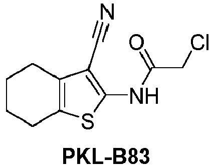

To solution of aminothiophene (200 mg, 1.12 mmol) in DMF, triethylamine (0.17 mL, 1.23 mmol) was added dropwise to the reaction mixture at 0 °C. The mixture was stirred for 30 min at room temperature. Then chloroacetyl chloride (0.1 mL, 1.23 mmol) was added, and the reaction mixture was stirred at room temperature for 1h. The residue was poured to ice, and then extracted with chloroform. The combined organic extracts were washed with brine, dried over magnesium sulphate and evaporated to dryness under reduced pressure to afford 230 mg (81%) of the desired product as a brown solid.

¹H NMR (500 MHz, CDCl₃) δ 9.22 (s, 1H), 4.21 (s, 2H), 2.59 (ddd, *J* = 7.8, 5.3,1.8 Hz, 2H), 2.54 (td, *J* = 6.0, 1.8 Hz, 2H), 1.81 - 1.73 (m, 5H). ¹³C NMR (126 MHz, CDCl₃) δ 162.80, 145.03, 131.40, 129.46, 113.79, 95.15, 42.03, 24.03, 23.98, 23.03, 22.04.

### N-[3-cyano-6-(2-oxo-2-((4,5,6,7-tetrahydrobenzo[b]thiophen-2-yl)amino)ethyl)-4.5.6.7-tetrahydrothieno[2.3-c]pyridin-2-yl)-1-naphthamide (PKL-B126)

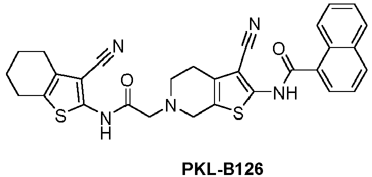

Following steps 1-3 (see Fig. 11), to obtain N-(3-cyano-4,5,6,7-tetrahydrothieno[2,3-c]pyridin-2-yl)-1-naphthamide.

Step 4, following general procedure D, PKL-A33 ((100 mg, 2.99 mmol), was reacted with 1-(2-bromoethyl)-1H-benzo[d]imidazole (67.51 mL, 2.99 mmol), to afford 95 mg (66 %) of the title compound as a yellow solid after column chromatography ( EtOAc in Hexane : 0-50%). ¹H NMR (500 MHz, DMSO-*d*₆) δ 12.47 (s, 1H), 8.22 (dd, *J* = 10.7, 7.9 Hz, 2H), 8.18 - 8.12 (m, 2H), 7.91 (d, *J* = 6.9 Hz, 1H), 7.75 - 7.68 (m, 3H), 3.93 (h, *J* = 6.4, 4.6 Hz, 2H), 3.71 (s, 2H), 3.07 (s, 2H), 2.79 (d, *J* = 5.7 Hz, 2H), 2.72 - 2.65 (m, 3H), 1.84 (dt, *J* = 7.7, 4.3 Hz, 5H). NMR (126 MHz, DMSO) δ 167.25, 146.53, 133.53, 132.23, 131.60, 131.25, 131.22, 130.27, 130.22, 129.95, 128.97, 128.11, 127.87, 127.26, 127.00, 125.44, 125.38, 125.20, 114.63, 114.37, 94.69, 93.94, 50.46, 49-42, 29.50, 29.47, 23.98, 23.86, 23.07, 22.16.

### Results

### The treatment of rats with therapeutic agents

To evaluate the potential toxicity of BX-912 and JNK-IN-5A in animals, three groups of 12-week-old rats (n = 5) were raised. The first group was fed with CHOW (Ctrl) for seven days. The second group was fed with CHOW plus BX-912 (30 mg/kg) for seven days. The third group was fed with CHOW plus JNK-IN-5A (30 mg/kg) for seven days. After the seven days, all rats were sacrificed, and tissue samples from major organs and blood samples were collected for biosafety evaluation. The frequencies of micronucleated polychromatic erythrocytes (MNPCEs) in the blood samples were analysed, and it was found that the frequencies of MNPCEs in the drug-treated groups were not statistically different (p>0.05) when compared to Ctrl animals. The analysis indicated that both tested agents exhibited non-genotoxic potential. In addition, histopathological examinations were performed using the tissue samples obtained from the rats and it was found that kidney, small intestine, large intestine, stomach, heart, pancreas, muscle tissues and liver from all three rat groups exhibited normal histological structures. Moreover, immunohistochemical (IHC) examination on the liver tissues from the rats was performed and negative 8-OH-dG expression was observed in all three groups. Therefore, it was concluded that both BX-912 and JNK-IN-5A had no side effects on the rats.

Next, the efficacy of these two drugs in a steatosis rat study was investigated by feeding the animals with HSD. Five groups of 12-week-old rats (n = 5) were raised. For two weeks, the first group was fed with CHOW and the remaining four groups were fed with HSD. At the end of week 2, the CHOW-fed rats (n = 5) and one group of HSD-fed rats (n = 5) were sacrificed for histopathological examination and it was confirmed that the HSD fed rats had already developed hepatic steatosis. One of the remaining groups was fed with HSD (HSD 3w) for seven days. Another one of the remaining groups was fed with HSD plus BX-912 (30 mg/kg) for seven days. The last one of the remaining groups was fed with HSD plus JNK-IN-5A (30 mg/kg) for seven days. After said seven days, all three groups were sacrificed, and tissue samples from the liver and other major organs and blood samples were collected for efficacy examination. A histopathological and immunohistochemical analysis was performed on the liver samples and other major organs obtained from the three rat groups. A standard histological structure on the small intestine, large intestine, stomach, heart and muscle tissues was observed in the examination of the drug-treated groups, and no toxic effect of the drugs on these tissues was found. In the rats from HSD 3w group, severe degeneration was detected in the renal tubule epithelium and severe fattening was detected in the parenchyma cells in the pancreatic tissues. In contrast, only mild degeneration in renal tubule epithelium and mild fattening in the parenchyma cells of pancreas tissues were detected in the drug-treated groups. In addition, severe degeneration and steatosis in hepatocytes in liver tissues of the rats from the HSD 3w group were observed. Mild degeneration and moderate steatosis were observed in hepatocytes in liver tissues of the HSD-fed rats treated with BX-12, whereas both degeneration and steatosis were mild in hepatocytes in liver tissues of the JNK-IN-5A-treated group (Fig. 3). Moreover, strong 8-OH-dG expression was observed in hepatocytes in liver tissues of the rats from HSD 3w group. In the drug-treated groups, the 8-OH-dG expression was however significantly decreased, see Fig. 18.

The rat studies, hence, showed that treating HSD fed rats with JNK-IN-5A decreased the degeneration and steatosis in hepatocytes in liver tissues.

The present inventors realized that the effect of the JNK-IN-5A can be tuned by modifying the chemical structure of the JNK-IN-5A molecule. Molecular docking was performed to select the target molecules which were then synthesised, and their effectiveness analysed.

### Analysis of target compounds

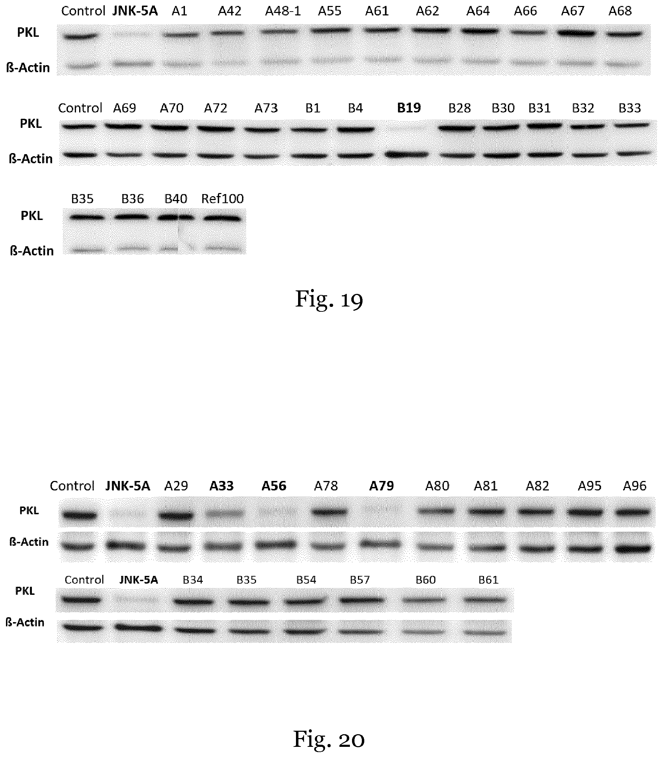

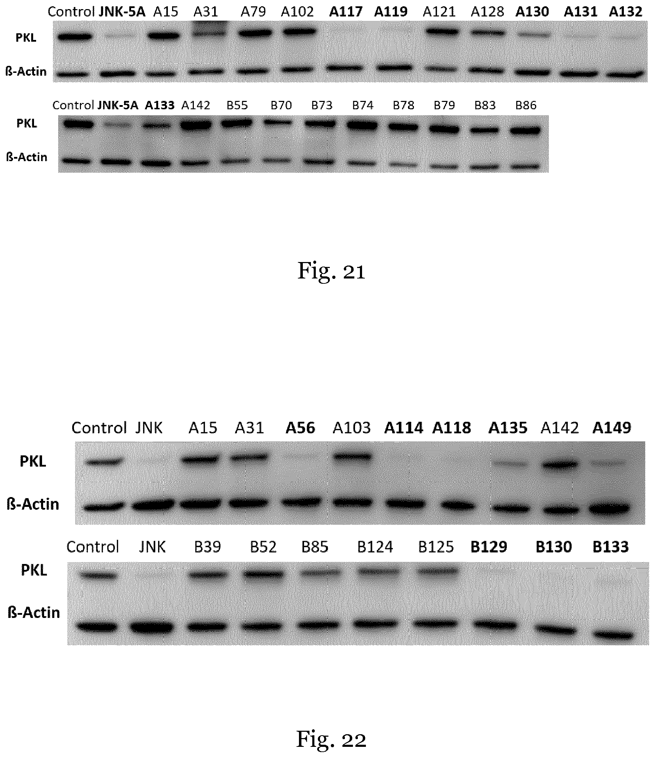

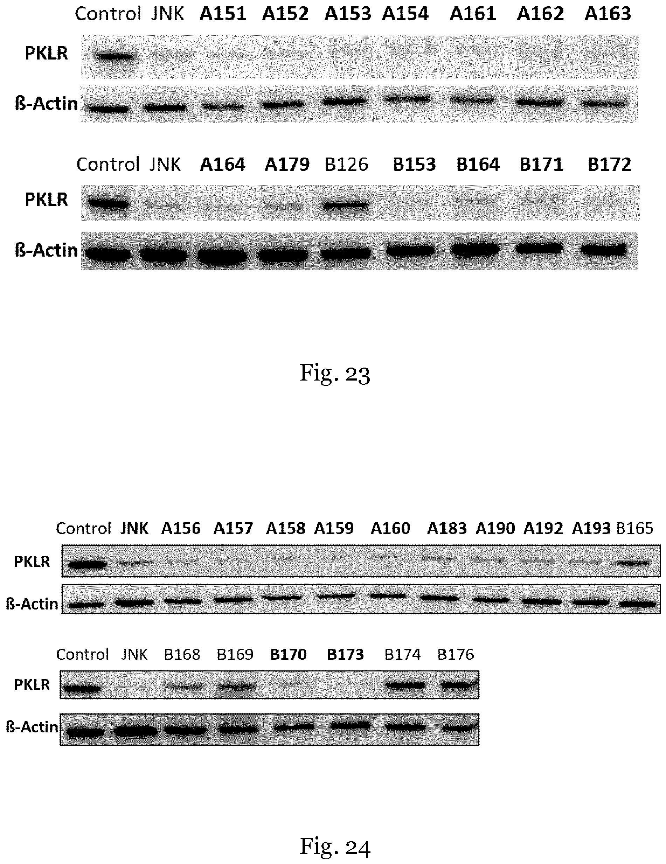

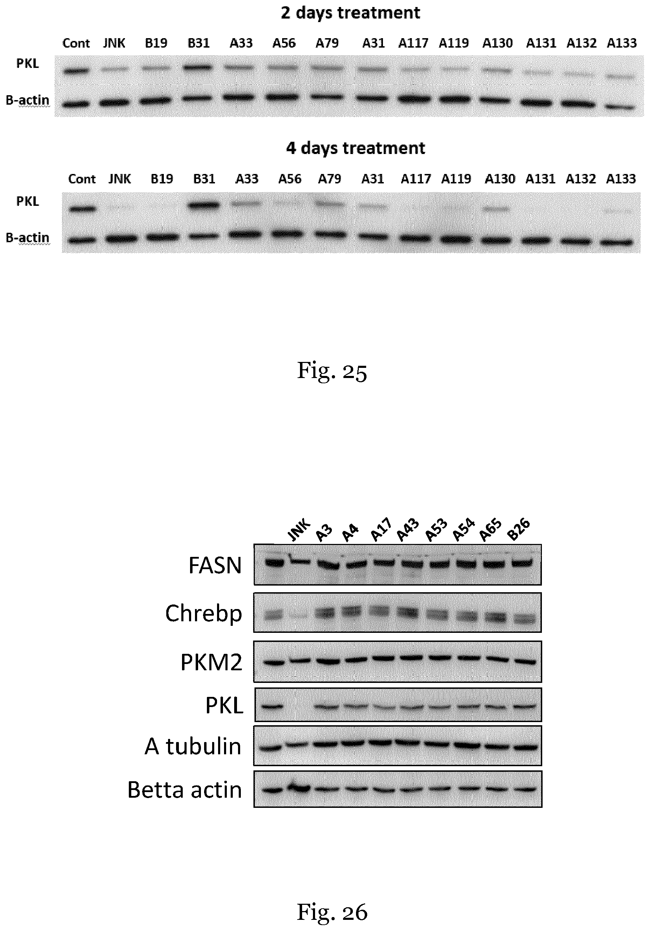

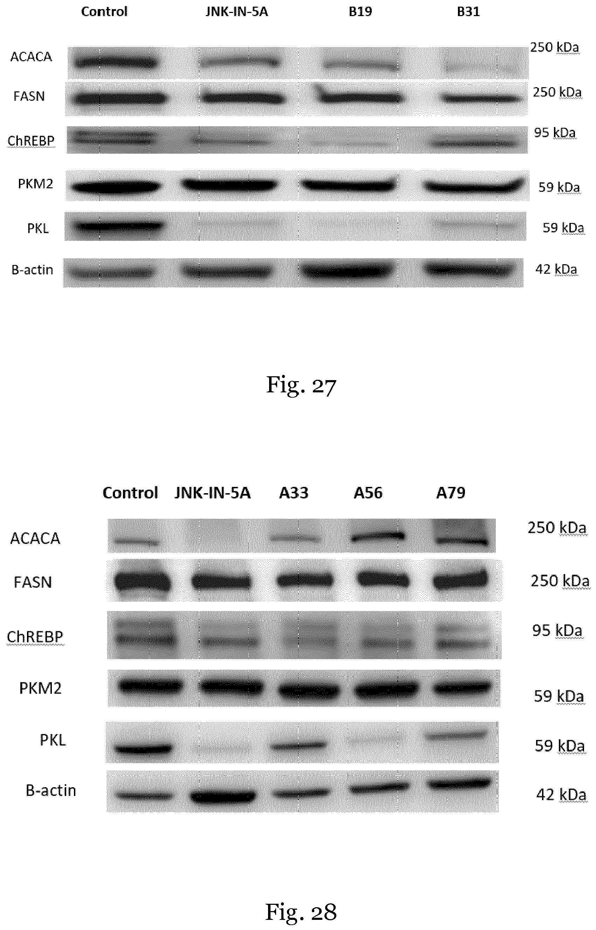

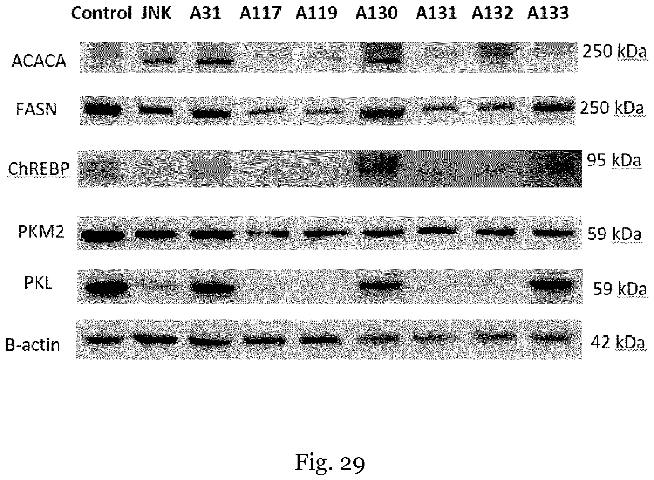

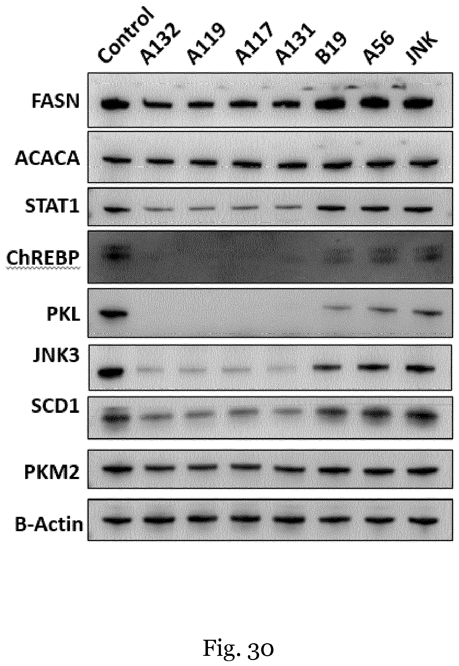

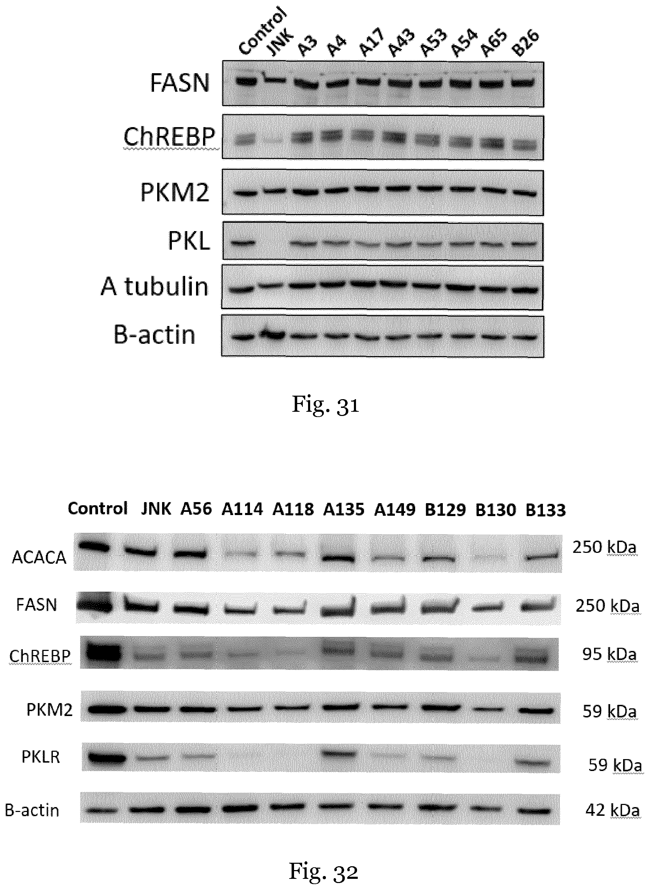

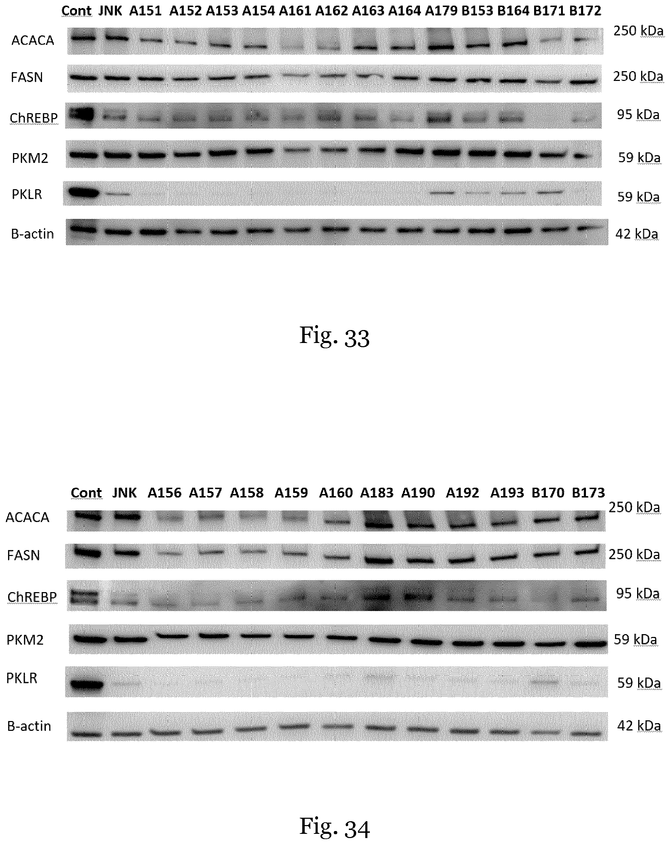

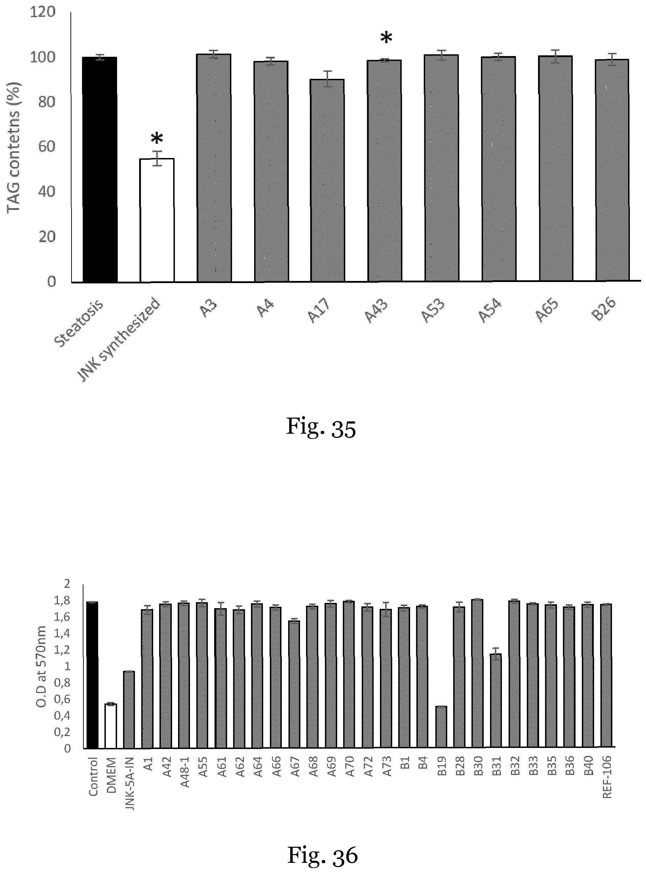

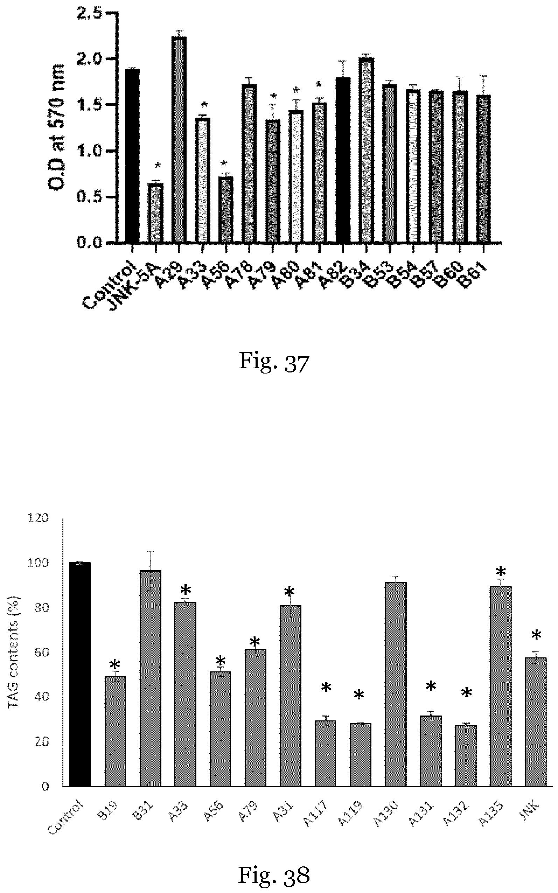

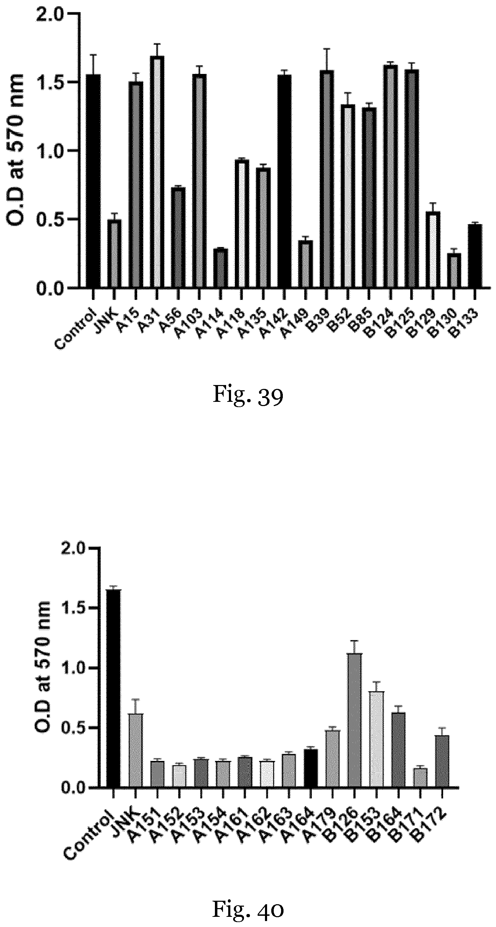

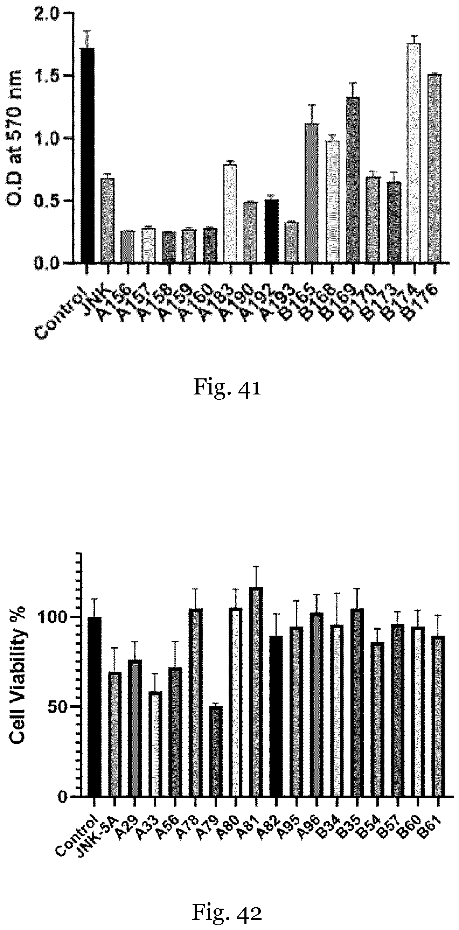

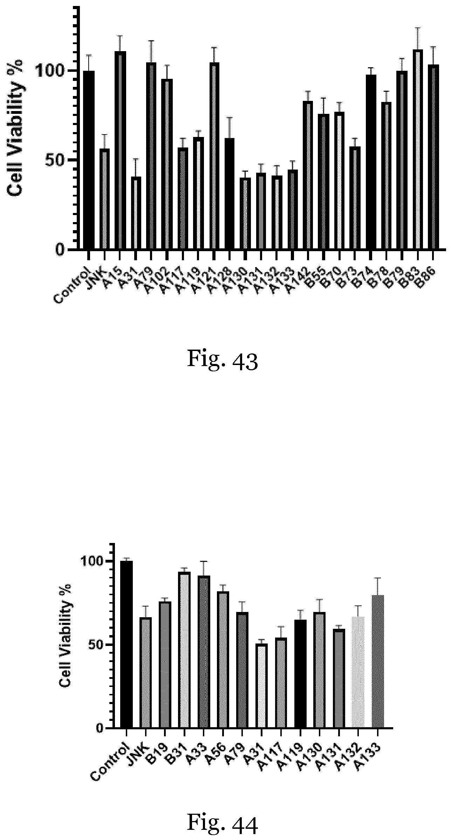

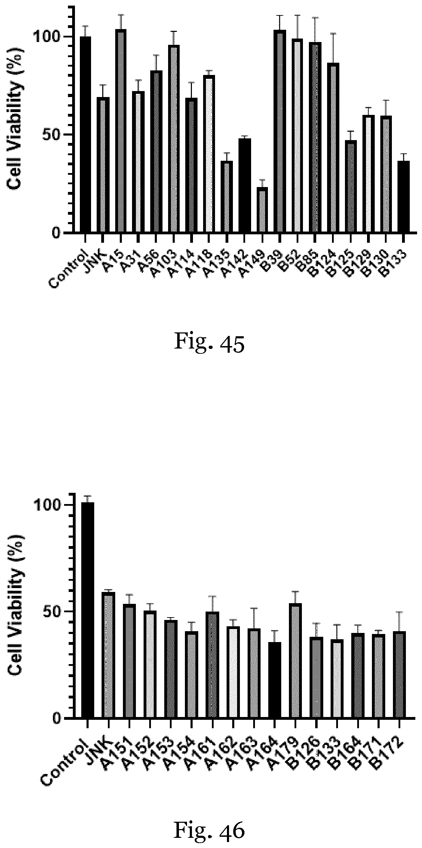

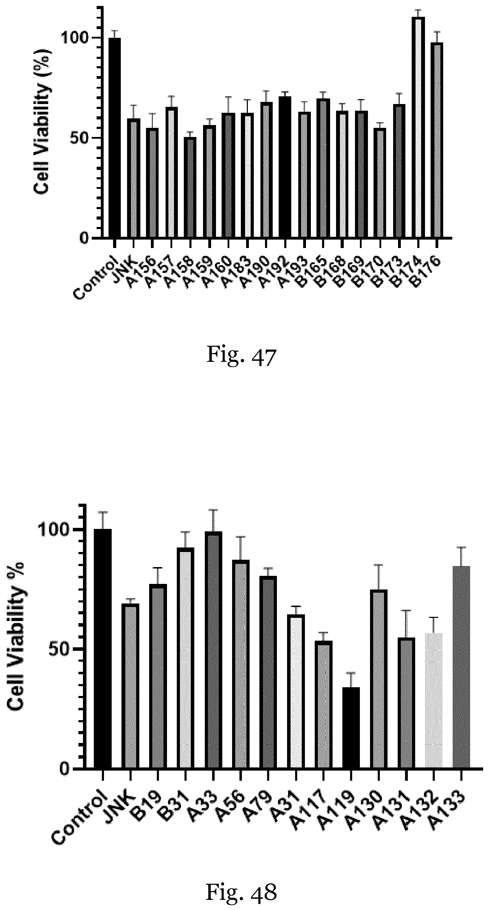

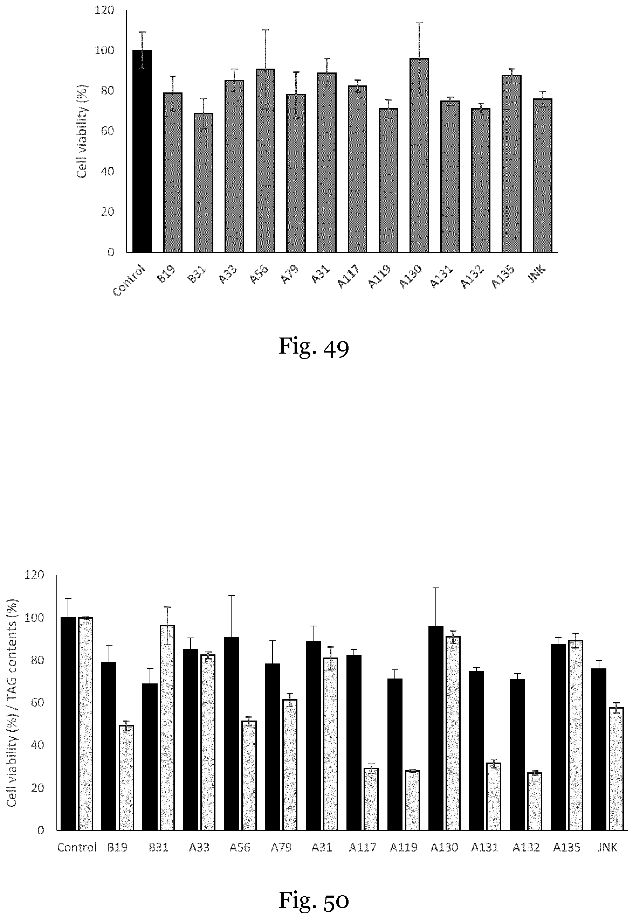

The activity of the synthesized target compounds was determined, see Table 1. All the active compounds resulted in reduced expression levels of PKLR (see Fig. 19-25) and other steatosis related proteins (see Fig. 26-34) as well as reduced TAG accumulation (see Fig. 35-41 and 47-51). Most of the active compounds also resulted in reduced cell viability of HepG2 (see Fig. 42-50).

**Table 1. A list of all synthesized compounds and their effectiveness.**

| **Compound** | **Structure** | **Active** | **Not active** |
|---|---|---|---|
| PKL-A17 | 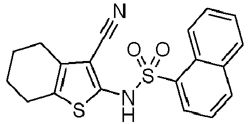 | X | |
| PKL-A43 | 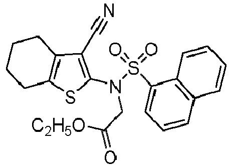 | | X |
| PKL-A44 | 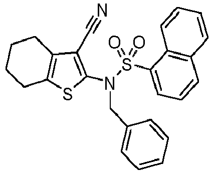 | | X |
| PKL-A53 | 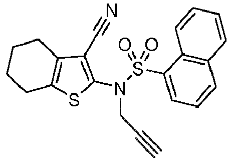 | | X |
| PKL-A54 | 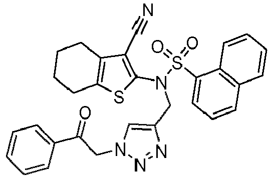 | | X |
| PKL-A58 | 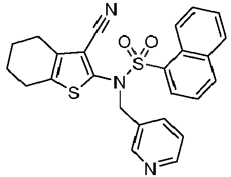 | | X |
| PKL-A2 | 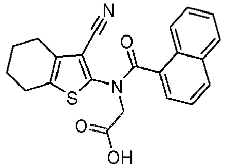 | | X |
| PKL-A3 | 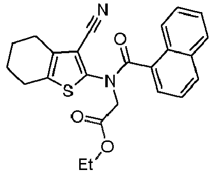 | | X |
| PKL-A4 | 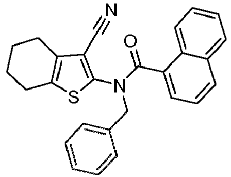 | | X |
| PKL-B29 | 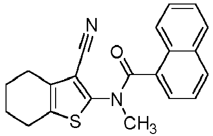 | | X |
| PKL-A65 | 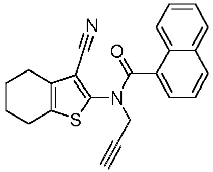 | | X |
| PKL-B26 | 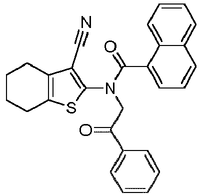 | | X |
| PKL-A66 | 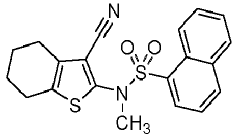 | | X |
| PKL-A42 | 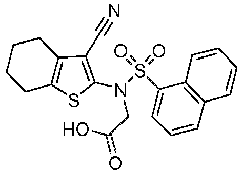 | | X |
| PKL-A61 | 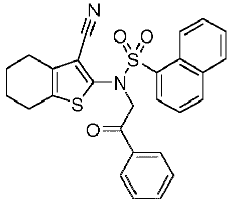 | | X |
| PKL-A62 | 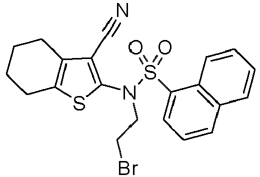 | | X |
| PKL-A55 | 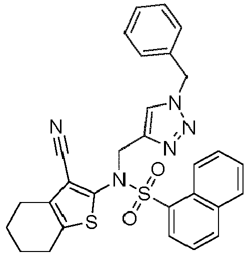 | | X |
| PKL-A64 | 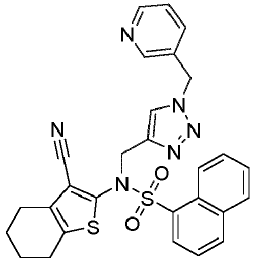 | | X |
| PKL-A48-1 | 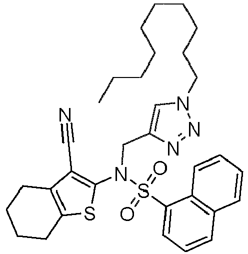 | | X |
| PKL-B1 | 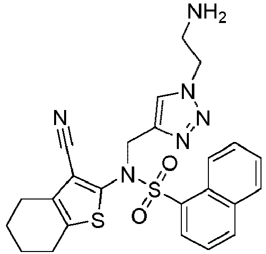 | | X |
| PKL-A73 | 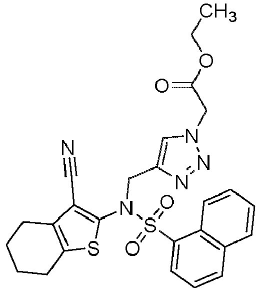 | | X |
| PKL-A67 | 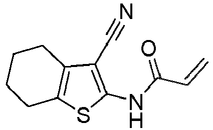 | | X |
| PKL-A69 | 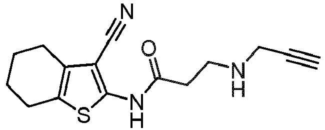 | | X |
| PKL-A68 | 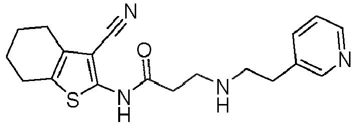 | | X |
| PKL-A72 | 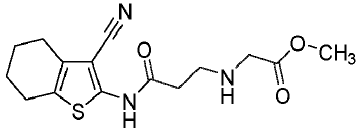 | | X |
| PKL-A70 | 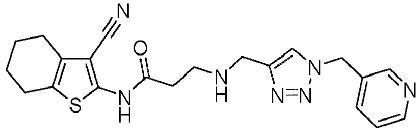 | | X |
| PKL-A1 | 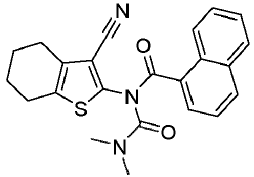 | | X |
| PKL-B28 | 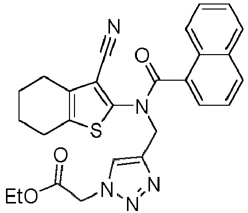 | | X |
| PKL-B3O | 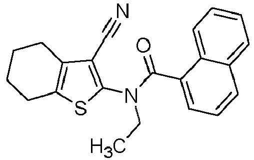 | | X |
| PKL-B31 | 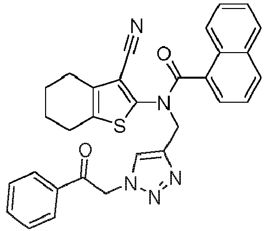 | | X |
| PKL-B32 | 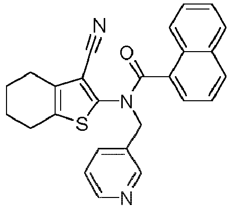 | | X |
| PKL-B33 | 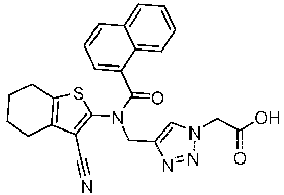 | | X |
| PKL-B35 | 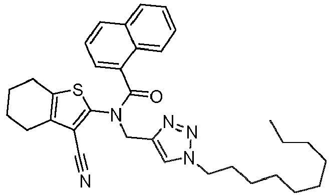 | | X |
| PKL-B4 | 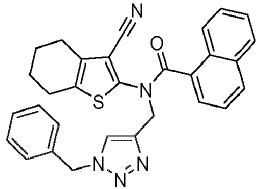 | | X |
| PKL-B40 | 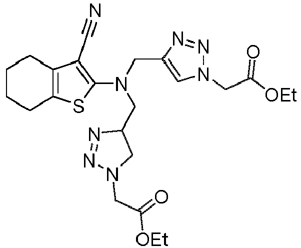 | | X |
| PKL-B36 | 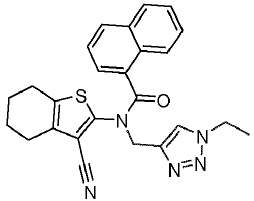 | | X |
| PKL-B19/ PKL-A131 | 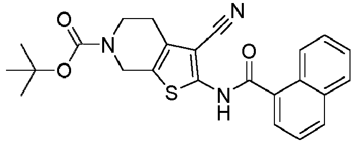 | X | |
| PKL-A15 | 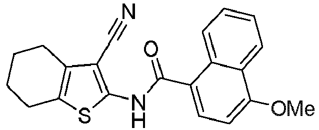 | | X |
| PKL-A79 | 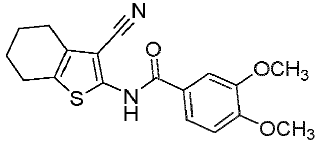 | | X |
| PKL-A102 | 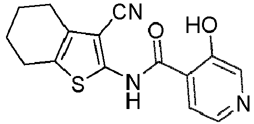 | | X |
| PKL-A117 | 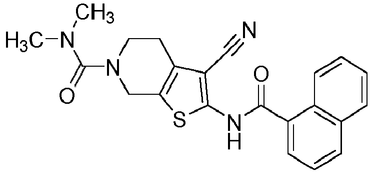 | X | |
| PKL-A119 | 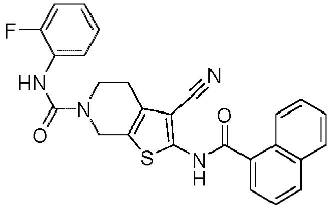 | X | |
| PKL-A130 | 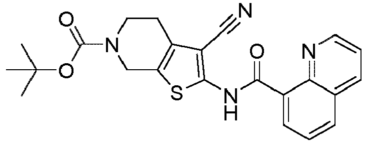 | X | |
| PKL-A131/ PKL-B19 | 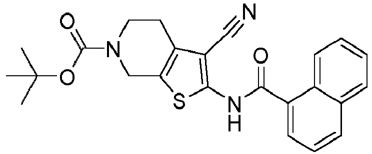 | X | |
| PKL-A132 | 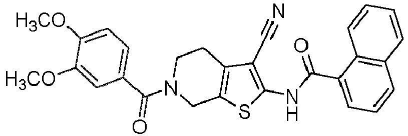 | X | |
| PKL-A133 | 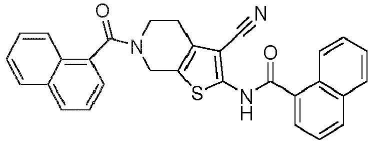 | X | |
| PKL-A121 | 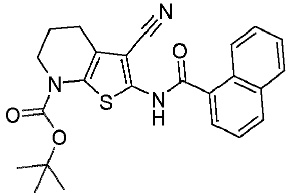 | | X |
| PKL-A128 | 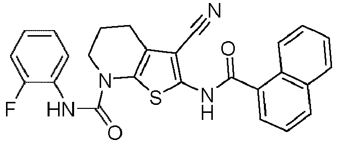 | | X |
| PKL-A142 | 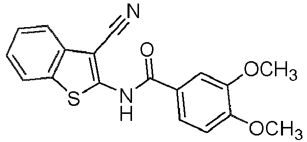 | | X |
| PKL-B74 | 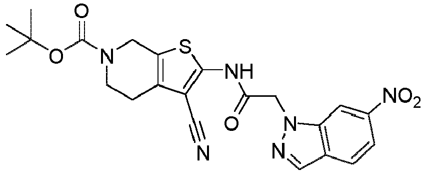 | | X |
| PKL-B70 | 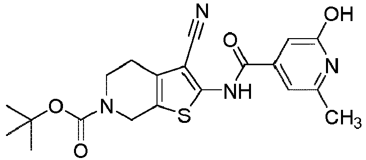 | | X |
| PKL-B55 | 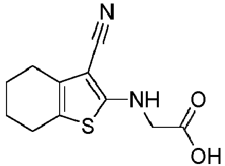 | | X |
| PKL-B79 | 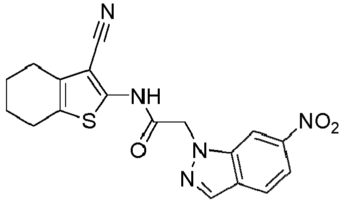 | | X |
| PKL-B73 | 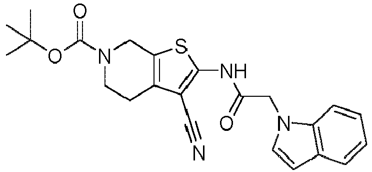 | | X |
| PKL-B78 | 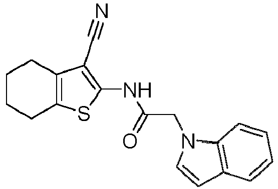 | | X |
| PKL-B86 | 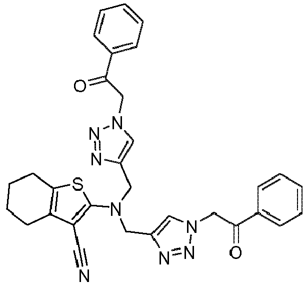 | | X |
| PKL-B83 | 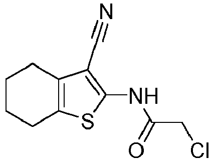 | | X |
| PKL-A15 | 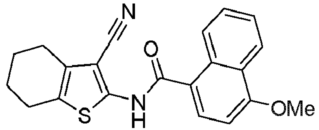 | | X |
| PKL-A31 | 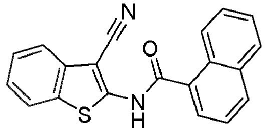 | | X |
| PKL-A56 | 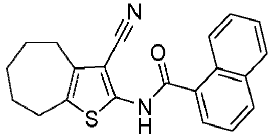 | X | |
| PKL-A103 | 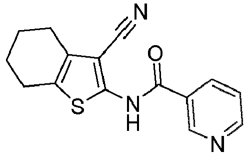 | | X |
| PKL-A114 | 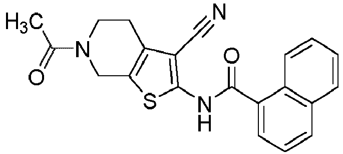 | X | |
| PKL-A118 | 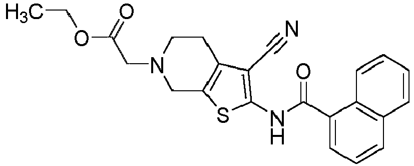 | X | |
| PKL-A135 | 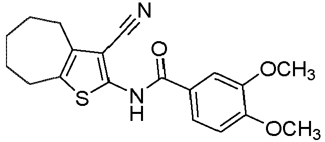 | X | |
| PKL-A142 | 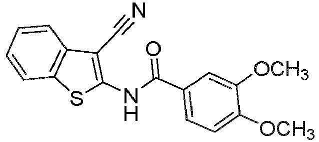 | | X |
| PKL-A149 | 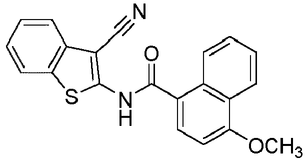 | X | |
| PKL-B85 | 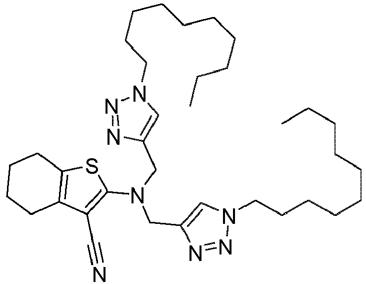 | | X |
| PKL-B52 | 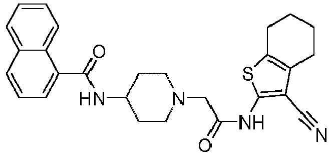 | | X |
| PKL-B129 | 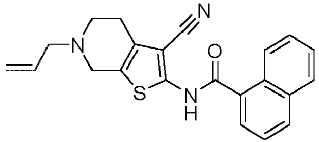 | X | |
| PKL-B125 | 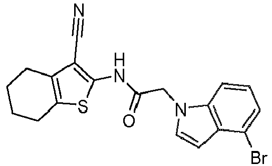 | | X |
| PKL-B133 | 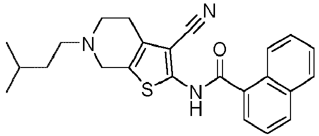 | X | |
| PKL-B124 | 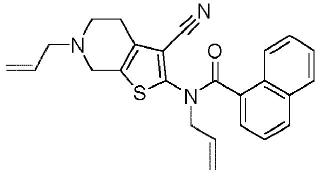 | | X |
| PKL-B130 | 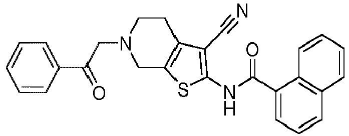 | X | |
| PKL-B39 | 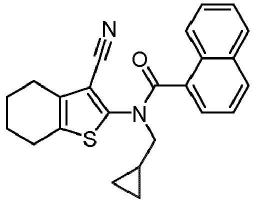 | | X |
| PKL-A151 | 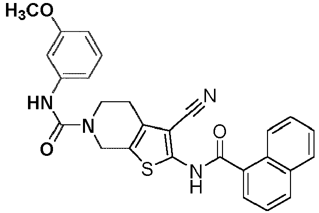 | X | |
| PKL-A152 | 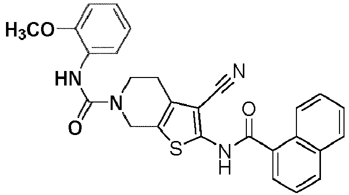 | X | |
| PKL-A153 | 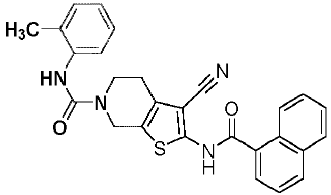 | X | |
| PKL-A154 | 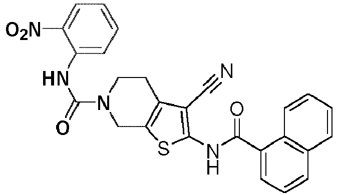 | X | |
| PKL-A161 | 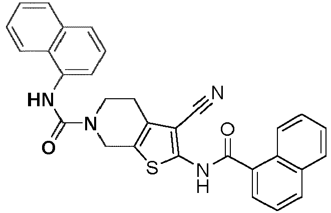 | X | |
| PKL-A162 | 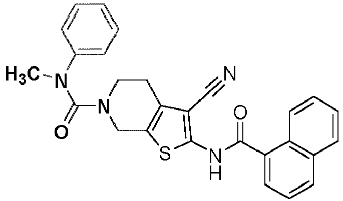 | X | |
| PKL-A163 | 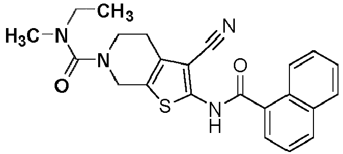 | X | |
| PKL-A164 | 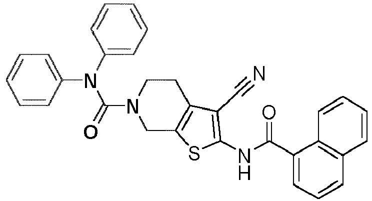 | X | |
| PKL-A179 | 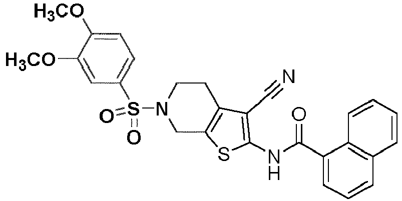 | X | |
| PKL-B126 | 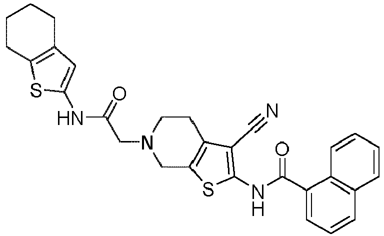 | | X |
| PKL-B153 | 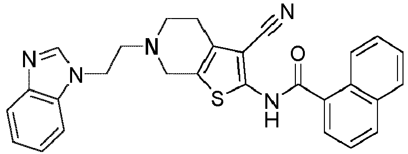 | X | |
| PKL-B164 | 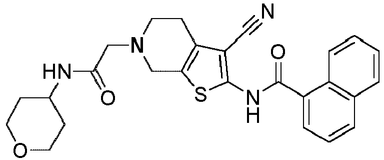 | X | |
| PKL-B171 | 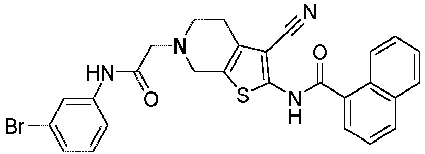 | X | |
| PKL-B172 | 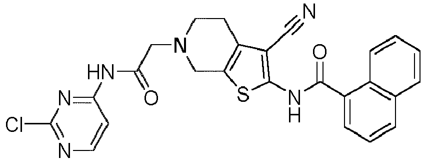 | X | |
| PKL-A156 | 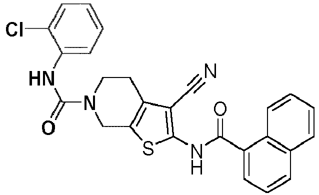 | X | |
| PKL-A157 | 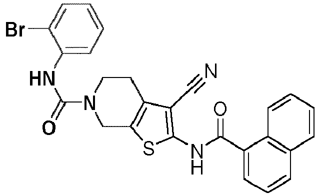 | X | |
| PKL-A158 | 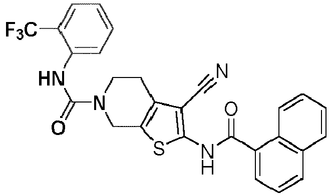 | X | |
| PKL-A159 | 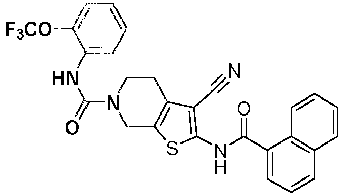 | X | |
| PKL-A160 | 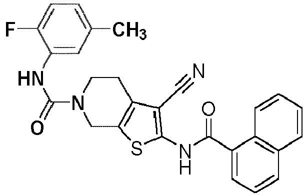 | X | |
| PKL-A183 | 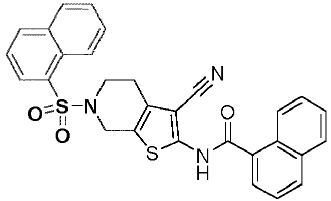 | X | |
| PKL-A190 | 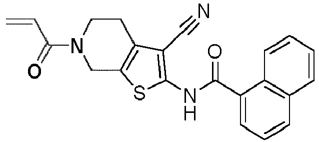 | X | |
| PKL-A192 | 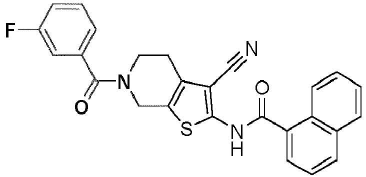 | X | |
| PKL-A193 | 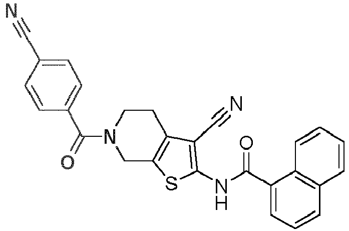 | X | |
| PKL-B165 | 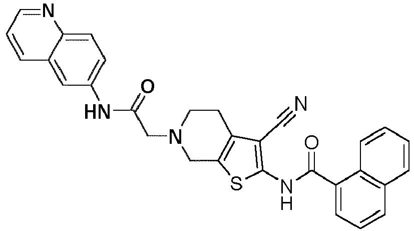 | | X |
| PKL-B168 | 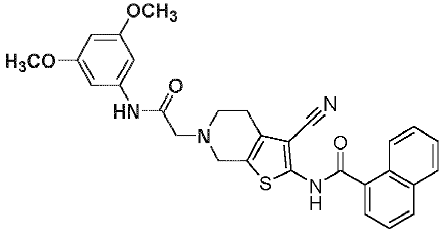 | | X |
| PKL-B169 | 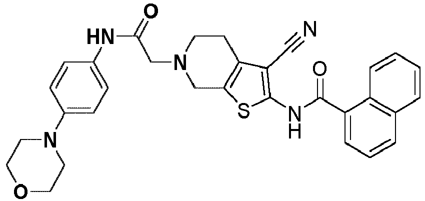 | | X |
| PKL-B170 | 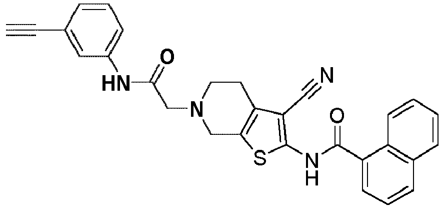 | X | |
| PKL-B173 | 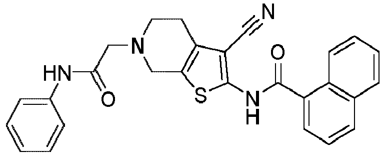 | X | |
| PKL-B174 | 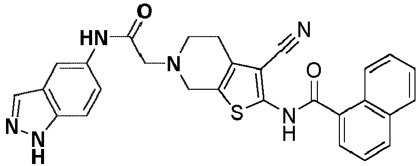 | | X |
| PKL-B176 | 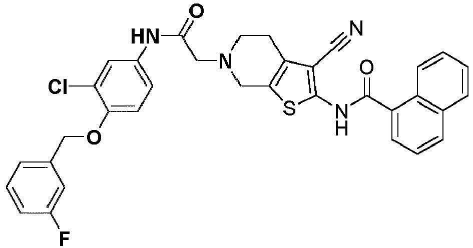 | | X |

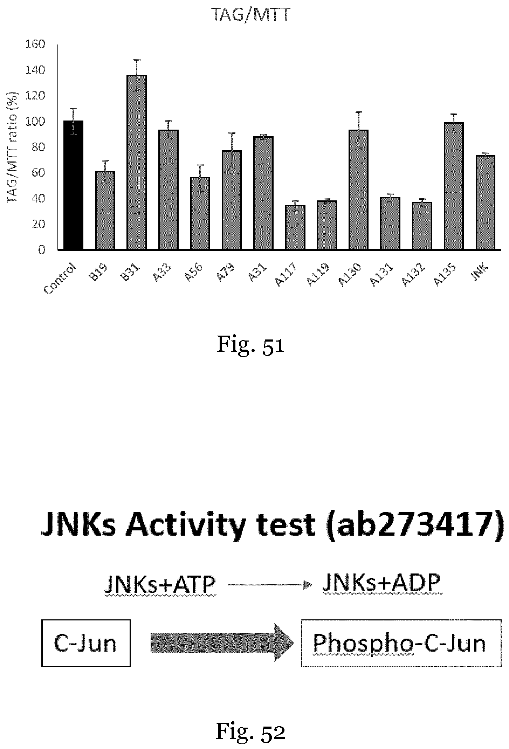

The JNK enzyme activity test, see Fig. 52-53, showed that the tested target molecules (A132, A119, A117 and A131) inhibited of expression of JNK1, JNK2 and JNK3. The greatest inhibition was obtained for JNK3.

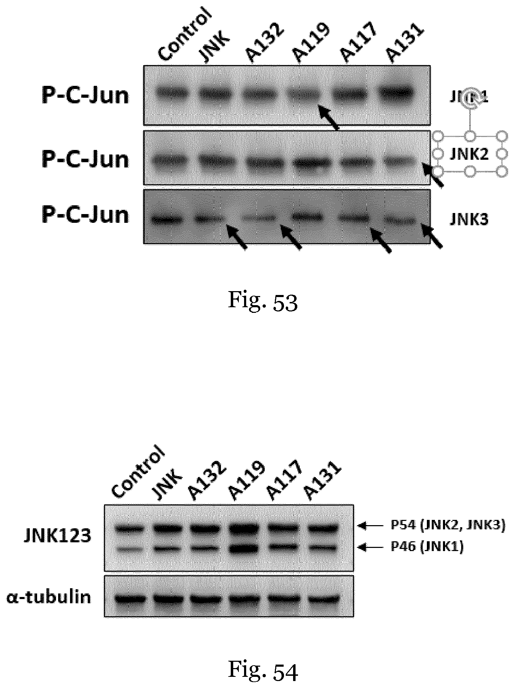

The protein-ligand binding was assessed for selected drug candidates, see Fig. 54-55. The tested drug candidates (A132, A119, A117 and A131) exhibited a better binding to the JNK protein than JNK-IN-5A.

### Ranking of the target compounds

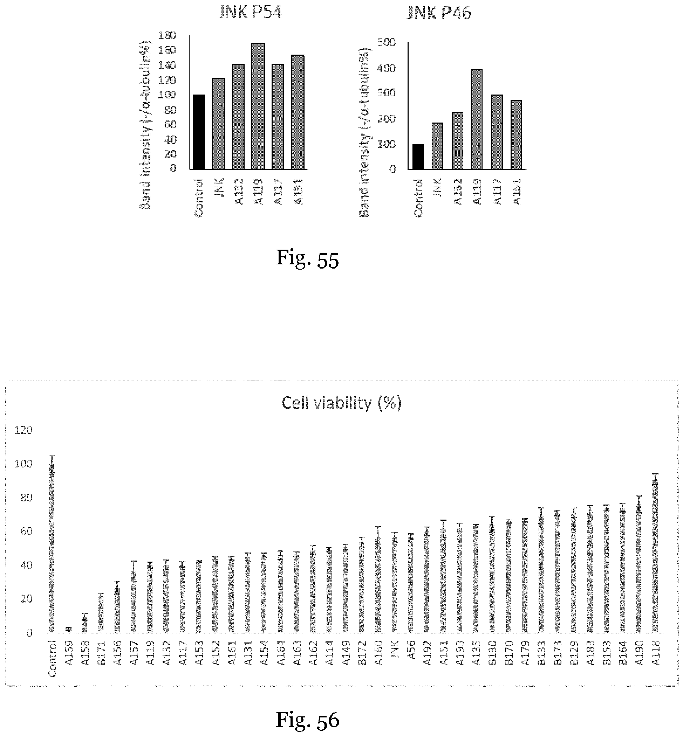

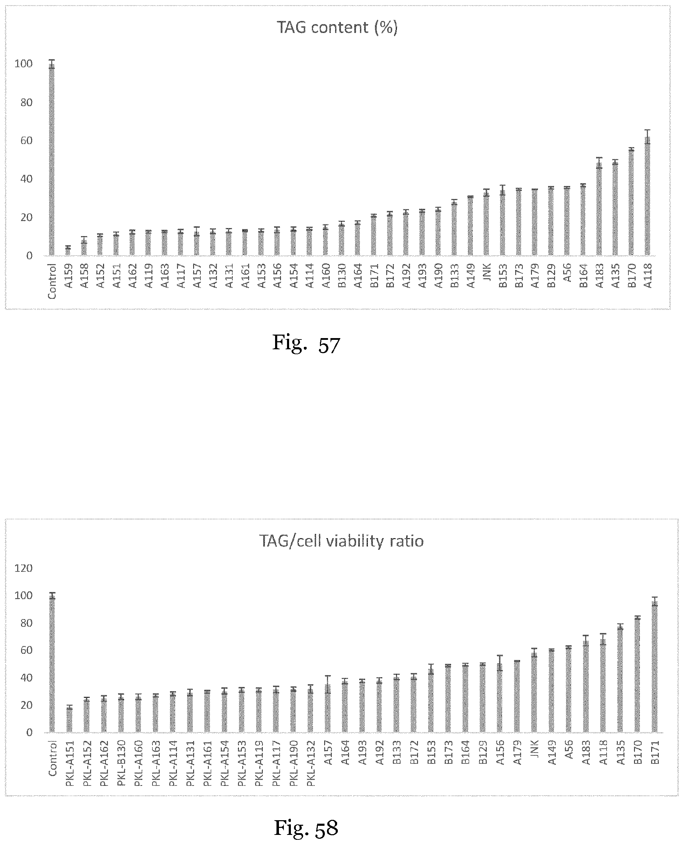

The ranking of the synthesized target compounds can be seen in Tables 2-4. The ranking is based on TAG contents, cell viability and the ratio between TAG content and cell viability. The ranking based on cell viability, Table 3 and Fig. 56, shows the compounds that may be suitable for treatment of HCC where a low cell viability is desired. The rankings based on TAG content and the ratio between TAG content and cell viability, Tables 2 and 4 and Fig. 57-58, show the compounds which are most suitable for treatment of fatty liver disease where a low TAG content together with a low toxicity is desired.

PKL-A159 and PKL-A158 had the greatest effect on TAG accumulation, having a TAG content of 4.54 % and 8.35 % respectively, see Table 2 and Fig. 57. These compounds were followed by PKL-A158 and PKL-A152 having a TAG content of 10.77 % and 11.51 %. 26 of the tested target compounds resulted in lower TAG content than JNK-IN-5A.

**Table 2. Ranking of the tested target compounds based on TAG content.**

| **Ranking** | **Compound** | **TAG (%)** | **SD ±** |
|---|---|---|---|
| - | Control | 100.00 | 2.16 |
| 1 | A159 | 4.54 | 0.75 |
| 2 | A158 | 8.35 | 1.71 |
| 3 | A152 | 10.77 | 0.67 |
| 4 | A151 | 11.51 | 0.90 |
| 5 | A162 | 12.35 | 0.96 |
| 6 | A119 | 12.54 | 0.58 |
| 7 | A163 | 12.68 | 0.50 |
| 8 | A117 | 12.75 | 0.90 |
| 9 | A157 | 12.80 | 2.25 |
| 10 | A132 | 12.85 | 1.18 |
| 11 | A131 | 13.10 | 1.03 |
| 12 | A161 | 13.15 | 0.38 |
| 13 | A153 | 13.25 | 0.68 |
| 14 | A156 | 13.54 | 1.49 |
| 15 | A154 | 13.94 | 0.99 |
| 16 | A114 | 14.11 | 0.69 |
| 17 | A160 | 14.87 | 1.27 |
| 18 | B130 | 16.76 | 1.28 |
| 19 | A164 | 17.28 | 0.91 |
| 20 | B171 | 21.05 | 0.69 |
| 21 | B172 | 21.97 | 1.10 |
| 22 | A192 | 22.85 | 1.16 |
| 23 | A193 | 23.41 | 0.74 |
| 24 | A190 | 24.22 | 1.11 |
| 25 | B133 | 28.11 | 1.33 |
| 26 | A149 | 30.78 | 0.37 |
| *27* | *JNK* | *32.97* | *1.83* |
| 28 | B153 | 34.34 | 2.63 |
| 29 | B173 | 34.71 | 0.48 |
| 30 | A179 | 34.72 | 0.18 |
| 31 | B129 | 35.52 | 0.58 |
| 32 | A56 | 35.59 | 0.44 |
| 33 | B164 | 36.79 | 0.75 |
| 34 | A183 | 48.61 | 2.75 |
| 35 | A135 | 49.06 | 1.25 |
| 36 | B170 | 55.63 | 0.72 |
| 37 | A118 | 62.07 | 3.72 |

PKL-A159 and PKL-A158 resulted in the greatest reduction and were hence the most toxic compounds, see Table 3 and Fig. 56. The cell viability of these compounds was 2.33 % and 9.59 % respectively. PKL-A159 and PKL-A158 were followed by PKL-B171 and PKL-A156, which resulted in higher cell viability, 22.00 % and 26.64 % respectively, but were still toxic. These four compounds and in particular PKL-A159, PKL-A158 and PKL-B171, may be particularly suitable for treatment of HCC where a great reductions of cell viability is desired.

**Table 3. Ranking of the tested target compounds based on cell viability.**

| **Ranking** | **Compound** | **Cell viability (%)** | **SD ±** |
|---|---|---|---|
| - | Control | 100.00 | 5.02 |
| 1 | A159 | 2.33 | 0.84 |
| 2 | A158 | 9.59 | 1.96 |
| 3 | B171 | 22.00 | 1.18 |
| 4 | A156 | 26.64 | 3.83 |
| 5 | A157 | 36.47 | 6.01 |
| 6 | A119 | 40.12 | 1.57 |
| 7 | A132 | 40.31 | 2.91 |
| 8 | A117 | 40.59 | 1.58 |
| 9 | A153 | 42.50 | 0.43 |
| 10 | A152 | 43.87 | 1.42 |
| 11 | A161 | 44.06 | 0.93 |
| 12 | A131 | 44.75 | 2.57 |
| 13 | A154 | 45.95 | 1.38 |
| 14 | A164 | 46.01 | 2.47 |
| 15 | A163 | 46.64 | 1.44 |
| 16 | A162 | 49.20 | 2.63 |
| 17 | A114 | 49.35 | 1.28 |
| 18 | A149 | 50.88 | 1.66 |
| 19 | B172 | 53.70 | 3.04 |
| 20 | A160 | 56.41 | 6.36 |
| 21 | JNK | 56.44 | 2.85 |
| 22 | A56 | 56.98 | 1.47 |
| 23 | A192 | 60.10 | 2.44 |
| 24 | A151 | 61.60 | 5.00 |
| 25 | A193 | 62.32 | 2.39 |
| 26 | A135 | 63.24 | 0.68 |
| 27 | B130 | 64.10 | 4.88 |
| 28 | B170 | 66.03 | 1.06 |
| 29 | A179 | 66.54 | 0.97 |
| 30 | B133 | 69.29 | 4.79 |
| 31 | B173 | 70.83 | 1.40 |
| 32 | B129 | 71.23 | 3.01 |
| 33 | A183 | 72.28 | 3.00 |
| 34 | B153 | 74.00 | 1.70 |
| 35 | B164 | 74.13 | 2.45 |
| 36 | A190 | 76.16 | 5.12 |
| 37 | A118 | 91.00 | 3.20 |

PKL-A159 and PKL-A158 were removed from the ranking of the ratio between TAG content and cell viability due to their high toxicity which would not be suitable in the treatment of fatty liver disease. PKL-A151 exhibits the lowest ratio, see Table 4 and Fig. 58, and is hence particularly suitable for the treatment of fatty liver disease. PKL-A151 has a TAG content/cell viability ratio of 18.7 which is more than 3 times lower than JNK-IN-5A while having a higher cell viability than JNK-IN-5A. PKL-A151 is followed by PKL-A152 and PKL-A162 as top drug candidates for the treatment of fatty liver disease having a TAG/cell viability of 24.5 % and 25.1 % respectively. 27 of the tested compounds exhibited a lower TAG content than JNK-IN-5A.

**Table 4. Ranking of the tested target compounds based on the ratio between TAG content and cell viability.**

| **Ranking** | **Compound** | **TAG/cell viability** | **SD ±** |
|---|---|---|---|
| - | Control | 100.0 | 2.2 |
| 1 | A151 | 18.7 | 1.5 |
| 2 | A152 | 24.5 | 1.5 |
| 3 | A162 | 25.1 | 2.0 |
| 4 | B130 | 26.2 | 2.0 |
| 5 | A160 | 26.4 | 2.2 |
| 6 | A163 | 27.2 | 1.1 |
| 7 | A114 | 28.6 | 1.4 |
| 8 | A131 | 29.3 | 2.3 |
| 9 | A161 | 29.8 | 0.9 |
| 10 | A154 | 30.3 | 2.1 |
| 11 | A153 | 31.2 | 1.6 |
| 12 | A119 | 31.2 | 1.4 |
| 13 | A117 | 31.4 | 2.2 |
| 14 | A190 | 31.8 | 1.5 |
| 15 | A132 | 31.9 | 2.9 |
| 16 | A157 | 35.1 | 6.2 |
| 17 | A164 | 37.6 | 2.0 |
| 18 | A193 | 37.6 | 1.2 |
| 19 | A192 | 38.0 | 1.9 |
| 20 | B133 | 40.6 | 1.9 |
| 21 | B172 | 40.9 | 2.1 |
| 22 | B153 | 46.4 | 3.6 |
| 23 | B173 | 49.0 | 0.7 |
| 24 | B164 | 49.6 | 1.0 |
| 25 | B129 | 49.9 | 0.8 |
| 26 | A156 | 50.8 | 5.6 |
| 27 | A179 | 52.2 | 0.3 |
| 28 | JNK | 58.4 | 3.2 |
| 29 | A149 | 60.5 | 0.7 |
| 30 | A56 | 62.5 | 0.8 |
| 31 | A183 | 67.2 | 3.8 |
| 32 | A118 | 68.2 | 4.1 |
| 33 | A135 | 77.6 | 2.0 |
| 34 | B170 | 84.2 | 1.1 |
| 35 | B171 | 95.7 | 3.1 |

In conclusion, several promising candidate drugs have been developed the use in the treatment of fatty liver disease and HCC. The candidate drugs result in significant reduction of the expression levels of PKLR and other steatosis-related proteins as well as reduced TAG contents.

### REFERENCES

Angell RM, Atkinson FL, Brown MJ, Chuang TT, Christopher JA, Cichy-Knight M, Dunn AK, Hightower KE, Malkakorpi S, Musgrave JR, Neu M. N-(3-Cyano-4, 5, 6, 7-tetrahydro-1-benzothien-2-yl) amides as potent, selective, inhibitors of JNK2 and JNK3. Bioorganic & medicinal chemistry letters. 2007 Mar 1;17(5):1296-301.
Chella Krishnan, K., Kurt, Z., Barrere-Cain, R., Sabir, S., Das, A., Floyd, R., Vergnes, L., Zhao, Y., Che, N., Charugundla, S., et al. (2018). Integration of Multi-omics Data from Mouse Diversity Panel Highlights Mitochondrial Dysfunction in Non-alcoholic Fatty Liver Disease. Cell Syst 6, 103-115 eioy.
Gluchowski, N.L., Becuwe, M., Walther, T.C., and Farese, R.V., Jr. (2017). Lipid droplets and liver disease: from basic biology to clinical implications. Nat Rev Gastroenterol Hepatol 14, 343-355.
Gu, Y. G., Florjancic, A. S., Clark, R. F., Zhang, T., Cooper, C. S., Anderson, D. D., ... & Beutel, B. A. (2004). Structure-activity relationships of novel potent MurF inhibitors. Bioorganic & medicinal chemistry letters, 14(1), 267-270.
Halgren, Thomas A. 1996. "Merck Molecular Force Field. I. Basis, Form, Scope, Parameterization, and Performance of MMFF94." Journal of Computational Chemistry 17 (5-6): 490-519.
Lee, S., Zhang, C., Liu, Z., Klevstig, M., Mukhopadhyay, B., Bergentall, M., Cinar, R., Stahlman, M., Sikanic, N., Park, J.K., et al. (2017). Network analyses identify liver-specific targets for treating liver diseases. Mol Syst Biol 13, 938.
Liu, Z., Zhang, C., Lee, S., Kim, W., Klevstig, M., Harzandi, A.M., Sikanic, N., Arif, M., Stahlman, M., Nielsen, J., et al. (2019). Pyruvate kinase L/R is a regulator of lipid metabolism and mitochondrial function. Metab Eng 52, 263-272.
Molecular Operating Environment (MOE), 2019.01; Chemical Computing Group ULC, 1010 Sherbooke St. West, Suite #910, Montreal, QC, Canada, H3A 2R7, 2019." 2019.
Zhang, C., Shi, M., Kim, W., Arif, M., Klevstig, M., Li, X., ... & Mardinoglu, A. (2022). Discovery of therapeutic agents targeting PKLR for NAFLD using drug repositioning. EBioMedicine, 83, 104214.

## Claims

1. A compound of formula (I) 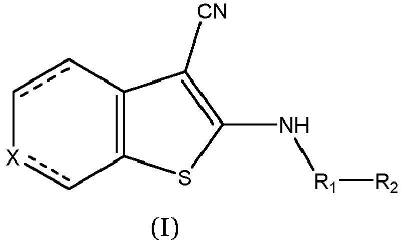 or a pharmaceutically acceptable salt thereof, wherein:
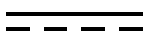 is a single bond or a double bond;
X is selected from -CH₂-, -(CH₂)₂- and -NR₃- when 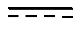 is a single bond;
X is -CH- when 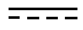 is a double bond;
R₁ is a carbonyl or a sulphonyl;
R₂ is selected from the group consisting of 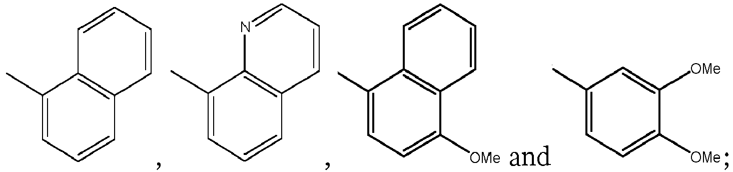
R₃ is selected from the group consisting of -H, 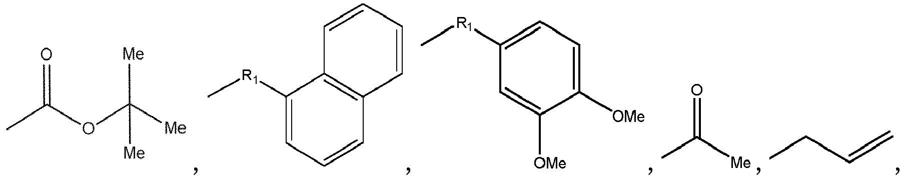 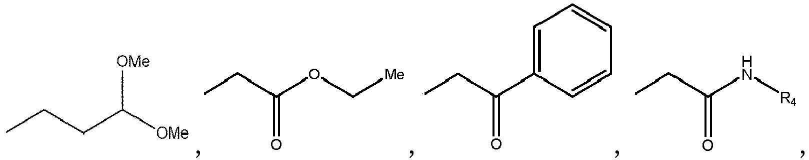 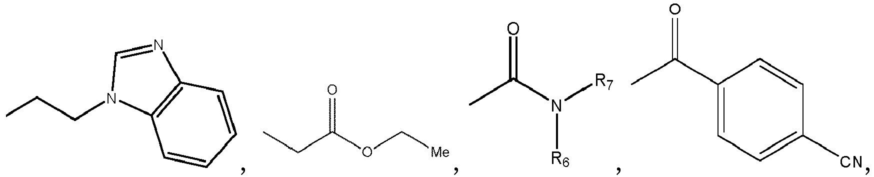 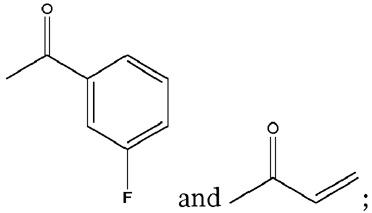
R₄ is 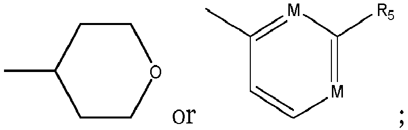
M is N or C;
R5 is -H, halide or -CN;
R6 is -H, -Me or benzyl; and
R7 is C1-C3 alkyl or an optionally substituted aryl;
provided that it is not a compound according to any one of formulae (II)-(VII).

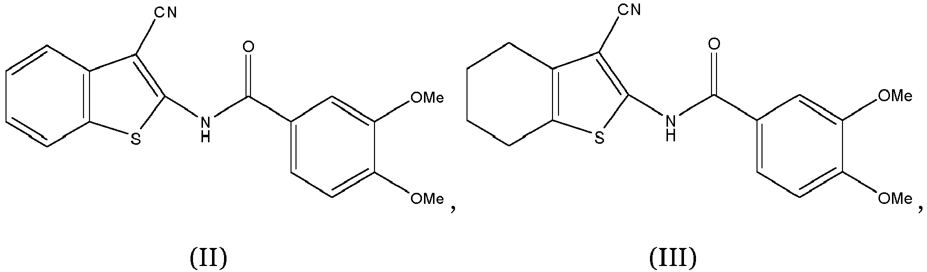

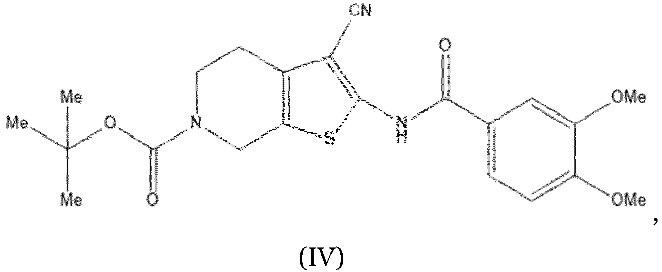

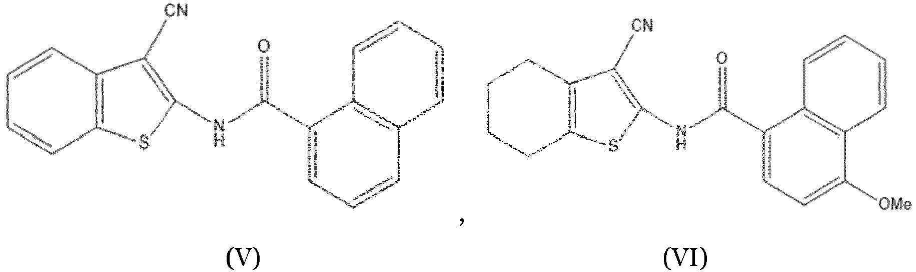

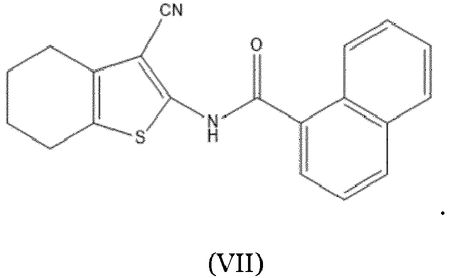

2. The compound of claim 1, wherein R4 is selected form the group consisting of 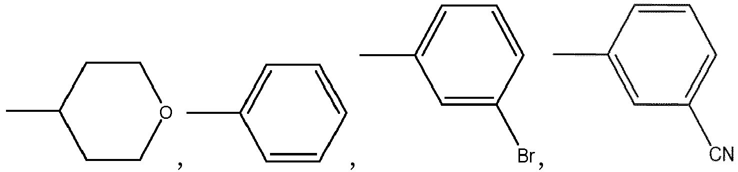 and

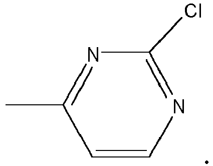

3. The compound of claim 1 or 2, wherein 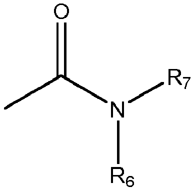 is selected form the group consisting of 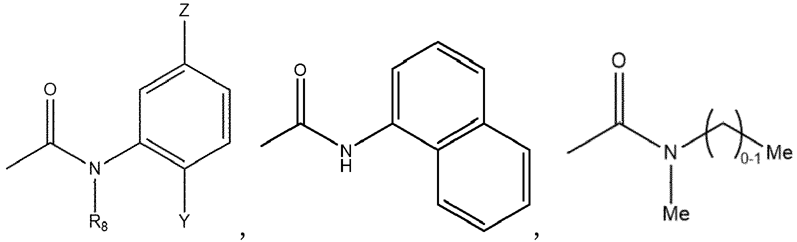 and 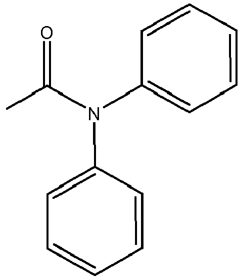 wherein:
Z is selected from the group consisting of -H , -Me and -OMe;
Y is selected from the group consisting of -H, -F, -Br, -Cl, -OMe, -OCF₃, -CF₃, - Me and -NO₂; and
R8 is selected from -H and -Me.

4. The compound of claim 3 wherein Y is selected from the group consisting of -H, -F, -Br, -Cl, -OMe, -Me and -NO₂.

5. The compound of any one of the preceding claims, wherein R1 is a carbonyl.

6. The compound of any one of the preceding claims, wherein 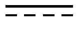 is a single bond and X is -NR₃-.

7. The compound of any one of the preceding claims, wherein R2 is

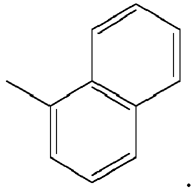

8. The compound of claim 1 having the formula (VIII)

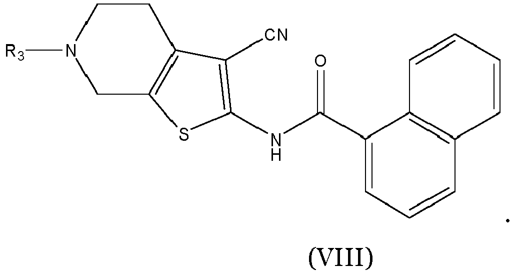

9. The compound of claim 8 wherein R3 is selected from 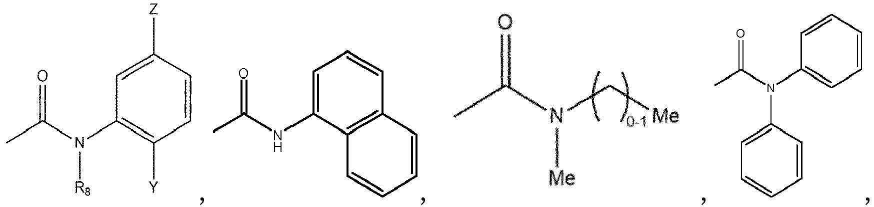 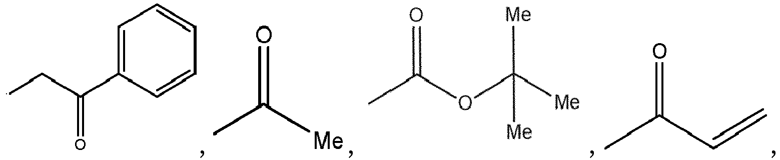 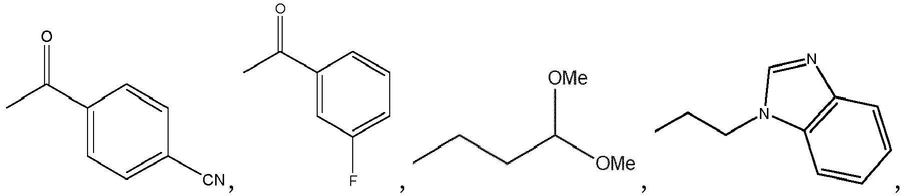 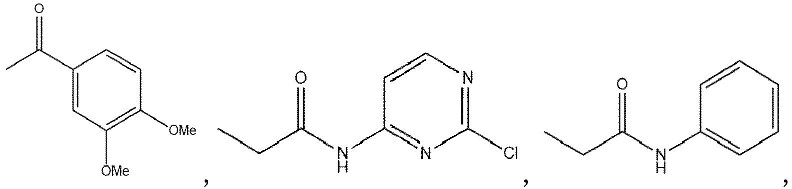 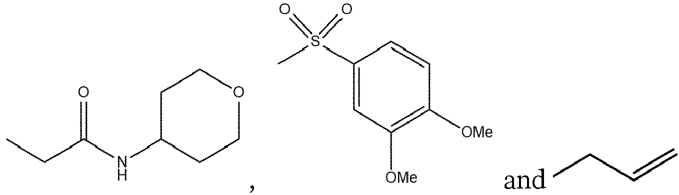 and wherein:
Z is -H, Y is-F, -Br, -Cl, -OMe or -NO₂, and R8 is -H;
Z is -OMe, Y is -H, and R8 is -H;
Z is -Me, Y is -F, and R8 is -H; or
Z is -H, Y is -H, and R8 is -Me.

10. The compound of claim 9 wherein R3 is selected from

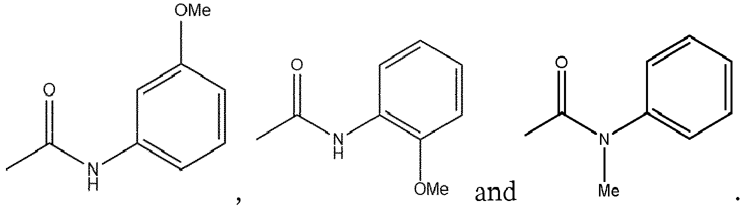

11. A compound according to any one of the preceding claims for use as a medicament.

12. A pharmaceutical composition comprising a compound according to any one of the preceding claims.

13. A compound or a pharmaceutical composition according to any one of the preceding claims for use in a method of treatment of fatty liver disease or hepatocellular carcinoma (HCC).

14. The compound or pharmaceutical composition for use according to claim 13, wherein the method of treatment comprises oral administration of the compound.

15. The compound or pharmaceutical composition for use according to claim 13 or 14, wherein said fatty liver disease is non-alcoholic fatty liver disease (NAFLD), wherein said NAFLD optionally has progressed to non-alcoholic steatohepatitis (NASH).

16. The compound or pharmaceutical composition for use according to any one of claims 13-16, wherein the method of treatment comprises detection of overexpression of PKLR in the liver of the subject to be treated prior to administration of the compound to the subject.
